(19) Europäisches Patentamt
European Patent Office
Office européen des brevets

(11) EP 3 410 118 B1

(12) EUROPEAN PATENT SPECIFICATION

(45) Date of publication and mention of the grant of the patent:
**30.09.2020 Bulletin 2020/40**

(21) Application number: **16888162.1**

(22) Date of filing: **08.12.2016**

(51) Int Cl.:
***G01N 33/574*** *(2006.01)* ***C12Q 1/68*** *(2018.01)*
***G01N 27/447*** *(2006.01)* ***G01N 37/00*** *(2006.01)*

(86) International application number:
**PCT/JP2016/086537**

(87) International publication number:
**WO 2017/130578 (03.08.2017 Gazette 2017/31)**

(54) **PROSTATE CARCINOMA DETERMINATION METHOD**

VERFAHREN ZUR BESTIMMUNG EINES PROSTATAKARZINOMS

PROCÉDÉ DE DÉTERMINATION DU CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2016 JP 2016013034**

(43) Date of publication of application:
**05.12.2018 Bulletin 2018/49**

(73) Proprietors:
- **FUJIFILM Wako Pure Chemical Corporation Osaka-shi, Osaka 540-8605 (JP)**
- **HIROSAKI UNIVERSITY Hirosaki-shi, Aomori 036-8560 (JP)**

(72) Inventors:
- **OHYAMA, Chikara Hirosaki-shi Aomori 036-8560 (JP)**
- **YONEYAMA, Tohru Hirosaki-shi Aomori 036-8560 (JP)**
- **TOBISAWA, Yuki Hirosaki-shi Aomori 036-8560 (JP)**
- **ISHIKAWA, Tomokazu Amagasaki-shi Hyogo 661-0963 (JP)**
- **KUROSAWA, Tatsuo Amagasaki-shi Hyogo 661-0963 (JP)**
- **NAKAMURA, Kenji Osaka-shi Osaka 540-8605 (JP)**

(74) Representative: **Winter, Brandl, Fürniss, Hübner, Röss, Kaiser, Polte - Partnerschaft mbB Patent- und Rechtsanwaltskanzlei Alois-Steinecker-Straße 22 85354 Freising (DE)**

(56) References cited:
**WO-A1-2010/090264 WO-A1-2014/057983**
**WO-A1-2017/077162 JP-A- 2013 156 245**
**JP-B2- 4 514 919 US-A1- 2014 193 832**

- **ESTHER LLOP ET AL: "Improvement of Prostate Cancer Diagnosis by Detecting PSA Glycosylation-Specific Changes", THERANOSTICS, vol. 6, no. 8, 1 January 2016 (2016-01-01) , pages 1190-1204, XP55402495, AU ISSN: 1838-7640, DOI: 10.7150/thno.15226**
- **ARIADNA SARRATS ET AL: "Differential percentage of serum prostate-specific antigen subforms suggests a new way to improve prostate cancer diagnosis", PROSTATE., vol. 70, no. 1, 1 January 2010 (2010-01-01), pages 1-9, XP55609282, US ISSN: 0270-4137, DOI: 10.1002/pros.21031**
- **YONEYAMA, T. et al.: "Measurement of aberrant glycosylation of prostate specific antigen can improve specificity in early detection of prostate cancer", Biochemical and Biophysical Research Communications, vol. 448, no. 4 9 May 2014 (2014-05-09), pages 390-396, XP055537541, Retrieved from the Internet: URL:doi: 10.1016/j.bbrc.2014.04.107**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a novel prostate carcinoma determination method and a novel prostate carcinoma malignancy determination method.

### Background Art

Prostate carcinoma (hereinafter, abbreviated as "Pca") has been the most prevalent cancer among males in Western countries, but has also been growing rapidly in Japan in recent years, so it has been estimated that prostate carcinoma has become a malignant tumor with the highest incidence even among Japanese males in 2015.

Prostate specific antigen (hereinafter, abbreviated as "PSA") is a kind of prostate-specific glycoprotein produced in the prostate. A PSA value is recognized as the most important tumor marker for determining Pca in that the PSA value in blood increases in the case where an individual is affected with Pca (Non-Patent Literature 1).

PSA exists as complexed PSA in which most of PSA bind to a binding protein such as $\alpha$1-antichymotrypsin or $\alpha$2-macroglobulin to form a complex in blood (hereinafter, abbreviated as "complexed PSA"). In addition, some PSA exists as a free form that does not form a complex (hereinafter, abbreviated as "free PSA").

In the currently widely performed serum PSA examination, the total amount of PSA without distinguishing between free PSA and complexed PSA (that is, the total amount of free PSA and complexed PSA, hereinafter abbreviated as "total PSA value") is measured. The reference value (normal value) of the total PSA value is less than 4 ng/mL. In the case where there is any disease in the prostate, the total PSA value increases. It is said that Pca is found in about 40% of patients in the case where the total PSA value is 10 to 20 ng/mL and Pca is found in 50% or more of patients in the case where the total PSA value is 20 ng/mL or more.

The intermediate range of 4.1 to 10 ng/mL where the total PSA value is higher than the normal value but is lower than the high value is called a so-called gray zone (Non-Patent Literature 2). It is said that there is about 25% to 30% probability that cancer will be found in the patient showing the total PSA value of this gray zone. It is known that the total PSA value often becomes higher even in the case where other prostatic diseases such as benign prostatic hyperplasia (hereinafter, abbreviated as "BPH") and prostatitis are present. That is, even in the case where the total PSA value is higher than the normal value, it does not necessarily mean that an individual is affected with Pca. Therefore, in the case where the total PSA value is in the gray zone in the serum PSA examination, the needle biopsy is usually carried out in order to obtain a definite diagnosis, but it is a problem that the risk of infectious diseases increases due to the needle biopsy.

In order to solve this problem, an attempt has been made to determine Pca using an index, for example, a PSA density, a PSA gradient, or a ratio of free PSA/total PSA as an index, in addition to the total PSA value. Although the possibility of improving the diagnostic accuracy of Pca has been reported by a combination assay using these indices, it has not yet reached a level that the method is generally prevalent in the clinical field.

Meanwhile, many of the molecules currently used as tumor markers are glycoproteins. It is known that the structure of the glycan of this tumor marker is greatly different between those derived from normal tissue and those derived from cancer.

PSA is also a glycoprotein having a molecular weight of 34 kDa, and glycans account for about 8% thereof.

In studies of glycans in PSA, it has been reported that PSA has a double-stranded glycan, and that the glycan is only an *N*-glycan in which sialic acid is linked to galactose through an $\alpha$(2,6) linkage at the terminal (Non-Patent Literature 3).

However, Ohyama and others have completed a method for distinguishing between Pca and BPH by affinity chromatography using *Maackia amurensis* lectin (MAA) capable of specifically recognizing a glycan in which a terminal sialic acid residue is linked to galactose through an $\alpha$(2,3) linkage (Patent Literature 1). The method is a "method of taking a percentage ratio of free PSA value and total PSA value of the lectin-bound fraction to free PSA value and total PSA value of the pre-fractionated serum, or taking a percentage ratio of free PSA value of the lectin-bound fraction to free PSA value of the pre-fractionated serum, and then distinguishing between Pca and BPH from the value thus taken".

After that, it was revealed by Ohyama and others that glycans of PSA are highly diverse (Non-Patent Literature 4), and terminal sialic acid of the glycan of PSA being linked to galactose through an $\alpha$(2,3) linkage is also present in a proportion of about 10% as well as terminal sialic acid being linked to galactose through an $\alpha$(2,6) linkage. Then, it was revealed that PSA in which the terminal sialic acid residue of the *N*-glycan is linked to galactose through an $\alpha$(2,3) linkage, rather than PSA in which the terminal sialic acid residue of the *N*-glycan is linked to galactose through an $\alpha$(2,6) linkage, increases in the sera of Pca patients (Non-Patent Literature 5).

In addition, disclosed is a method of confirming whether or not a patient has Pca (whether or not a patient is affected with Pca) by analyzing the degree of $\alpha$(2,6)-linked sialylation of the glycan contained in PSA and the degree of

α(2,3)-linked sialylation of the glycan contained in PSA in the serum sample of the patient using Sambucus nigra lectin (SNA) (Patent Literature 2).

In addition, Ohyama and others have reported a method for distinguishing between Pca and BPH, including measuring an amount of free PSA having an *N*-glycan in which a terminal sialic acid residue is linked to galactose through an α(2,3) linkage, in a sample, using an anti-free PSA antibody and a monoclonal antibody capable of specifically recognizing a glycan in which a terminal sialic acid residue is linked to galactose through an α(2,3) linkage; and comparing the measured amount thus obtained with a preset cutoff value for Pca and BPH, thereby determining that Pca is developed or the probability of developing Pca is high in the case where the measured amount is larger than the cutoff value, and BPH is developed or the probability of developing BPH is high in the case where the measured amount is smaller than the cutoff value (Patent Literature 3 and Non-Patent Literature 6).

Citation List

Patent Literature

Patent Literature 1: JP4514919B
Patent Literature 2: JP2011-529184A
Patent Literature 3: WO2014/057983A

Non-Patent Literature

Non-Patent Literature 1: Stamey T.A. et al., N. Engl. J. Med., 1987, vol. 317, pp. 909 to 916
Non-Patent Literature 2: Catalona W.J., et al., JAMA, 1998, vol. 279, pp. 1542 to 1547
Non-Patent Literature 3: Belanger A, Van Halbeek H, Gravuxes HC, et al., Prostate, 1995, vol. 27, pp. 187 to 197
Non-Patent Literature 4: Ohyama C., et al., Glycobiology, 2004, vol. 14, pp. 671 to 679
Non-Patent Literature 5: Tajiri M., Ohyama C., Wada Y., Glycobiolgy, 2008, vol. 18, pp. 2 to 8
Non-Patent Literature 6: Yoneyama T. et al., Biochem Biophys Res Commun. 2014 vol. 448, No. 4, pp. 390 to 396

Summary of Invention

Technical Problem

In Patent Literature 1, it is described that "the ratios of MAA-bound fraction to free PSA and total PSA in cancer patients were 16.9±5.2 (mean±standard deviation)% and 7.5±4.2%, respectively. On the other hand, in the case of BPH, the ratios were 0.6±0.2% and 0.3±0.1%, respectively".

However, the number of specimens examined in Patent Literature 1 is 17 for Pca patients and 15 for BPH patients, which are extremely small. Therefore, "the ratios of MAA-bound fraction to free PSA and total PSA in cancer patients" described in Patent Literature 1 and specific numerical values obtained could not be used as an index of Pca determination.

In addition, the median value of the total PSA value of the sample derived from a Pca patient used in Patent Literature 1 was 138 ng/mL, which was much higher than the gray zone. Therefore, it was very difficult for the distinguishing method described in Patent Literature 1 to determine Pca of the patient whose total PSA value is in a gray zone.

In addition, even in the method of Patent Literature 3 in which the determination of Pca is carried out using the cutoff value, it was found that it is difficult to make a highly accurate determination of Pca, as a result of verification in Example 6, Comparative Example 4, and Comparative Example 6 of the present specification.

That is, as described above, it has been difficult to make a highly accurate determination of Pca for a patient whose serum PSA value was in a gray zone, especially in clinical examinations even with various conventional indices.

In addition, Pca has benign Pca and malignant Pca. In the case of benign Pca, its progression is slow, and therefore, there are also options to observe the progress without invasive treatment such as surgery. On the other hand, since malignant Pca progresses rapidly, it is necessary to find Pca at an early stage and determine its malignancy. However, markers or determination methods capable of determining between benign Pca and malignant Pca have not yet been known.

The present invention has been made in view of the circumstances, and an object thereof is to provide a novel Pca determination method and a Pca malignancy determination method.

Solution to Problem

As a result of extensive research to solve the problems, the present inventors have found that the ratio of the amount of free PSA having a glycan in which the terminal sialic acid residue of the glycan is α(2,3)-linked to a second galactose residue from the terminal of the glycan to the amount of free PSA in a biological sample can be an index for determining whether or not Pca is developed. As a result of further research, the present inventors have found that it can be determined that Pca is developed or the probability of developing Pca is high in the case where the ratio is higher than 40% and it can be determined that the malignancy of Pca is high in the case where the ratio is higher than 47%. The present invention has been completed on the basis of these findings.

That is, the present invention has the following constitution.

"A prostate carcinoma determination method, including measuring a ratio of an amount of free prostate specific antigen having a glycan of which a terminal sialic acid residue is α(2,3)-linked to a second galactose residue from a terminal of the glycan to an amount of free PSA in a biological sample, and determining that prostate carcinoma is developed or the probability of developing prostate carcinoma is high in the case where the ratio is higher than 40%"

Advantageous Effects of Invention

The Pca determination method of the present invention enables non-invasive and convenient determination (diagnosis or examination) of Pca and malignancy thereof with high accuracy. In particular, it is possible to determine whether or not Pca is developed or the probability of developing Pca is high in a patient whose total PSA value is in a gray zone, who has been conventionally difficult to be determined.

In addition, since the Pca determination method of the present invention can determine the malignancy of Pca, the determination result obtained by the determination method of the present invention is an important guideline for setting a therapeutic strategy of Pca thereafter.

In addition, the Pca determination method of the present invention can determine Pca with high accuracy even in the case of using a sample from non-Japanese that is suspected of affecting measurement of PSA or determination of Pca such as glycan diversity.

Brief Description of Drawings

Fig. 1 is a schematic diagram of an example of a glycan in which a terminal sialic acid residue of the glycan is α(2,3)-linked to a second galactose residue from the terminal of the glycan.

Fig. 2 is a diagram showing a method for preparing a DNA-labeled anti-PSA antibody in Example 1.

Fig. 3 is a schematic diagram of a microchip used in Example 1.

Fig. 4 is a schematic diagram of an in-chip flow channel of the microchip used in Example 1.

Fig. 5 is an electropherogram obtained in Example 1.

Figs. 6(1) and 6(2) are electropherograms obtained in Example 2. Fig. 6(1) shows the results obtained by using electrophoresis sample A containing recombinant α(2,3) free PSA, and Fig. 6(2) shows the results obtained by using electrophoresis sample A containing recombinant α(2,6) free PSA.

Figs. 7(1) and 7(2) show the results of confirming an efficiency of capturing α(2,3) free PSA and α(2,6) free PSA, as measured by microchip capillary electrophoresis, obtained in Example 3. Fig. 7(1) shows a relationship between the ratio of the amount of α(2,3) free PSA to the amount of free PSA calculated on the basis of the actual measurement value of each sample solution and the theoretical value thereof. Fig. 7(2) shows a relationship between the actual measurement value (■) of the peak area of the fraction of Complex 1 or the actual measurement value (♦) of the peak area of the fraction of Complex 2 obtained for each sample solution and the theoretical value of the sample solution.

Fig. 8 shows the results of comparing the ratios of the amount of α(2,3) free PSA to the amount of free PSA between Pca patients and BPH patients, obtained in Example 4.

Figs. 9(1) to 9(3) show the results obtained in Example 4, Comparative Example 1, and Comparative Example 2. Fig. 9(1) (upper panel) shows the results of comparing the ratios of the amount of α(2,3) free PSA to the amount of free PSA between Pca patients and BPH patients, obtained in Example 4. Fig. 9(2) (upper panel) shows the results of comparing total PSA values (total PSA) between Pca patients and BPH patients, obtained in Comparative Example 1. Fig. 9(3) (upper panel) shows the results of comparing the ratios (%fPSA) of a free PSA value to a total PSA value between Pca patients and BPH patients, obtained in Comparative Example 2. In addition, the lower panels of Figs. 9(1) to 9(3) show the results of ROC analysis obtained on the basis of the respective measurement results.

Fig. 10 is a ROC curve obtained in Example 5.

Figs. 11(1) and 11(2) show the results obtained in Example 7 and Comparative Examples 3 and 4. Fig. 11(1) shows the results of comparing the ratios of the amount of α(2,3) free PSA to the amount of free PSA between Pca patients and BPH patients, obtained in Example 7. Fig. 11(2) shows the results of ROC analysis obtained in Example 7 and Comparative Examples 3 and 4. (a) shows the results of analyzing the ratio of the amount of α(2,3) free PSA to the amount of free PSA obtained in Example 7, (b) shows the results of analyzing the amount of α(2,3) free PSA obtained in Comparative Example 3, and (c) shows the results of analyzing the total PSA value obtained in Comparative Example 4.

Figs. 12(1) and 12(2) show the results obtained in Comparative Examples 3 and 4. Fig. 12(1) shows the results of comparing the total PSA values between Pca patients and BPH patients, obtained in Comparative Example 3, and Fig. 12(2) shows the results of comparing the amounts of α(2,3) free PSA (MFI) between Pca patients and BPH patients, obtained in Comparative Example 4.

Fig. 13 shows the results of comparing the ratios of the amount of α(2,3) free PSA to the amount of free PSA between Pca patients and non-cancer subjects, obtained in Example 8.

Fig. 14 shows the results of ROC analysis obtained in Example 8. (1) of Fig. 14 shows the results of analyzing the ratio of the amount of α(2,3) free PSA to the amount of free PSA. (2) of Fig. 14 shows the results of analyzing the total PSA value.

Fig. 15 shows the results of the test of cutoff values on the basis of the results of ROC analysis obtained in Example 8.

Figs. 16(1) and 16(2) show the results obtained in Example 9 and Comparative Example 5. Fig. 16(1) shows the relationship between the Gleason score and the "ratio of the amount of α(2,3) free PSA to the amount of free PSA" obtained in Example 9. Fig. 16(2) shows the relationship between the Gleason score and the total PSA value obtained in Comparative Example 5.

Fig. 17 shows the results of the test of cutoff values on the basis of the results of ROC curve analysis obtained in Example 9.

Figs. 18(1) and 18(2) show the results obtained in Example 10 and Comparative Example 6. Fig. 18(1) shows the results of comparing the ratios of the amount of α(2,3) free PSA to the amount of free PSA between Pca patients and BPH patients, obtained in Example 10. Fig. 18(2) shows the results of comparing the amounts (MFI) of α(2,3) free PSA between Pca patients and non-cancer subjects, obtained in Comparative Example 6.

Fig. 19 is a sensorgram obtained by a surface plasmon resonance method carried out in Example 11.

Description of Embodiments

<Regarding PSA according to present invention>

The "complexed PSA" according to the present invention refers to PSA commonly referred to as "complexed PSA", that is, "PSA that forms a complex by binding to a binding protein such as α1-antichymotrypsin or α2-macroglobulin".

The "free PSA" according to the present invention refers to PSA commonly referred to as "free PSA", that is, "PSA not bound to a binding protein such as α1-antichymotrypsin or α2-macroglobulin".

The "glycan in which a terminal sialic acid residue of the glycan is α(2,3)-linked to a second galactose residue from the terminal of the glycan" according to the present invention refers to a glycan in which the terminal (non-reducing terminal) of the glycan has a structure of Siaα2→3 Gal. The terminal sialic acid residue may be, for example, *N*-acetylneuraminic acid.

The "glycan in which the terminal sialic acid residue of the glycan is α(2,3)-linked to the second galactose residue from the terminal of the glycan" according to the present invention specifically refers to the following structure.

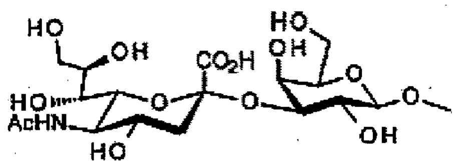

An example of the structure of the glycan of PSA having the "glycan in which the terminal sialic acid residue of the glycan is α(2,3)-linked to the second galactose residue from the terminal of the glycan" according to the present invention is represented by the following formula (I).

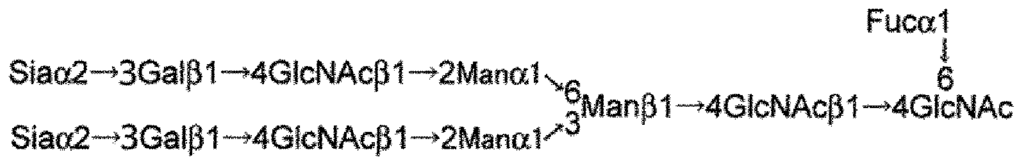 (I)

In addition, an example of PSA having a glycan of the formula (I) is shown in a schematic diagram in Fig. 1. In Fig. 1, the terminal sialic acid residue of the glycan is α(2,3)-linked to the second galactose residue from the terminal of the glycan. In addition, in Fig. 1, the glycan is linked to the asparagine residue (N) of the amino acid sequence of isoleucine-arginine-asparagine-lysine (IRNK) of the PSA protein.

The PSA having a glycan in which the terminal sialic acid residue of the glycan is α(2,3)-linked to the second galactose residue from the terminal of the glycan according to the present invention appears in sera of Pca patients (Non-Patent Literature 5).

The "free PSA having a glycan in which the terminal sialic acid residue of the glycan is α(2,3)-linked to the second galactose residue from the terminal of the glycan" according to the present invention refers to free PSA having a glycan in which the terminal sialic acid residue of the glycan is α(2,3)-linked to the second galactose residue from the terminal of the glycan. Hereinafter, it is abbreviated as "α(2,3) free PSA".

In addition, the "free PSA having a glycan in which the terminal sialic acid residue of the glycan is α(2,6)-linked to the second galactose residue from the terminal of the glycan" according to the present invention refers to free PSA having a glycan in which the terminal sialic acid residue of the glycan is α(2,6)-linked to the second galactose residue from the terminal of the glycan. Hereinafter, it is abbreviated as "α(2,6) free PSA".

<Pca determination method of present invention>

The Pca determination method of the present invention is "a prostate carcinoma determination method, including determining the ratio of the amount of α(2,3) free PSA to the amount of free PSA in a biological sample (hereinafter, also simply referred to as "sample"), and determining that prostate carcinoma is developed or the probability of developing prostate carcinoma is high in the case where the ratio is higher than 40%."

The amount of free PSA according to the present invention is a total amount of the amount of α(2,3) free PSA and the amount of free PSA other than α(2,3) free PSA in the sample.

The "free PSA other than α(2,3) free PSA" according to the present invention refers to free PSA without a glycan in which the terminal sialic acid residue of the glycan is α(2,3)-linked to the second galactose residue from the terminal of the glycan according to the present invention.

The Pca determination method of the present invention is preferably a "prostate carcinoma determination method, including measuring the amount of free PSA and the amount of α(2,3) free PSA in a biological sample, determining the ratio of the amount of α(2,3) free PSA to the amount of free PSA obtained, and determining that prostate carcinoma is developed or the probability of developing prostate carcinoma is high in the case where the ratio is 40% or higher".

(1) Method of measuring amount of free PSA

The method of measuring the amount of free PSA in a sample according to the present invention may be, for example,

1) a method of directly measuring the amount of free PSA in a sample, or
2) a method in which the amount of α(2,3) free PSA and the amount of free PSA other than α(2,3) free PSA in a sample are respectively measured, and the sum thereof is taken as the amount of free PSA.

1) Method of directly measuring amount of free PSA in sample

In the case of directly measuring the amount of free PSA, it may be measured by a known method of measuring the amount of free PSA. For example, the amount of free PSA may be measured by a known immunoassay using an anti-free PSA antibody, or an anti-PSA antibody and an anti-free PSA antibody.

The anti-PSA antibody according to the present invention is an antibody having an affinity for PSA, specifically an antibody having an affinity for (binding to) the core protein of PSA. That is, the anti-PSA antibody according to the present invention contains an antibody that binds to both free PSA and complexed PSA and an antibody that specifically binds to free PSA (anti-free PSA antibody). Unless otherwise specified, the case of simply referring to anti-PSA antibody in the present specification includes an antibody that binds to both free PSA and complexed PSA and an antibody that

specifically binds to free PSA.

The type of anti-PSA antibody according to the present invention is not particularly limited, and it may be, for example, a polyclonal antibody or a monoclonal antibody, and these antibodies may be used alone or in combination thereof as appropriate. In addition, the anti-PSA antibody may also be Fab, $F(ab')_2$, Fab' fragments, or antibody variable regions of these antibodies.

A commercially available antibody may be used as the anti-PSA antibody according to the present invention.

Among the anti-PSA antibodies according to the present invention, examples of commercially available products of the antibody having an affinity for both free PSA and complexed PSA include Anti PSA monoclonal antibody PSA10 (Anti PSA monoclonal antibody clone No. PSA10, Wako Pure Chemical Industries, Ltd.), Anti PSA monoclonal antibody (5A6) (HyTest Ltd.), Anti PSA monoclonal antibody (5G6) (HyTest Ltd.), Anti PSA monoclonal antibody (PS6) (HyTest Ltd.), Anti PSA monoclonal antibody (PSA14) (Wako Pure Chemical Industries, Ltd.), Anti-Prostate Specific Antigen antibody (EP1588Y) (Abcam Plc.), Anti-Prostate Specific Antigen antibody (A67-B/E3) (Abcam Plc.), Anti-Prostate Specific Antigen antibody (35H9) (Abcam Plc.), Anti-Prostate Specific Antigen antibody (KLK3/801) (Abcam Plc.), Anti-Prostate Specific Antigen antibody (3E6) (Abcam Plc.), Anti-Prostate Specific Antigen antibody (8301) (Abcam Plc.), Anti-Prostate Specific Antigen antibody (A5D5) (Abcam Plc.), Anti-Prostate Specific Antigen antibody (PSA 28/A4) (Abcam Plc.), and Anti-Prostate Specific Antigen antibody (1H12) (Abcam Plc.).

Among the anti-PSA antibodies according to the present invention, examples of commercially available products of the antibody that specifically binds to free PSA (anti-free PSA antibody) include Anti PSA monoclonal antibody PSA12 (Anti PSA monoclonal antibody clone No. PSA12, Wako Pure Chemical Industries, Ltd.), Anti PSA monoclonal antibody (8A6) (HyTest Ltd.), Anti PSA monoclonal antibody (PS1) (HyTest Ltd.), Anti PSA monoclonal antibody (clone 108) (Anogen-Yes Biotech Laboratories Ltd.), Anti-Prostate Specific Antigen antibody (PS2) (Abcam Plc.), and Anti-Prostate Specific Antigen antibody (2H9) (Abcam Plc.).

The anti-PSA antibody (containing anti-free PSA antibody) according to the present invention may be labeled with a detectable labeling substance. The labeling substance used for labeling the antibody may be the same as the labeling substance for labeling an "antibody used as an affinity substance according to the present invention", which will be described later. In addition, the labeling method of the antibody may be the same method as the method of labeling an antibody used as an affinity substance according to the present invention, which will be described later.

The amount of free PSA may be measured by using a commercially available kit for measurement of free PSA (for example, Human Circulating Cancer BioMarker Panel 1 Select Kit (manufactured by LUMINEX Corporation)), free PSA·Abbott (Abbott Laboratories, Inc.), Lumipulse Free PSA (Fujirebio, Inc.), Vitros free PSA (Ortho Clinical Diagnostics, Inc.), ST AIA-PACK free PSA (Tosoh Corporation), or ECLusys™ reagent free PSA (Roche Diagnostics K.K.).

2) Method in which amount of α(2,3) free PSA and amount of free PSA other than α(2,3) free PSA in sample are respectively measured, and total amount thereof is taken as amount of free PSA

The method may be, for example, the method described in the section "Method of measuring amount of α(2,3) free PSA, or amount of α(2,3) free PSA and amount of free PSA other than α(2,3) free PSA" which will be described later.

<u>(2) Method of measuring amount of α(2,3) free PSA, or amount of α(2,3) free PSA and amount of free PSA other than α(2,3) free PSA</u>

In the determination method of the present invention, the "method of measuring the amount of α(2,3) free PSA" contains a method of measuring "the amount of α(2,3) free PSA", or a "method of measuring the amount of α(2,3) free PSA and the amount of free PSA other than α(2,3) free PSA".

In addition, in the case of measuring the amount of α(2,3) free PSA, it is preferable to carry out the measurement by a measuring method by which the efficiency of capturing α(2,3) free PSA is 80% or more.

In the present invention, the efficiency of capturing α(2,3) free PSA refers to:

- an efficiency for capturing and detecting (measuring) free PSA having a glycan (α2,3 sialyl glycan) in which the terminal sialic acid residue of the target glycan is α(2,3)-linked to the second galactose residue from the terminal of the glycan, from the free PSA having various glycan-modified isomers coexisting in a sample, or
- an efficiency for detecting (measuring) free PSA having a glycan (α2,3 sialyl glycan) in which the terminal sialic acid residue of the target glycan is α(2,3)-linked to the second galactose residue from the terminal of the glycan, from the free PSA having various glycan-modified isomers coexisting in a sample.

In other words, it can be said that the efficiency of capturing α(2,3) free PSA according to the present invention is the ratio of final detectable α(2,3) free PSA to the total amount of free PSA present in a sample.

The efficiency of capturing α(2,3) free PSA according to the present invention is 80% or more, preferably 90%

or more, more preferably 95% or more, still more preferably 98% or more, and particularly preferably 100%.

The efficiency of capturing α(2,3) free PSA according to the present invention can be confirmed by the method which will be described below. However, it is not necessary to confirm the capture efficiency every time the amount of α(2,3) free PSA or the like is measured.

The "method of measuring the amount of α(2,3) free PSA, or the amount of α(2,3) free PSA and the amount of free PSA other than α(2,3) free PSA" may be, for example,

1) a method of measuring the amount of α(2,3) free PSA, or the amount of α(2,3) free PSA and the amount of free PSA other than α(2,3) free PSA, using a substance having an affinity for a glycan in which the terminal sialic acid residue of the glycan is α(2,3)-linked to the second galactose residue from the terminal of the glycan (hereinafter, abbreviated as "affinity substance"), and utilizing the interaction between the glycan in which the terminal sialic acid residue of the glycan of α(2,3) free PSA is α(2,3)-linked to the second galactose residue from the terminal of the glycan and the affinity substance, or

2) a method of measuring the amount of α(2,3) free PSA, or the amount of α(2,3) free PSA and the amount of free PSA other than α(2,3) free PSA, without using an affinity substance.

1) Method of measuring amount of α(2,3) free PSA, or amount of α(2,3) free PSA and amount of free PSA other than α(2,3) free PSA, utilizing the interaction between glycan in which terminal sialic acid residue of glycan of α(2,3) free PSA is α(2,3)-linked to second galactose residue from terminal of glycan and affinity substance

Affinity substance

The affinity substance used in the method is a substance having an affinity for (binding to) a glycan in which the terminal sialic acid residue of the glycan is α(2,3)-linked to the second galactose residue from the terminal of the glycan. Preferred is a substance having a specific affinity for (specifically binding to) a glycan in which the terminal sialic acid residue of the glycan is α(2,3)-linked to the second galactose residue from the terminal of the glycan. Particularly preferred is a substance having an affinity for (binding to) a glycan in which the terminal sialic acid residue of the glycan is α(2,3)-linked to the second galactose residue from the terminal of the glycan, but having no affinity for (not binding to) other glycans (for example, a glycan in which the terminal sialic acid residue of the glycan is α(2,6)-linked to the second galactose residue from the terminal of the glycan).

The affinity substance according to the present invention may be, for example, a lectin or antibody having such properties. Hereinafter, lectins and antibodies as affinity substances will be described in more detail.

Affinity substance: lectin

The lectin used as the affinity substance according to the present invention may be, for example, a lectin having an affinity for (binding to) a glycan in which the terminal sialic acid residue of the glycan is α(2,3)-linked to the second galactose residue from the terminal of the glycan. Preferred is a lectin having a specific affinity for (specifically binding to) a glycan in which the terminal sialic acid residue of the glycan is α(2,3)-linked to the second galactose residue from the terminal of the glycan. Particularly preferred is a lectin having an affinity for (binding to) a glycan in which the terminal sialic acid residue of the glycan is α(2,3)-linked to the second galactose residue from the terminal of the glycan, but having no affinity for (not binding to) other glycans (for example, a glycan in which the terminal sialic acid residue of the glycan is α(2,6)-linked to the second galactose residue from the terminal of the glycan).

Examples of the lectin having such properties include plant lectins such as MAA which is a lectin derived from *Maackia amurensis,* and ACG which is a lectin derived from *Agrocybe cylindracea,* and animal lectins such as CD169 (sialic acid-binding Ig-like lectin 1), rat MAG, CD328, and siglec-9 (sialic acid-binding Ig-like lectin-9). Among them, MAA is preferable.

The lectin may be labeled with a detectable labeling substance.

Examples of the labeling substance used for labeling a lectin include a fluorescent dye (fluorescein isothiocyanate (FITC), Cy5, Alexa Fluor 647, or the like), an enzyme (horseradish-derived peroxidase), a coenzyme, a chemiluminescent substance, a radioactive substance ($^{32}P$, $^{14}C$, $^{125}I$, $^{3}H$, $^{131}I$, or the like), and a labeling substance such as biotin. In addition, the labeling substance may be bound directly to a lectin, or may be bound to a lectin through a suitable spacer. The lectin may be labeled by a per se known labeling method corresponding to the type of the labeling substance.

Affinity substance: antibody

The antibody used as the affinity substance according to the present invention may be, for example, an antibody having a specific affinity for (binding to) a glycan in which the terminal sialic acid residue of the glycan is α(2,3)-linked

to the second galactose residue from the terminal of the glycan. Preferred is an antibody having a specific affinity for (specifically binding to) a glycan in which the terminal sialic acid residue of the glycan is α(2,3)-linked to the second galactose residue from the terminal of the glycan. Particularly preferred is an antibody having an affinity for (binding to) a glycan in which the terminal sialic acid residue of the glycan is α(2,3)-linked to the second galactose residue from the terminal of the glycan, but having no affinity for (not binding to) other glycans (for example, a glycan in which the terminal sialic acid residue of the glycan is α(2,6)-linked to the second galactose residue from the terminal of the glycan).

The type of antibody used as an affinity substance according to the present invention is not particularly limited, and it may be, for example, a polyclonal antibody or a monoclonal antibody, and these antibodies may be used alone or in combination thereof as appropriate. In addition, the antibody may also be Fab, $F(ab')_2$, Fab' fragments, or variable regions of these antibodies.

A commercially available antibody may be used as the antibody which is used as the affinity substance according to the present invention.

Examples of commercially available products of the antibody used as the affinity substance according to the present invention, that is, the antibody having an affinity for a glycan in which the terminal sialic acid residue of the glycan is α(2,3)-linked to the second galactose residue from the terminal of the glycan, include Anti Siaα2-3, Monoclonal Antibody (HYB4) (manufactured by Wako Pure Chemical Industries, Ltd.), Anti GM3(Neu Ac), Monoclonal Antibody (Clone: M2590) (manufactured by Wako Pure Chemical Industries, Ltd.), Anti-sialyl Lea antigen, Monoclonal Antibody (MSW113) (manufactured by Wako Pure Chemical Industries, Ltd.), Anti-GM3 Monoclonal Antibody (Tokyo Chemical Industry Co., Ltd.), Anti-sialyl Lewis A Monoclonal Antibody (1H4) (Tokyo Chemical Industry Co., Ltd.), and Anti-sialyl Lewis A Monoclonal Antibody (NKH3) (GlycoNex Inc.).

The antibody used as the affinity substance according to the present invention may be labeled with a detectable labeling substance. Examples of the labeling substance used for labeling the antibody include enzymes as used in EIA (ELISA, Enzyme-Linked Immunosorbent Assay), for example, alkaline phosphatase, β-galactosidase, peroxidase, microperoxidase, glucose oxidase, glucose-6-phosphate dehydrogenase, acetylcholinesterase, malate dehydrogenase, and luciferase; radioisotopes as used in radioimmunoassay (RIA), for example, $^{99m}Tc$, $^{131}I$, $^{125}I$, $^{14}C$, and $^{3}H$; fluorescent substances, for example, HiLyte 647 (manufactured by AnaSpec, Inc.), fluorescein, dansyl, fluorescamine, coumarin, naphthylamine, and derivatives thereof; luminescent substances, for example, luciferin, isoluminol, luminol, and bis(2,4,6-trifluorophenyl)oxalate; substances having absorption in ultraviolet region, for example, phenol, naphthol, anthracene, and derivatives thereof; and substances having a property as a spin-labeling agent represented by a compound having an oxyl group, for example, 4-amino-2,2,6,6-tetramethylpiperidine-1-oxyl, 3-amino-2,2,5,5-tetramethylpyrrolidine-1-oxyl, and 2,6-di-t-butyl-α-(3,5-di-t-butyl-4-oxo-2,5-cyclohexadien-1-ylidene)-p-tolyloxyl.

In order to bind the labeling substance to the antibody (or in order to label the antibody with the labeling substance), for example, a labeling method known per se which is used commonly in EIA, RIA, or FIA known per se (for example, the method described in "Course on Experimental Medical Chemistry", vol. 8, supervised by Yuichi Yamamura, the 1st edition, Nakayama Shoten Co., Ltd., 1971; "Illustrative Description of Fluorescent Antibody", Akira Kawaoi, the 1st edition, Soft Science Inc., 1983; "Enzyme Immunoassay", Eiji Ishikawa, Tadashi Kawai, Kiyoshi Muroi ed, the 2nd edition, Igaku-Shoin Ltd., 1982; and the like) may be appropriately used. In addition, it goes without saying that a conventional method utilizing the reaction of avidin (or streptavidin) and biotin may be used as a labeling method.

In the method 1), that is, the method of measuring the amount of α(2,3) free PSA, or the amount of α(2,3) free PSA and the amount of free PSA other than α(2,3) free PSA, utilizing the interaction between a glycan in which a terminal sialic acid residue of the glycan of α(2,3) free PSA is α(2,3)-linked to a second galactose residue from the terminal of the glycan and an affinity substance, the efficiency of capturing α(2,3) free PSA is preferably 80% or more.

The efficiency of capturing α(2,3) free PSA in the method is an "efficiency for capturing and detecting (measuring) free PSA having a glycan (α2,3 sialyl glycan) in which the terminal sialic acid residue of the target glycan is α(2,3)-linked to the second galactose residue from the terminal of the glycan, from the free PSA proteins having various glycan-modified isomers coexisting in a sample".

In other words, it can also be said that the efficiency of capturing α(2,3) free PSA in the method is the ratio of final detectable α(2,3) free PSA to the total amount of α(2,3) free PSA present in a sample.

In the method, for example, the following method can be mentioned as a method of determining the efficiency of capturing α(2,3) free PSA.

The measurement of α(2,3) free PSA is carried out using an affinity substance and α(2,3) free PSA of known concentration having only a glycan in which the terminal sialic acid residue of the glycan is α(2,3)-linked to the second galactose residue from the terminal of the glycan "(hereinafter, referred to as "α(2,3) free PSA standard"). The capture efficiency can be obtained by determining the ratio of the measurement value of the amount of α(2,3) free PSA to the amount of α(2,3) free PSA used for the measurement as a sample.

Alternatively, the measurement of α(2,3) free PSA is carried out using an affinity substance and an α(2,3) free PSA standard labeled with a detectable labeling substance. Next, the measurement of α(2,3) free PSA labeled with a detectable labeling substance is carried out in the same manner without using an affinity substance. The capture efficiency

can be obtained by determining the ratio of the measurement result obtained using an affinity substance to the measurement result obtained without using an affinity substance.

As a specific example of the method of determining the capture efficiency, for example, the following method can be mentioned. That is, an affinity substance is reacted with an α(2,3) free PSA standard (of known concentration). After the reaction, the fraction in which the α(2,3) free PSA and the affinity substance were complexed and the unreacted fraction were separated, and the amount of the target glycan in each fraction was analyzed with a mass spectrometer. The capture efficiency can be obtained by determining (calculating) (the amount of target glycan of complex formed fraction) / (amount of target glycan of complex formed fraction+amount of target glycan of unreacted fraction).

As a method for preparing an α(2,3) free PSA standard, for example, the method described in Example 2(1) described below can be mentioned. That is, r free PSA (containing r α(2,3) free PSA and r α(2,6) free PSA) is obtained according to the method disclosed in, for example, Non-Patent Literature 6, 2. Materials and methods (2.7 Forced expression of FLAG-tag-fused S2,3PSA). From the r free PSA thus obtained, r α(2,3) free PSA and r α(2,6) free PSA are separated and purified by a known method. For example, first, r α(2,3) free PSA and r α(2,6) free PSA are separated by lectin column chromatography using a lectin showing a high affinity for the sialyl α2,3-galactose structure. Next, gel filtration is carried out to purify r α(2,3) free PSA and r α(2,6) free PSA, respectively. The resulting purified r α(2,3) free PSA can be used as an "α(2,3) free PSA standard". In addition, the resulting purified r α(2,6) free PSA can be used as an "α(2,6) free PSA standard" as necessary.

It should be noted that it is not necessary to confirm the capture efficiency every time the amount of α(2,3) free PSA or the like is measured. Once it is confirmed that the efficiency of capturing α(2,3) free PSA is 80% or more, preferably 90% or more, more preferably 95% or more, still more preferably 98% or more, and particularly preferably 100% in the case where the amount of α(2,3) free PSA, or the amount of α(2,3) free PSA and the amount of free PSA other than α(2,3) free PSA are measured using a certain affinity substance, it is not necessary to confirm the capture efficiency again in the case of measuring the amount of α(2,3) free PSA using the affinity substance.

The efficiency of capturing α(2,3) free PSA in the method of measuring α(2,3) free PSA according to the method 1) is 80% or more, preferably 90% or more, more preferably 95% or more, still more preferably 98% or more, and particularly preferably 100%.

The "method of measuring amount of α(2,3) free PSA, or amount of α(2,3) free PSA and amount of free PSA other than α(2,3) free PSA, utilizing interaction between glycan in which terminal sialic acid residue of glycan of α(2,3) free PSA is α(2,3)-linked to second galactose residue from terminal of glycan and an affinity substance" according to the present invention may be, for example,
a "method of forming a complex of α(2,3) free PSA with an affinity substance by the interaction between the glycan in which the terminal sialic acid residue of the glycan of α(2,3) free PSA is α(2,3)-linked to the second galactose residue from terminal of the glycan and the affinity substance; measuring the amount of the complex; and on the basis of the results, measuring the amount of α(2,3) free PSA, or the amount of α(2,3) free PSA and the amount of free PSA other than α(2,3) free PSA".

In the method, the efficiency of capturing α(2,3) free PSA is preferably 80% or more.

In the method, the "method of forming a complex of α(2,3) free PSA with an affinity substance by the interaction between the glycan in which the terminal sialic acid residue of the glycan of α(2,3) free PSA is α(2,3)-linked to the second galactose residue from terminal of the glycan and the affinity substance" may be, for example, a "method of forming a complex of α(2,3) free PSA with the affinity substance by the affinity of the affinity substance for the glycan in which the terminal sialic acid residue of the glycan of α(2,3) free PSA is α(2,3)-linked to the second galactose residue from terminal of the glycan". The specific method varies depending on the method of measuring the complex that is subsequently carried out. For each method of measuring the complex to be carried out, an optimum method may be selected and carried out as appropriate.

A more preferred example of the method may be, for example, a "method of forming a complex of α(2,3) free PSA with the affinity substance by the interaction between the glycan in which the terminal sialic acid residue of the glycan of α(2,3) free PSA is α(2,3)-linked to the second galactose residue from terminal of the glycan and the affinity substance; measuring the amount of the complex without removing components other than the complex from the system; and on the basis of the results, measuring the amount of α(2,3) free PSA, or the amount of α(2,3) free PSA and the amount of free PSA other than α(2,3) free PSA".

The "method of measuring the amount of the complex (complex of α(2,3) free PSA with an affinity substance) without removing components other than the complex from the system" refers to a method of measuring the amount of the complex of α(2,3) free PSA with an affinity substance by a homogeneous method such a metnod without carrying out so-called B/F separation. For example, a method of measuring the amount of the complex without performing a washing operation which is normally carried out in order to remove components other than the complex from the measurement system can be mentioned.

Among the methods, preferred is a "method of forming a complex of α(2,3) free PSA with an affinity substance by the interaction between the glycan in which the terminal sialic acid residue of the glycan of α(2,3) free PSA is

α(2,3)-linked to the second galactose residue from terminal of the glycan and the affinity substance; measuring the amount of the complex without removing components other than the complex from the system (without performing a washing operation which is normally carried out in order to remove components other than the complex from the measurement system); and on the basis of the results, measuring the amount of α(2,3) free PSA, or the amount of α(2,3) free PSA and the amount of free PSA other than α(2,3) free PSA".

In addition, in the method, the efficiency of capturing α(2,3) free PSA is preferably 80% or more.

The affinity substance used for the method of measuring the amount of α(2,3) free PSA, or the amount of α(2,3) free PSA and the amount of free PSA other than α(2,3) free PSA according to the present invention should be in an amount sufficient to measure 80% or more of α(2,3) free PSA in a sample.

That is, at the time of measuring α(2,3) free PSA, it is necessary to use a sufficient amount of an affinity substance capable of forming a complex with α(2,3) free PSA which can measure the amount of α(2,3) free PSA in a sample by 80% or more.

The amount of such an affinity substance to be used is determined taking into account the binding constant of the affinity substance for α(2,3) free PSA and the amount of α(2,3) free PSA in a sample.

In the case of using an affinity substance that has a strong affinity for α(2,3) free PSA and does not re-dissociate in the case where it binds to α(2,3) free PSA, a sufficient amount of affinity substance is used so that 80% or more of α(2,3) free PSA in a sample forms a complex with the affinity substance, at the time of measurement.

In the case of using an affinity substance that does not have a sufficient affinity for α(2,3) free PSA and re-dissociates even in the case where once it binds to α(2,3) free PSA, it is necessary to use a sufficient amount of affinity substance so that 80% or more of α(2,3) free PSA in a sample forms a complex with the affinity substance, at the time of measurement even after re-dissociation.

The amount of the affinity substance used to satisfy the conditions is preferably an excess amount (saturated amount) of affinity substance with respect to α(2,3) free PSA in a sample.

It should be noted that, in the method 1), that is, the method of measuring the amount of α(2,3) free PSA, or the amount of α(2,3) free PSA and the amount of free PSA other than α(2,3) free PSA, utilizing the interaction between a glycan in which a terminal sialic acid residue of the glycan of α(2,3) free PSA is α(2,3)-linked to a second galactose residue from the terminal of the glycan and an affinity substance, the method of measuring only α(2,3) free PSA may be, for example, a method of measuring only α(2,3) free PSA by detecting only a complex of a labeled affinity substance with α(2,3) free PSA without detecting free PSA other than α(2,3) free PSA, using a labeled affinity substance in which an affinity substance is labeled with a detectable labeling substance. The method of measuring both the amount of α(2,3) free PSA and the amount of free PSA other than α(2,3) free PSA, may be, for example, a method of measuring both α(2,3) free PSA and free PSA other than α(2,6) free PSA by separating α(2,3) free PSA and free PSA other than α(2,6) free PSA utilizing the interaction between the α(2,3) free PSA and the affinity substance, and then measuring PSA using an anti-PSA antibody or the like.

Hereinafter, specific examples of the method of measuring only the amount of α(2,3) free PSA and the method of measuring both the amount of α(2,3) free PSA and the amount of free PSA other than α(2,3) free PSA will be described.

In the following specific examples, the "glycan in which the terminal sialic acid residue of the glycan is α(2,3)-linked to the second galactose residue from the terminal of the glycan" may be referred to as "target glycan" for the sake of convenience.

(a) Method of measuring only amount of α(2,3) free PSA

Method 1

1) a step of bringing a biological sample, a first antibody labeled with a labeling substance and having an affinity for a glycan in which the terminal sialic acid residue of the glycan is α(2,3)-linked to the second galactose residue from the terminal of the glycan, a second antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of free PSA other than α(2,3) free PSA and the second antibody,
2) optionally, a step of separating the first complex and the second complex obtained in 1), and
3) a step of measuring the amount of the first complex by measuring the signal derived from the labeling substance of the labeled antibody constituting the complex, and on the basis of the measurement value, determining the amount of α(2,3) free PSA.

The second antibody used in Method 1 may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

Method 2

1) a step of bringing a biological sample, a first antibody (affinity substance) having an affinity for a glycan in which the terminal sialic acid residue of the glycan is α(2,3)-linked to the second galactose residue from the terminal of the glycan and labeled with a labeling substance, and a second antibody having an affinity for PSA into contact with each other to form a complex (first complex) of the labeled first antibody, α(2,3) free PSA and the second antibody, and a complex (second complex) of free PSA other than α(2,3) free PSA and the second antibody,
2) optionally, a step of separating the first complex and the second complex obtained in the step 1), and
3) a step of measuring the amount of the first complex by measuring the signal derived from the labeling substance of the labeled first antibody constituting the complex, and on the basis of the measurement value, determining the amount of α(2,3) free PSA.

The second antibody used in Method 2 may be an antibody that specifically binds to free PSA.

In addition, the first complex and the second complex may be separated on the basis of the affinity of the first antibody for the target glycan or may be separated on the basis of the difference in mass between the first complex and the second complex (for example, electrophoresis).

Method 3

1) a step of bringing a biological sample, a first antibody (affinity substance) having an affinity for a glycan in which the terminal sialic acid residue of the glycan is α(2,3)-linked to the second galactose residue from the terminal of the glycan and labeled with a labeling substance, a second antibody having an affinity for PSA, and a third antibody specifically binding to free PSA into contact with each other to form a complex (first complex) of the labeled first antibody, α(2,3) free PSA, the second antibody and the third antibody, and a complex (second complex) of free PSA other than α(2,3) free PSA, the second antibody and the third antibody,
2) optionally, a step of separating the first complex and the second complex obtained in the step 1), and
3) a step of measuring the amount of the first complex by measuring the signal derived from the labeling substance of the labeled first antibody constituting the complex, and on the basis of the measurement value, determining the amount of α(2,3) free PSA.

In addition, the first complex and the second complex may be separated on the basis of the affinity of the first antibody for the target glycan or may be separated on the basis of the difference in mass between the first complex and the second complex (for example, electrophoresis).

In the case of Methods 1, 2, and 3, the amount of free PSA may be determined by carrying out the method of directly determining an amount of free PSA. In addition, the amount of α(2,3) free PSA may be determined by conversion into quantitative values by a conventional method using the results obtained by carrying out the measurement in the same manner using an α(2,3) free PSA standard of known concentration in advance.

(b) Method of measuring both amount of α(2,3) free PSA and amount of free PSA other than α(2,3) free PSA

Method 4

1) a step of bringing a biological sample, an antibody (labeled anti-PSA antibody) having an affinity for PSA and labeled with a labeling substance, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled anti-PSA antibody, α(2,3) free PSA and the lectin, and a complex (second complex) of the labeled anti-PSA antibody and free PSA other than α(2,3) free PSA,

2) a step of separating the first complex and the second complex obtained in the step 1),
3) a step of measuring the signal derived from the labeling substance of the labeled anti-PSA antibody constituting the complex, thereby measuring the amount of the first complex and the amount of the second complex separated in the step 2), and
4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

The antibody related to an antibody having an affinity for PSA and labeled with a labeling substance used in Method 4 may be an antibody that specifically binds to free PSA.
In addition, the first complex and the second complex may be separated on the basis of the affinity of the lectin for the target glycan or may be separated on the basis of the difference in mass between the first complex and the second complex (for example, electrophoresis).

Method 5

1) a step of bringing a biological sample, a first antibody having an affinity for PSA and labeled with a labeling substance, a second antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of the labeled first antibody, free PSA other than α(2,3) free PSA and the second antibody,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

Examples of combinations of the first antibody and the second antibody used in Method 5 include the following combinations. However, it is preferred that the epitopes of the first antibody and the second antibody are different.

- The first antibody and the second antibody are antibodies having an affinity for PSA.
- The first antibody is an antibody having an affinity for PSA and the second antibody is an antibody which specifically binds to free PSA.
- The first antibody and the second antibody are antibodies that specifically bind to free PSA.
- The first antibody is an antibody specifically binding to free PSA and the second antibody is an antibody having an affinity for PSA.

In addition, the first complex and the second complex may be separated on the basis of the affinity of the lectin for the target glycan or may be separated on the basis of the difference in mass between the first complex and the second complex (for example, electrophoresis).

Method 6

1) a step of bringing a biological sample, a first antibody having an affinity for PSA and labeled with a labeling substance, a second antibody having an affinity for a glycan in which the terminal sialic acid residue of the glycan is α(2,3)-linked to the second galactose residue from the terminal of the glycan, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of the labeled first antibody and free PSA other than α(2,3) free PSA,
2) a step of separating the first complex and the second complex obtained in the step 1),

3) a step of measuring the signal derived from the labeling substance of the labeled first antibody constituting the complex, thereby measuring the amount of the first complex and the amount of the second complex separated in the step 2), and

4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

Examples of combinations of the first antibody and the second antibody used in Method 6 include the following combinations. However, it is preferred that the epitopes of the first antibody and the second antibody are different.

- The first antibody is an antibody having an affinity for PSA and the second antibody is an antibody having an affinity for a glycan in which the terminal sialic acid residue of the glycan is α(2,3)-linked to the second galactose residue from the terminal of the glycan.
- The first antibody is an antibody that specifically binds to free PSA, and the second antibody is an antibody having an affinity for a glycan in which the terminal sialic acid residue of the glycan is α(2,3)-linked to the second galactose residue from the terminal of the glycan.

In addition, the first complex and the second complex may be separated on the basis of the affinity of the lectin for the target glycan or may be separated on the basis of the difference in mass between the first complex and the second complex (for example, electrophoresis).

Method 7 (Method without using labeled antibody)

1) a step of bringing a biological sample, an antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the antibody, α(2,3) free PSA and the lectin, and a complex (second complex) of the antibody and free PSA other than α(2,3) free PSA,

2) a step of separating the first complex and the second complex obtained in the step 1),

3) a step of measuring the amount of the first complex and the amount of the second complex separated in the step 2), and

4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

The antibody having an affinity for PSA used in Method 7 may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method 8 (Method without using labeled antibody)

1) a step of bringing a biological sample, a first antibody having an affinity for PSA, a second antibody specifically binding to free PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the first antibody, α(2,3) free PSA, the second antibody and the lectin, and a complex (second complex) of the first antibody, free PSA other than α(2,3) free PSA and the second antibody,

2) a step of separating the first complex and the second complex obtained in the step 1),

3) a step of measuring the amount of the first complex and the amount of the second complex separated in the step 2), and

4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

The first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method 9 (Method without using labeled antibody)

1) a step of bringing a biological sample, a first antibody having an affinity for PSA, a second antibody having an affinity for a glycan in which the terminal sialic acid residue of the glycan is α(2,3)-linked to the second galactose residue from the terminal of the glycan, and a lectin (affinity substance) having an affinity for a glycan in which the terminal sialic acid residue of the glycan is α(2,3)-linked to the second galactose residue from the terminal of the glycan into contact with each other to form a complex (first complex) of the first antibody, α(2,3) free PSA, the second antibody and the lectin, and a complex (second complex) of the first antibody and free PSA other than α(2,3) free PSA,

2) a step of separating the first complex and the second complex obtained in the step 1),

3) a step of measuring the amount of the first complex and the amount of the second complex separated in the step 2), and

4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

The first antibody used in Method 9 may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method 10

1) a step of bringing a biological sample, a first antibody having an affinity for PSA and labeled with a labeling substance, and a second antibody (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA and the second antibody, and a complex (second complex) of the labeled first antibody and free PSA other than α(2,3) free PSA,

2) a step of separating the first complex and the second complex obtained in the step 1),

3) a step of measuring the signal derived from the labeling substance of the labeled first antibody constituting the complex, thereby measuring the amount of the first complex and the amount of the second complex separated in the step 2), and

4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

The first antibody used in Method 10 may be an antibody that specifically binds to free PSA.

In addition, the first complex and the second complex may be separated on the basis of the affinity of the second antibody for the target glycan or may be separated on the basis of the difference in mass between the first complex and the second complex (for example, electrophoresis).

Method 11

1) a step of bringing a biological sample, a first antibody having an affinity for PSA and labeled with a labeling substance, a second antibody having an affinity for PSA, and a third antibody (affinity substance) having an affinity for a glycan in which the terminal sialic acid residue of the glycan is $\alpha$(2,3)-linked to the second galactose residue from the terminal of the glycan into contact with each other to form a complex (first complex) of the labeled first antibody, $\alpha$(2,3) free PSA, the second antibody and the third antibody, and a complex (second complex) of the labeled first antibody, free PSA other than $\alpha$(2,3) free PSA and the second antibody,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), taking the amount of the first complex as the amount of $\alpha$(2,3) free PSA, and taking the sum of the amount of the first complex and the amount of the second complex as the amount of free PSA.

Examples of combinations of the first antibody and the second antibody used in Method 11 include the following combinations. However, it is preferred that the epitopes of the first antibody and the second antibody are different.

- The first antibody and the second antibody are antibodies having an affinity for PSA.
- The first antibody is an antibody having an affinity for PSA and the second antibody is an antibody which specifically binds to free PSA.
- The first antibody is an antibody specifically binding to free PSA and the second antibody is an antibody having an affinity for PSA.
- The first antibody and the second antibody are antibodies that specifically bind to free PSA.

In addition, the first complex and the second complex may be separated on the basis of the affinity of the third antibody for the target glycan or may be separated on the basis of the difference in mass between the first complex and the second complex (for example, electrophoresis).

Method 12 (Method without using labeled antibody)

1) a step of bringing a biological sample, a first antibody having an affinity for PSA, and a second antibody (affinity substance) having an affinity for a glycan in which the terminal sialic acid residue of the glycan is $\alpha$(2,3)-linked to the second galactose residue from the terminal of the glycan into contact with each other to form a complex (first complex) of the first antibody, $\alpha$(2,3) free PSA and the second antibody, and a complex (second complex) of the first antibody and free PSA other than $\alpha$(2,3) free PSA,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), taking the amount of the first complex as the amount of $\alpha$(2,3) free PSA, and taking the sum of the amount of the first complex and the amount of the second complex as the amount of free PSA.

The first antibody used in Method 12 may be an antibody that specifically binds to free PSA.

In addition, the first complex and the second complex may be separated on the basis of the affinity of the second antibody for the target glycan or may be separated on the basis of the difference in mass between the first complex and the second complex (for example, electrophoresis).

To measure the amount of the first complex and the amount of the second complex, for example, the amount

of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method 13 (Method without using labeled antibody)

1) a step of bringing a biological sample, a first antibody having an affinity for PSA, a second antibody specifically binding to free PSA, and a third antibody having an affinity for a glycan in which the terminal sialic acid residue of the glycan is α(2,3)-linked to the second galactose residue from the terminal of the glycan into contact with each other to form a complex (first complex) of the first antibody, α(2,3) free PSA, the second antibody and the third antibody (affinity substance), and a complex (second complex) of the first antibody, free PSA other than α(2,3) free PSA and the second antibody,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), taking the amount of the first complex as the amount of α(2,3) free PSA, and taking the sum of the amount of the first complex and the amount of the second complex as the amount of free PSA.

In addition, the first complex and the second complex may be separated on the basis of the affinity of the third antibody for the target glycan or may be separated on the basis of the difference in mass between the first complex and the second complex (for example, electrophoresis).
To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

2) Method of measuring amount of α(2,3) free PSA, or amount of α(2,3) free PSA and amount of free PSA other than α(2,3) free PSA without using affinity substance

In the method, the efficiency of capturing α(2,3) free PSA is preferably 80% or more.
For example, a method using a mass spectrometer can be mentioned.
In addition, the efficiency of capturing α(2,3) free PSA in the method refers to an efficiency for capturing and detecting (measuring) free PSA having a glycan (α2,3 sialyl glycan) in which the terminal sialic acid residue of the target glycan is α(2,3)-linked to the second galactose residue from the terminal of the glycan, from the free PSA proteins having various glycan-modified isomers coexisting in a sample.
In other words, it can be said that the efficiency of capturing α(2,3) free PSA in the method is the ratio of final detectable α(2,3) free PSA to the total amount of α(2,3) free PSA present in a sample.

(3) Method of determining ratio

1) Method of measuring ratio - 1

As a method of "determining the ratio of α(2,3) free PSA to the amount of free PSA in a biological sample" according to the present invention, for example, the following method can be mentioned.
That is, using one or more reference solutions whose ratio of the amount of α(2,3) free PSA to the amount of free PSA is known, prepared in advance using an α(2,3) free PSA standard, the amount of α(2,3) free PSA is measured by the method. Subsequently, using a test sample, the amount of α(2,3) free PSA is measured by the method in the same manner. By comparing the measurement results obtained using the reference solutions with the measurement results obtained using the test sample, the ratio of α(2,3) free PSA to the amount of free PSA in the test sample can be determined.
As a reference solution to be used, one having a numerical value at which the ratio of the amount of α(2,3) free PSA to the amount of free PSA will serve as an index in the subsequent determination of Pca is used. For example, it is sufficient to use a reference solution in which the ratio is 25%, 45%, or 50%.

As a method for preparing the reference solution, for example, the following methods can be mentioned.

That is, the α(2,3) free PSA standard and the α(2,6) free PSA standard are dissolved in appropriate buffer solutions, respectively, and adjusted so that the protein amounts of the respective solutions are the same. Then, a solution of α(2,3) free PSA standard is diluted with a solution of α(2,6) free PSA standard and adjusted to the target ratio, whereby a reference solution can be obtained.

2) Method of determining ratio - 2

As another method of measuring the ratio, for example, there is a "method in which the amount of free PSA and the amount of α(2,3) free PSA in a biological sample are measured, and the ratio of the amount of α(2,3) free PSA to the amount of free PSA obtained is determined" according to the determination method of the present invention.

Details of the method of measuring the amount of free PSA and the amount of α(2,3) free PSA in the method are the same as those described in "(1) Method of measuring amount of free PSA" and "(2) Method of measuring amount of α(2,3) free PSA, or amount of α(2,3) free PSA and amount of free PSA other than α(2,3) free PSA".

A specific example of the method of measuring the ratio - 2 may be, for example,

(i) a "method of measuring the amount of free PSA in a biological sample; in addition, measuring the amount of α(2,3) free PSA by a method in which a complex of α(2,3) free PSA and an affinity substance is formed by the interaction between a glycan in which the terminal sialic acid residue of the glycan of α(2,3) free PSA is α(2,3)-linked to the second galactose residue from the terminal of the glycan and an affinity substance (lectin or antibody), the amount of the complex is measured, and the amount of α(2,3) free PSA is measured on the basis of the results; and determining the ratio of the amount of α(2,3) free PSA to the amount of free PSA obtained",

(ii) a "method of measuring the amount of α(2,3) free PSA and the amount of free PSA other than α(2,3) free PSA by a method in which a complex of α(2,3) free PSA and an affinity substance is formed by the interaction between a glycan in which the terminal sialic acid residue of the glycan of α(2,3) free PSA is α(2,3)-linked to the second galactose residue from the terminal of the glycan and an affinity substance (lectin or antibody), the amount of the complex is measured, and the amount of α(2,3) free PSA and the amount of free PSA other than α(2,3) free PSA are measured on the basis of the results; and determining the ratio of the amount of α(2,3) free PSA to the sum of the amount of α(2,3) free PSA and the amount of free PSA other than α(2,3) free PSA obtained", or

(iii) a "method of measuring the amount of α(2,3) free PSA, or the amount of α(2,3) free PSA and the amount of free PSA other than α(2,3) free PSA by a method of measuring the amount of α(2,3) free PSA, or the amount of α(2,3) free PSA and the amount of free PSA other than α(2,3) free PSA without using an affinity substance; and determining the ratio of the amount of α(2,3) free PSA to the amount of free PSA obtained".

In the methods (i) to (iii), the efficiency of capturing α(2,3) free PSA in the method of measuring the amount of α(2,3) free PSA, or the amount of α(2,3) free PSA and the amount of free PSA other than α(2,3) free PSA is preferably 80% or more.

In addition, the efficiency of capturing α(2,3) free PSA according to the present invention is 80% or more, preferably 90% or more, more preferably 95% or more, still more preferably 98% or more, and particularly preferably 100%.

Among the methods, the method (i) or (ii) is preferred.

Among them, more preferred is

(i)' a "method of measuring the amount of free PSA in a biological sample; in addition, measuring the amount of α(2,3) free PSA by a method in which a complex of α(2,3) free PSA and an affinity substance is formed by the interaction between a glycan in which the terminal sialic acid residue of the glycan of α(2,3) free PSA is α(2,3)-linked to the second galactose residue from the terminal of the glycan and an affinity substance (lectin or antibody), the amount of the complex is measured without removing the components other than the complex from the system, and the amount of α(2,3) free PSA is measured on the basis of the results; and determining the ratio of the amount of α(2,3) free PSA to the amount of free PSA obtained", or

(ii)" a "method of measuring the amount of α(2,3) free PSA and the amount of free PSA other than α(2,3) free PSA by a method in which a complex of α(2,3) free PSA and an affinity substance is formed by the interaction between a glycan in which the terminal sialic acid residue of the glycan of α(2,3) free PSA is α(2,3)-linked to the second galactose residue from the terminal of the glycan and an affinity substance (lectin or antibody), the amount of the complex is measured without removing the components other than the complex from the system, and the amount of α(2,3) free PSA and the amount of free PSA other than α(2,3) free PSA are measured on the basis of the results; and determining the ratio of the amount of α(2,3) free PSA to the sum of the amount of α(2,3) free PSA and the amount of free PSA other than α(2,3) free PSA obtained"

In the method (i)' or (ii)', the efficiency of capturing α(2,3) free PSA in the method of measuring the amount of α(2,3) free PSA, or the amount of α(2,3) free PSA and the amount of free PSA other than α(2,3) free PSA is preferably 80% or more.

In the method, the method using a lectin as an affinity substance is more preferable.

In the method (ii)', a method using a lectin as an affinity substance, that is,
a "method of measuring the amount of α(2,3) free PSA and the amount of free PSA other than α(2,3) free PSA by a method in which a complex of α(2,3) free PSA and a lectin is formed by the interaction between a glycan in which the terminal sialic acid residue of the glycan of α(2,3) free PSA is α(2,3)-linked to the second galactose residue from the terminal of the glycan and a lectin having an affinity for a glycan in which the terminal sialic acid residue of the glycan is α(2,3)-linked to the second galactose residue from the terminal of the glycan, the amount of the complex is measured without removing the components other than the complex from the system, and the amount of α(2,3) free PSA and the amount of free PSA other than α(2,3) free PSA are measured on the basis of the results; and determining the ratio of the amount of α(2,3) free PSA to the sum of the amount of α(2,3) free PSA and the amount of free PSA other than α(2,3) free PSA obtained" is particularly preferable.

In the method, the efficiency of capturing α(2,3) free PSA in the method of measuring the amount of α(2,3) free PSA and the amount of free PSA other than α(2,3) free PSA is preferably 80% or more.

It should be noted that, in the present invention, in the case of determining the ratio of the amount of α(2,3) free PSA to the amount of free PSA, it is not necessary to measure the absolute amount (PSA protein amount) of free PSA, α(2,3) free PSA, and free PSA other than α(2,3) free PSA. It is also possible to determine the ratio by using the actual measurement values (signal value such as fluorescence intensity or absorbance, and peak area) of the measurement. For example, in the case of separating α(2,3) free PSA and free PSA other than α(2,3) free PSA, the peak area of each separated fraction is determined, and peak area of α(2,3) free PSA fraction/(peak area of α(2,3) free PSA fraction+peak area of free PSA fraction other than α(2,3) free PSA) may be taken as the ratio of the amount of α(2,3) free PSA to the amount of free PSA.

In addition, for example, in the case of separating α(2,3) free PSA and free PSA other than α(2,3) free PSA and detecting each PSA using a fluorescently labeled anti-PSA antibody, fluorescence amount of α(2,3) free PSA fraction/(fluorescence amount of α(2,3) free PSA fraction+fluorescence amount of free PSA fraction other than α(2,3) free PSA) may also be taken as the ratio of the amount of α(2,3) free PSA to the amount of free PSA.

An example of a specific embodiment of the "method of measuring the amount of free PSA and the amount of α(2,3) free PSA in a biological sample, and determining the ratio of the amount of α(2,3) free PSA to the amount of free PSA obtained" according to the determination method of the present invention will be described below.

In the example of the following embodiment, the "glycan in which the terminal sialic acid residue of the glycan is α(2,3)-linked to the second galactose residue from the terminal of the glycan" may be referred to as "target glycan" for the sake of convenience.

It should be noted that, in the present specification, the phrase "having an affinity" means, for example, "binding to". In addition, the phrase "separating on the basis of affinity" means, for example, "to separate an object to be separated on the basis of the difference in strength of binding therebetween". For example, in the following embodiments, the phrase "separating the first complex and the second complex on the basis of the affinity for the lectin" means, for example, "the first complex and the second complex are separated from each other on the basis of the difference in the strength of binding of the first complex to the lectin and the strength of binding of the second complex to the lectin".

(a) Method using lectin having affinity for glycan in which terminal sialic acid residue of glycan is α(2,3)-linked to second galactose residue from terminal of glycan as affinity substance

Method A

1) a step of bringing a biological sample, an antibody (labeled anti-PSA antibody) having an affinity for PSA and labeled with a labeling substance, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled anti-PSA antibody, α(2,3) free PSA and the lectin, and a complex (second complex) of the labeled anti-PSA antibody and free PSA other than α(2,3) free PSA,
2) a step of separating the first complex and the second complex obtained in the step 1),
3) a step of measuring the signal derived from the labeling substance of the labeled anti-PSA antibody constituting the complex, thereby measuring the amount of the first complex and the amount of the second complex separated in the step 2), and
4) a step of determining the sum of the amount of the first complex and the amount of the second complex obtained

in the step 3), and determining the ratio of the amount of the first complex obtained in the step 3) to the sum, thereby determining the ratio of the amount of α(2,3) free PSA to the amount of free PSA.

The antibody related to an antibody having an affinity for PSA and labeled with a labeling substance used in Method A may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

Method B

1) a step of bringing a biological sample, a first antibody having an affinity for PSA and labeled with a labeling substance, a second antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of the labeled first antibody, free PSA other than α(2,3) free PSA and the second antibody,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

Examples of combinations of the first antibody and the second antibody used in Method B include the following combinations. However, it is preferred that the epitopes of the first antibody and the second antibody are different.

- The first antibody and the second antibody are antibodies having an affinity for PSA.
- The first antibody is an antibody having an affinity for PSA and the second antibody is an antibody which specifically binds to free PSA.
- The first antibody and the second antibody are antibodies that specifically bind to free PSA.
- The first antibody is an antibody specifically binding to free PSA and the second antibody is an antibody having an affinity for PSA.

In addition, the first complex and the second complex may be separated on the basis of the affinity of the lectin for the target glycan or may be separated on the basis of the difference in mass between the first complex and the second complex (for example, electrophoresis).

Method C

1) a step of bringing a biological sample, a first antibody having an affinity for PSA and labeled with a labeling substance, a second antibody having an affinity for a glycan in which the terminal sialic acid residue of the glycan is α(2,3)-linked to the second galactose residue from the terminal of the glycan, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of the labeled first antibody and free PSA other than α(2,3) free PSA,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby

determining the ratio of the amount of α(2,3) free PSA to the amount of free PSA.

Examples of combinations of the first antibody and the second antibody used in Method C include the following combinations. However, it is preferred that the epitopes of the first antibody and the second antibody are different.

- The first antibody is an antibody having an affinity for PSA and the second antibody is an antibody having an affinity for a glycan in which the terminal sialic acid residue of the glycan is α(2,3)-linked to the second galactose residue from the terminal of the glycan.
- The first antibody is a labeled antibody that specifically binds to free PSA, and the second antibody is an antibody having an affinity for a glycan in which the terminal sialic acid residue of the glycan is α(2,3)-linked to the second galactose residue from the terminal of the glycan.

In addition, the first complex and the second complex may be separated on the basis of the affinity of the lectin for the target glycan or may be separated on the basis of the difference in mass between the first complex and the second complex (for example, electrophoresis).

Method D

1) a step of bringing a biological sample, a first antibody labeled with a labeling substance and having an affinity for a glycan in which the terminal sialic acid residue of the glycan is α(2,3)-linked to the second galactose residue from the terminal of the glycan, a second antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the lectin, and a complex (second complex) of free PSA other than α(2,3) free PSA and the second antibody,
2) optionally, a step of separating the first complex and the second complex obtained in 1),
3) a step of measuring the amount of the first complex by measuring the signal derived from the labeling substance of the labeled antibody constituting the complex, and on the basis of the measurement value, determining the amount of α(2,3) free PSA, and
4) a step of determining the ratio of the amount of α(2,3) free PSA determined in 3) to the amount of free PSA in the biological sample determined in advance.

The second antibody used in Method D may be an antibody that specifically binds to free PSA.
In addition, the first complex and the second complex may be separated on the basis of the affinity of the lectin for the target glycan or may be separated on the basis of the difference in mass between the first complex and the second complex (for example, electrophoresis).
In addition, in Method D, the amount of free PSA may be determined by carrying out the method of directly determining an amount of free PSA. In addition, the amount of α(2,3) free PSA may be determined by conversion into quantitative values by a conventional method using the results obtained by carrying out the measurement in the same manner using an α(2,3) free PSA standard of known concentration in advance.

Method E (Method without using labeled antibody)

1) a step of bringing a biological sample, an antibody having an affinity for PSA, and a lectin (affinity substance) having an affinity for a glycan in which the terminal sialic acid residue of the glycan is α(2,3)-linked to the second galactose residue from the terminal of the glycan into contact with each other to form a complex (first complex) of the antibody, α(2,3) free PSA and the lectin, and a complex (second complex) of the antibody and free PSA other than α(2,3) free PSA,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

The antibody having an affinity for PSA used in Method E may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method F (Method without using labeled antibody)

1) a step of bringing a biological sample, a first antibody having an affinity for PSA, a second antibody specifically binding to free PSA, and a lectin (affinity substance) having an affinity for a glycan in which the terminal sialic acid residue of the glycan is $\alpha$(2,3)-linked to the second galactose residue from the terminal of the glycan into contact with each other to form a complex (first complex) of the first antibody, $\alpha$(2,3) free PSA, the second antibody and the lectin, and a complex (second complex) of the first antibody, free PSA other than $\alpha$(2,3) free PSA and the second antibody,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of $\alpha$(2,3) free PSA to the amount of free PSA.

In addition, the first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method G (Method without using labeled antibody)

1) a step of bringing a biological sample, a first antibody having an affinity for PSA, a second antibody having an affinity for a glycan in which the terminal sialic acid residue of the glycan is $\alpha$(2,3)-linked to the second galactose residue from the terminal of the glycan, and a lectin (affinity substance) having an affinity for a glycan in which the terminal sialic acid residue of the glycan is $\alpha$(2,3)-linked to the second galactose residue from the terminal of the glycan into contact with each other to form a complex (first complex) of the first antibody, $\alpha$(2,3) free PSA, the second antibody and the lectin, and a complex (second complex) of the first antibody and free PSA other than $\alpha$(2,3) free PSA,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of $\alpha$(2,3) free PSA to the amount of free PSA.

The first antibody used in Method G may be an antibody that specifically binds to free PSA.

In addition, the first complex and the second complex may be separated on the basis of the affinity of the lectin for the target glycan or may be separated on the basis of the difference in mass between the first complex and the second complex (for example, electrophoresis).

To measure the amount of the first complex and the amount of the second complex, for example, the amount

of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

(b) Method using antibody having affinity for glycan in which terminal sialic acid residue of glycan is α(2,3)-linked to second galactose residue from terminal of glycan as affinity substance

Method H

1) a step of bringing a biological sample, a first antibody having an affinity for PSA and labeled with a labeling substance, and a second antibody (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA and the second antibody, and a complex (second complex) of the labeled first antibody and free PSA other than α(2,3) free PSA,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

The first antibody used in Method H may be an antibody that specifically binds to free PSA.
In addition, the first complex and the second complex may be separated on the basis of the affinity of the second antibody for the target glycan or may be separated on the basis of the difference in mass between the first complex and the second complex (for example, electrophoresis).

Method I

1) a step of bringing a biological sample, a first antibody (affinity substance) having an affinity for a glycan in which the terminal sialic acid residue of the glycan is α(2,3)-linked to the second galactose residue from the terminal of the glycan and labeled with a labeling substance, and a second antibody having an affinity for PSA into contact with each other to form a complex (first complex) of the labeled first antibody, α(2,3) free PSA and the second antibody, and a complex (second complex) of free PSA other than α(2,3) free PSA and the second antibody,
2) optionally, a step of separating the first complex and the second complex obtained in the step 1),
3) a step of measuring the amount of the first complex by measuring the signal derived from the labeling substance of the labeled first antibody constituting the complex, and on the basis of the measurement value, determining the amount of α(2,3) free PSA, and
4) a step of determining the ratio of the amount of α(2,3) free PSA determined in 3) to the amount of free PSA in the biological sample determined in advance.

The second antibody used in Method I may be an antibody that specifically binds to free PSA.
In addition, the first complex and the second complex may be separated on the basis of the affinity of the first antibody for the target glycan or may be separated on the basis of the difference in mass between the first complex and the second complex (for example, electrophoresis).
In addition, in Method I, the amount of free PSA may be determined by carrying out the method of directly determining an amount of free PSA. In addition, the amount of α(2,3) free PSA may be determined by conversion into quantitative values by a conventional method using the results obtained by carrying out the measurement in the same manner using an α(2,3) free PSA standard of known concentration in advance.

Method J

1) a step of bringing a biological sample, a first antibody having an affinity for PSA and labeled with a labeling substance, a second antibody having an affinity for PSA, and a third antibody (affinity substance) having an affinity for a glycan in which the terminal sialic acid residue of the glycan is α(2,3)-linked to the second galactose residue from the terminal of the glycan into contact with each other to form a complex (first complex) of the labeled first antibody, α(2,3) free PSA, the second antibody and the third antibody, and a complex (second complex) of the labeled first antibody, free PSA other than α(2,3) free PSA and the second antibody,
2) a step of separating the first complex and the second complex obtained in the step 1),
3) a step of measuring the signal derived from the labeling substance of the labeled first antibody constituting the complex, thereby measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

Examples of combinations of the first antibody and the second antibody used in Method J include the following combinations. However, it is preferred that the epitopes of the first antibody and the second antibody are different.

- The first antibody and the second antibody are antibodies having an affinity for PSA.
- The first antibody is an antibody having an affinity for PSA and the second antibody is an antibody which specifically binds to free PSA.
- The first antibody is an antibody specifically binding to free PSA and the second antibody is an antibody having an affinity for PSA.
- The first antibody and the second antibody are antibodies that specifically bind to free PSA.

In addition, the first complex and the second complex may be separated on the basis of the affinity of the third antibody for the target glycan or may be separated on the basis of the difference in mass between the first complex and the second complex (for example, electrophoresis).

Method K

1) a step of bringing a biological sample, a first antibody (affinity substance) having an affinity for a glycan in which the terminal sialic acid residue of the glycan is α(2,3)-linked to the second galactose residue from the terminal of the glycan and labeled with a labeling substance, a second antibody having an affinity for PSA, and a third antibody specifically binding to free PSA into contact with each other to form a complex (first complex) of the labeled first antibody, α(2,3) free PSA, the second antibody and the third antibody, and a complex (second complex) of free PSA other than α(2,3) free PSA, the second antibody and the third antibody,
2) optionally, a step of separating the first complex and the second complex obtained in the step 1),
3) a step of measuring the amount of the first complex by measuring the signal derived from the labeling substance of the labeled first antibody constituting the complex, and on the basis of the measurement value, determining the amount of α(2,3) free PSA, and
4) a step of determining the ratio of the amount of α(2,3) free PSA determined in 3) to the amount of free PSA in the biological sample determined in advance.

In addition, the first complex and the second complex may be separated on the basis of the affinity of the first antibody for the target glycan or may be separated on the basis of the difference in mass between the first complex and the second complex (for example, electrophoresis).
In addition, in Method K, the amount of free PSA may be determined by carrying out the method of directly determining an amount of free PSA. In addition, the amount of α(2,3) free PSA may be determined by conversion into quantitative values by a conventional method using the results obtained by carrying out the measurement in the same manner using an α(2,3) free PSA standard of known concentration in advance.

Method L (Method without using labeled antibody)

1) a step of bringing a biological sample, a first antibody having an affinity for PSA, and a second antibody (affinity

substance) having an affinity for a glycan in which the terminal sialic acid residue of the glycan is α(2,3)-linked to the second galactose residue from the terminal of the glycan into contact with each other to form a complex (first complex) of the first antibody, α(2,3) free PSA and the second antibody, and a complex (second complex) of the first antibody and free PSA other than α(2,3) free PSA,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

The first antibody used in Method L may be an antibody that specifically binds to free PSA.
In addition, the first complex and the second complex may be separated on the basis of the affinity of the second antibody for the target glycan or may be separated on the basis of the difference in mass between the first complex and the second complex (for example, electrophoresis).
To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.

Method M (Method without using labeled antibody)

1) a step of bringing a biological sample, a first antibody having an affinity for PSA, a second antibody specifically binding to free PSA, and a third antibody having an affinity for a glycan in which the terminal sialic acid residue of the glycan is α(2,3)-linked to the second galactose residue from the terminal of the glycan into contact with each other to form a complex (first complex) of the first antibody, α(2,3) free PSA, the second antibody and the third antibody (affinity substance), and a complex (second complex) of the first antibody, free PSA other than α(2,3) free PSA and the second antibody,
2) a step of separating the first complex and the second complex obtained in the step 1),
3) a step of measuring the amount of the first complex and the amount of the second complex separated in the step 2), and
4) a step of determining the sum of the amount of the first complex and the amount of the second complex obtained in the step 3), and determining the ratio of the amount of the first complex obtained in the step 3) to the sum, thereby determining the ratio of the amount of α(2,3) free PSA to the amount of free PSA.

The first complex and the second complex may be separated on the basis of the affinity of the third antibody for the target glycan or may be separated on the basis of the difference in mass between the first complex and the second complex (for example, electrophoresis).
To measure the amount of the first complex and the amount of the second complex, for example, the amount of PSA protein of each complex may be measured. Specifically, in the case where the first complex and the second complex are separated by electrophoresis, for example, there is a method in which a known Western blotting method using the anti-PSA antibody as a primary antibody and using a labeled antibody labeled with a measurable labeling substance as a secondary antibody is carried out to measure the amount of PSA protein in each separated fraction.
In the above Methods A to M, as a method of carrying out the "method of forming a complex of α(2,3) free PSA with an affinity substance by the interaction between the glycan in which the terminal sialic acid residue of the glycan of α(2,3) free PSA is α(2,3)-linked to the second galactose residue from terminal of the glycan and the affinity substance; measuring the amount of the complex without removing components other than the complex from the system; and on the basis of the results, measuring the amount of α(2,3) free PSA", for example, there is a method of measuring the amount of the complex without performing a washing operation which is normally carried out in order to remove components other than the complex from the measurement system.
Among Methods A to M, Methods A, B, E, F, and H to M are preferable. As a method using a labeled antibody, Methods B and I are more preferable. As a method without using a labeled antibody, Methods E, F, L, and M are more preferable. Methods B and I are particularly preferred.
As a specific example of the "method of forming a complex of α(2,3) free PSA with an affinity substance by the interaction between the glycan in which the terminal sialic acid residue of the glycan of α(2,3) free PSA is α(2,3)-linked

to the second galactose residue from terminal of the glycan and the affinity substance; measuring the amount of the complex; and on the basis of the results, measuring the amount of α(2,3) free PSA, or the amount of α(2,3) free PSA and the amount of free PSA other than α(2,3) free PSA" according to the present invention, for example, ELISA or RIA can be mentioned.

As a specific example of the "method of forming a complex of α(2,3) free PSA with an affinity substance by the interaction between the glycan in which the terminal sialic acid residue of the glycan of α(2,3) free PSA is α(2,3)-linked to the second galactose residue from terminal of the glycan and the affinity substance; measuring the amount of the complex without removing components other than the complex from the system; and on the basis of the results, measuring the amount of α(2,3) free PSA" according to the present invention, for example, capillary electrophoresis, a surface plasmon resonance method, mass spectrometry, or lectin microarray can be mentioned.

Hereinafter, a specific example of the "method of forming a complex of α(2,3) free PSA with an affinity substance utilizing the interaction between the glycan in which the terminal sialic acid residue of the glycan of α(2,3) free PSA is α(2,3)-linked to the second galactose residue from terminal of the glycan and the affinity substance; measuring the amount of the complex (without removing components other than the complex from the system); on the basis of the results, measuring the amount of α(2,3) free PSA, and determining the ratio of the amount of α(2,3) free PSA to the amount of free PSA obtained" by each method will be described.

The method of confirming the efficiency of capturing α(2,3) free PSA is as described above. For example, in the case where an affinity substance is used, it may be carried out as follows.

The affinity substance and the α(2,3) free PSA standard are reacted by the following method. After the reaction, the fraction in which the α(2,3) free PSA and the affinity substance were complexed and the unreacted fraction were separated, and the amount of the target glycan in each fraction was analyzed with a mass spectrometer. The capture efficiency can be obtained by calculating (the amount of target glycan of complex formed fraction) / (amount of target glycan of complex formed fraction+amount of target glycan of unreacted fraction).

It should be noted that, for each of the following methods, an example of how to determine the capture efficiency is described, but it is not limited to that method.

<u>Capillary electrophoresis</u>

As a method of determining the amount of α(2,3) free PSA and determining the ratio of the amount of α(2,3) free PSA to the amount of free PSA by capillary electrophoresis, for example, first, a biological sample containing PSA is brought into contact with and reacted with a labeled anti-PSA antibody in which an anti-PSA antibody (preferably an anti-free PSA antibody) according to the present invention is labeled with a labeling substance, the [labeled anti-PSA antibody-α(2,3) free PSA] complex and the [labeled anti-PSA antibody-free PSA other than α(2,3) free PSA] complex in the obtained reaction solution are separated by carrying out capillary electrophoresis in the presence of an affinity substance, and the amount of the labeling substance derived from the [labeled anti-PSA antibody-α(2,3) free PSA] Complex 1 and the amount of the labeling substance derived from the [labeled anti-PSA antibody-free PSA other than α(2,3) free PSA] Complex 2 are measured. The amount of free PSA in the sample is the sum of the amounts of labeling substances of Complex 1 and Complex 2. Then, the ratio of the amount of α(2,3) free PSA to the amount of free PSA obtained is determined.

Since the amount of the labeling substance derived from Complex 1 and the amount of the labeling substance derived from Complex 2 correspond to the respective peak area values obtained by capillary electrophoresis, the ratio may be determined using the respective peak area values.

Specific examples of the anti-PSA antibody and labeling substance in the labeled anti-PSA antibody used in the method are as described above.

As a solvent for reacting a biological sample with a labeled anti-PSA antibody (preferably an anti-free PSA antibody), for example, a buffer solution can be mentioned. The buffer solution to be used for this purpose is not particularly limited as long as it is commonly used in the art, but those having a buffering action in the vicinity of neutral pH, usually pH 5.0 to 10.0, preferably pH 6.5 to 8.5, can be mentioned. Specific examples of the buffer solution include a Tris-HCl buffer solution, a MES buffer solution, a HEPES buffer solution, a borate buffer solution, a phosphate buffer solution, a veronal buffer solution, and a Good's buffer solution. In addition, the concentration of a buffering agent in these buffer solutions is appropriately selected from the range of usually 5 to 1,000 mM and preferably 5 to 300 mM.

The buffer solution may further contain a sensitizer, a surfactant, a preservative (for example, sodium azide, salicylic acid, or benzoic acid), a stabilizer (for example, albumin, globulin, water-soluble gelatin, surfactant, or saccharides), an activator agent, and others which are used in the art and which do not inhibit the stability with coexisting reagents or do not inhibit an antigen-antibody reaction. In addition, concentration ranges and the like of these reagents and the like may be appropriately selected and used in a concentration range commonly used in the measuring method known per se.

In the case where a commercially available kit dedicated to a capillary electrophoresis apparatus is used, a

buffer solution included in the kit may be used.

The concentration of the antibody in the solution containing the labeled anti-PSA antibody may be any concentration as long as it is within the target concentration range in the case of mixing the sample and the solution containing the labeled anti-PSA antibody. For example, the concentration of the antibody in the solution containing the labeled anti-PSA antibody may be 0.1 to 200 μM, preferably 0.5 to 50 μM, more preferably 0.5 to 20 μM, and still more preferably 0.5 to 10 μM. That is, the lower limit value thereof is 0.1 μM and preferably 0.5 μM. In addition, the upper limit value thereof is 200 μM, preferably 50 μM, more preferably 20 μM, and still more preferably 10 μM.

The concentration of the labeled anti-PSA antibody according to the present invention in the reaction solution in the case of contacting/reacting the biological sample with the labeled anti-PSA antibody according to the present invention varies depending on the concentration of PSA in the sample and is not particularly limited, but it is usually 0.1 to 1,000 nM, preferably 0.1 to 500 nM, and more preferably 0.5 to 200 nM. That is, the lower limit value thereof is 0.1 nM and preferably 0.5 nM. In addition, the upper limit value thereof is 1,000 nM, preferably 500 nM, and more preferably 200 nM.

In addition, the pH during the reaction between the biological sample and the labeled anti-PSA antibody according to the present invention is not particularly limited as long as it does not inhibit an antigen-antibody reaction, and it is in the range of usually 6.0 to 10.0 and preferably 6.0 to 8.0. The temperature during the reaction is not particularly limited as long as it does not inhibit an antigen-antibody reaction, and it is in the range of usually 10 to 50°C and preferably 20 to 40°C. In addition, the reaction time varies depending on the antibody according to the present invention to be used and reaction conditions such as pH and temperature, so that the reaction may be carried out for about 1 to 60 minutes and preferably 1 to 15 minutes depending on each.

After the reaction, the [labeled anti-PSA antibody-α(2,3) free PSA] complex and the [labeled anti-PSA antibody-free PSA other than α(2,3) free PSA] complex in the obtained reaction solution are separated by carrying out capillary electrophoresis in the presence of an affinity substance, and the amount of the [labeled anti-PSA antibody-α(2,3) free PSA] complex is measured.

In the present invention, among capillary electrophoresis, it is preferable to carry out electrophoresis in a capillary chip. The (micro)chip capillary electrophoresis is a technique of forming a capillary having a cross section with a diameter of 100 μm or less on a chip substrate and carrying out electrophoresis in this capillary, and is a method of separating a substance utilizing the difference in charge of the substance present in a sample as a difference in its mobility by applying a voltage to the capillary.

Capillary electrophoresis is classified into capillary zone electrophoresis and capillary gel electrophoresis depending on the electrophoresis solution to be used, but the method of the present invention can be applied to any of them. In view of the accuracy of separation, capillary gel electrophoresis is preferable among the capillary electrophoresis.

The electrophoresis solution used in the capillary electrophoresis according to the present invention is not particularly limited as long as it is commonly used in the art, and specifically it may be, for example, a buffer solution having a pH of 5 to 10 and preferably 6 to 8. Specific examples of the buffer solution include, but are not limited to, a Tris-HCl buffer solution, a HEPES buffer solution, a lactate buffer solution, a citrate buffer solution, an acetate buffer solution, a succinate buffer solution, a glycine buffer solution, a phthalate buffer solution, a phosphate buffer solution, a triethanolamine buffer solution, a borate buffer solution, a glycine buffer solution, a barbital buffer solution, a tartrate buffer solution, and a borate buffer solution. The concentration of a buffering agent in these buffer solutions is suitably selected from the range of usually 10 to 500 mM and preferably 10 to 300 mM.

In addition, the electrophoresis solution may contain a substance for reducing the effect of electroosmotic flow, for example, polyethylene glycol, polyacrylamide, polyethylene imine, fluorine-containing aromatic hydrocarbon, saccharide, or the like, the concentration of which may be set within the range commonly used in the art. In addition, the buffer solution may contain salts such as NaCl, surfactants, preservatives, proteins such as BSA, and the like as long as they do not interfere with an antigen-antibody reaction and a glycan-affinity substance reaction.

In addition, in the case of capillary gel electrophoresis, polymers may be added to the above buffer solution used as an electrophoresis solution for capillary electrophoresis, and the polymer includes, for example, polyethers such as polyethylene oxide (polyethylene glycol) and polypropylene oxide; polyalkylene imines such as polyethylene imine; polyacrylic acid-based polymers such as polyacrylic acid, polyacrylic acid ester, and methyl polyacrylate; polyamide-based polymers such as polyacrylamide and polymethacrylamide; polymethacrylic acid-based polymers such as polymethacrylic acid, polymethacrylic acid ester, and methyl polymethacrylate; polyvinyl-based polymers such as polyvinyl acetate, polyvinyl pyrrolidone, and polyvinyl oxazolidone; water-soluble hydroxyl polymers such as pullulan, elsinan, xanthan, dextran, and guar gum; water-soluble cellulose compounds such as methyl cellulose, hydroxyethyl cellulose, and hydroxypropyl cellulose; derivatives thereof, and copolymers containing a plurality of monomer units constituting these polymers.

Two or more of the polymers may be added in combination. In addition, the molecular weight of the polymer as described above is usually 500 Da to 6,000 kDa, preferably 1 to 1,000 kDa, and more preferably 100 to 1,000 kDa. In addition, the concentration of the polymer may be appropriately selected from the range commonly used in the art,

and is usually 0.01 to 40% (w/v), preferably 0.01 to 20% (w/v), and more preferably 0.1 to 10% (w/v). It should be noted that, in the case where the filler is added to an electrophoresis buffer solution, the viscosity of the electrophoresis buffer solution is usually 2 to 1,000 centipoise, preferably 5 to 200 centipoise, and more preferably 10 to 100 centipoise.

The affinity substance may be contained in the electrophoresis solution. However, during the separation by capillary electrophoresis, it is preferred that the affinity substance is at a higher concentration than the amount at which the $\alpha$(2,3) free PSA and the affinity substance can be completely bound.

For example, in the case where the affinity substance is a lectin, the concentration thereof is 0.1 mg/mL to 20 mg/mL, preferably 1.0 mg/mL to 10 mg/mL, and more preferably 2.0 mg/mL to 5.0 mg/mL. In the case where the affinity substance is an antibody, the concentration thereof is 0.01 mg/mL to 20 mg/mL, preferably 0.05 mg/mL to 10 mg/mL, and more preferably 0.1 mg/mL to 5.0 mg/mL.

The concentration of MAA in the microchannel may be 0.1 mg/mL to 20 mg/mL.

In the case of separating a complex by capillary electrophoresis, it is preferred that any antibody used in the measurement system is labeled with a charged carrier molecule such as an anionic substance, even in the case where a lectin is used as the affinity substance or an antibody is used as the affinity substance. Specifically, the lectin or the antibody is preferably labeled with, for example, a nucleic acid chain. In this case, the antibody may be labeled with both a charged carrier molecule and the labeling substance for detection.

The nucleic acid chain used for such a purpose is a chain polynucleotide which has a nucleotide residue consisting of a purine base or pyrimidine base, a sugar moiety pentose, and phosphoric acid as a basic unit and in which the respective nucleotides are linked and polymerized by phosphor-diester bond between the carbons at the 3' and 5' positions of the sugar. For example, RNA in which the sugar moiety is ribose or/and DNA in which the sugar moiety is deoxyribose can be mentioned. In addition, the nucleic acid chain may be a single-stranded nucleic acid chain or may be a nucleic acid chain consisting of two or more nucleic acid strands.

The length of the nucleic acid chain to be used may be of any length as long as it can achieve the object of the present invention, and it is usually 1 bp to 1,000 kbp, preferably 5 bp to 100 kbp, more preferably 10 bp to 50 kbp, and still more preferably 50 bp to 2,500 bp.

As a method of binding the nucleic acid chain and the antibody according to the present invention, known methods disclosed in, for example, JP4214779B and JP4862093B can be mentioned.

For example, the functional group of each of the antibody and the nucleic acid chain may be linked directly or through a linker [for example, sulfosuccinimidyl 4-(p-maleimidophenyl)butyrate (Sulfo-SMPB), sulfosuccinimidyl 4-(N-maleimidomethyl)cyclohexane-1-carboxylate (Sulfo-SMCC), or N-($\varepsilon$-maleimidocaproyloxy)succinimide (EMCS)] or the like.

In addition, after a reactive functional group is introduced in advance into the nucleic acid chain, the reactive functional group-introduced nucleic acid chain may be bound to the antibody according to the present invention by the method described in JP4214779B. As a method of introducing the reactive functional group into the nucleic acid chain, a method known per se can be mentioned.

In addition, as a method of binding the nucleic acid chain and the antibody, a reactive functional group can be introduced into the nucleic acid terminus, for example, by a method in which PCR is carried out using a PCR primer in which a reactive functional group is introduced at the 5' terminal, thereby obtaining a nucleic acid chain having a reactive functional group introduced at the 5' terminal as a PCR product (Molecular Cloning: A Laboratory Manual Second Edition, J. Sambrook, E. F. Fritsch, T. Maniatis, Cold Spring Harbor Laboratory Press, or the like).

Examples of solutions for dissolving a sample, an antibody, or the like to be subjected to capillary electrophoresis include a Tris buffer solution, a Bis-Tris buffer solution, a Good's buffer solution, a HEPES buffer solution, a glycine buffer solution, a borate buffer solution, a phosphate buffer solution, a veronal buffer solution, and a MOPS buffer solution. In addition, the concentration of a buffering agent in these buffer solutions is suitably selected from the range of usually 10 to 500 mM and preferably 10 to 300 mM. In addition, the pH is not particularly limited as long as it does not interfere with an antigen-antibody reaction and a glycan-affinity substance reaction, but it is usually preferably in the range of 5 to 9. The solution may contain, for example, saccharides, salts such as NaCl, surfactants, preservatives, or proteins as long as they do not interfere with an antigen-antibody reaction and a glycan-affinity substance reaction.

The method of introducing a sample (solution containing a [labeled anti-PSA antibody-free PSA] complex) in the capillary electrophoresis according to the present invention may be any method commonly used in the art and is not particularly limited, and examples thereof include a suction method, a pressurization method, and an electric introduction method. Among them, a pressurization method is preferable. The introduction amount of the sample varies depending on the conditions such as the inner diameter and length of the capillary, and the type and sensitivity of the detector.

Normally, the sample may be concentrated by isotachophoresis (ITP) known per se, and then the concentrate may be directly introduced into capillary electrophoresis (CE). In this case, for example, in the case of carrying out by microchip electrophoresis, it is desirable to carry out ITP electrophoresis and CE electrophoresis consecutively using a chip in which ITP electrophoresis and CE electrophoresis are coupled.

Specific conditions of capillary electrophoresis may be in accordance with methods known per se. For example,

in the case where capillary electrophoresis is used, it may be carried out according to the method described in J. Chromatogr. 593, 253-258 (1992), Anal. Chem. 64, 1926-1932 (1992), WO2007/027495, and the like.

Since the voltage during electrophoresis in the electrophoresis according to the present invention varies depending on the electrophoresis solution, the apparatus to be used, and the like, it may be appropriately set from the range commonly used in the art.

In the case where capillary electrophoresis is carried out by an automatic analyzer, capillary electrophoresis may be carried out according to the method described in the instructions attached to the apparatus under the conditions described in the instructions.

As a specific example of the method of measuring α(2,3) free PSA of the present invention, given below is a method in which, using MAA as an affinity substance and using a labeled first antibody in which an anti-free PSA antibody is labeled with a fluorescent substance, as a labeled anti-PSA antibody, and using a second antibody in which an anti-PSA antibody is labeled with DNA, microchip capillary electrophoresis utilizing lectin affinity is carried out to separate PSA on the basis of the degree of affinity of lectin for the glycan of PSA, and the separated PSA is measured with a fluorescence detector.

That is, 1 to 50 μL of a biological sample containing PSA is reacted with a reagent containing a fluorescently labeled anti-free PSA antibody in amount of usually 0.001 to 10 μM and preferably 0.01 to 1 μM.

The biological sample used for capillary electrophoresis may be a sample collected from a living body or a sample prepared by subjecting a sample collected from a living body to desalting and various purification steps.

The obtained reaction solution, 2 to 50 μL of a test solution containing a DNA-labeled anti-PSA antibody in the amount of usually 0.001 to 10 μM, preferably 0.01 to 1 μM, a electrophoresis buffer solution, and an internal standard substance (for example, fluorescent substance: HiLyte 647 (manufactured by AnaSpec, Inc.) or the like) are introduced into a capillary of, for example, an internal diameter of 5 to 500 μm, preferably 50 to 200 μm, and more preferably 50 to 100 μm, and a length of 1 to 10 cm by a pressure method of 1 to 10 psi for 30 to 60 seconds. The reaction is carried out for 5 seconds to 30 minutes and preferably 10 seconds to 15 minutes while keeping the temperature at 20°C to 40°C. The [fluorescently labeled anti-free PSA antibody-α(2,3) free PSA-DNA-labeled anti-PSA antibody] complex and the [fluorescently labeled anti-free PSA antibody-free PSA other than α(2,3) free PSA-DNA-labeled anti-PSA antibody] complex obtained are separated by carrying out electrophoresis with application of a voltage of 1,000 to 5,000 V for 10 seconds to 60 minutes in the presence of MAA (0.1 mg/mL to 20 mg/mL). Then, the electrophoretic state of the complex is measured with a detector such as a fluorescence detector or a UV detector to obtain an electropherogram.

The peak of α(2,3) free PSA (containing a [fluorescently labeled anti-free PSA antibody]-[α(2,3) free PSA]-[DNA-labeled anti-PSA antibody] complex), and the peak of other free PSA peak (containing a [fluorescently labeled anti-free PSA antibody]-[free PSA other than α(2,3) free PSA]-[DNA-labeled anti-PSA antibody] complex) can be distinguished from their electrophoretic migration positions. Therefore, the amount of α(2,3) free PSA in the sample is taken as the peak area of the peak. In addition, the sum of the peak area of α(2,3) free PSA and the peak area of free PSA other than α(2,3) free PSA obtained is taken as the amount of free PSA.

As described above, in the case where capillary electrophoresis is used, the amount of free PSA and the amount of α(2,3) free PSA can be measured and determined at the same time with the same sample.

The ratio of the amount of α(2,3) free PSA to the amount of free PSA can be obtained by calculating the ratio of the obtained peak area (peak area of α(2,3) free PSA/amount of free PSA).

The capillary electrophoresis may be carried out using a commercially available fully automated measuring apparatus. For example, μTAS Wako i30 (manufactured by Wako Pure Chemical Industries, Ltd.) can be mentioned.

The principle of measuring PSA by performing microchip capillary electrophoresis utilizing lectin affinity, using a fully automated fluorescence immunoassay system μTAS Wako i30 (manufactured by Wako Pure Chemical Industries, Ltd.) and using MAA as an affinity substance, a labeled first antibody in which an anti-free PSA antibody is labeled with a fluorescent substance as a labeled anti-PSA antibody, and a second antibody in which an anti-PSA antibody is labeled with DNA, will be briefly described below.

First, in the case where PSA in a sample and a fluorescently labeled anti-free PSA antibody are reacted in a liquid phase, PSA binds to the fluorescently labeled anti-free PSA antibody to form a [fluorescently labeled anti-free PSA antibody-free PSA] complex.

A solution (electrophoresis sample A) containing this [fluorescently labeled anti-free PSA antibody-free PSA] complex is dispensed into a predetermined well (immune reaction solution well) on a dedicated chip of a fully automated fluorescence immunoassay system μTAS Wako i30. Then, electrophoresis buffer solution 2 (R2 well) (containing MAA), electrophoresis buffer solution 3 (R3 well), electrophoresis buffer solution 4 (R4 well), DNA-labeled antibody solution (C1 well), and fluorescent solution (FD well) are also dispensed into predetermined wells on the dedicated chip.

A schematic diagram of the dedicated chip (microchip) is schematically shown in Fig. 3.

In Fig. 3, the Waste well is used as a waste reservoir (drain well) at the time of introducing R2, R3, R4, C1, and electrophoresis sample A into an analysis flow channel.

Electrophoresis sample A and each test solution are dispensed to each well, and a voltage is applied thereto.

Then, the DNA-labeled anti-PSA antibody dispensed into the C1 well migrates while being concentrated in the positive electrode direction according to the principle of isotachophoresis. Next, the concentrated DNA-labeled anti-PSA antibody binds to the [fluorescently labeled anti-free PSA antibody-free PSA] complex in the electrophoresis sample A, and therefore the following Complex 1 and Complex 2 are formed.

Complex 1
[Fluorescently labeled anti-free PSA antibody]-[α(2,3) free PSA]-[DNA-labeled anti-PSA antibody]
Complex 2
[Fluorescently labeled anti-free PSA antibody]-[free PSA other than α(2,3) free PSA]-[DNA-labeled anti-PSA antibody]

In the case where electrophoresis is further continued, Complex 1 and Complex 2 and the free DNA-labeled anti-PSA antibody further migrate toward the positive electrode due to the charge of the anion. Since the unreacted fluorescently labeled anti-free PSA antibody is not bound to the DNA-labeled PSA antibody, it is not charged and thus does not migrate further toward the positive electrode.
Next, among the complexes dispensed into the R2 well and migrated to the electrophoresis zone containing MAA, Complex 1 interacts with MAA contained in the zone, but Complex 2 does not interact with MAA.
Therefore, since Complex 1 migrates later than Complex 2, the appearance position of the peak of fluorescence derived from the fluorescently labeled anti-PSA antibody detected is different between Complex 1 and Complex 2. Therefore, it is possible to know the peak of Complex 1 and the peak of Complex 2 from the position of the peak, respectively.
The amount of PSA of each peak can be obtained by determining the peak area of each peak.
The ratio of the amount of α(2,3) free PSA to the amount of free PSA is obtained by determining the ratio of the obtained peak area (peak area of α(2,3) free PSA/amount of free PSA).
In the capillary electrophoresis method, as a method of determining the efficiency of capturing α(2,3) free PSA, for example, the following method can be mentioned, but it is not limited thereto.
In the case where the capillary electrophoresis is carried out using an affinity substance and an α(2,3) free PSA standard of known concentration, the peak area of Complex 1 ([fluorescently labeled anti-free PSA antibody]-[α(2,3) free PSA]-[DNA-labeled anti-PSA antibody]) in which the electrophoretic mobility is shifted by the interaction between the glycan and the affinity substance is determined (measurement value 1).
Separately, the capillary electrophoresis is carried out using an α(2,3) free PSA standard of known concentration without using an affinity substance, whereby the peak area of Complex 1 ([fluorescently labeled anti-free PSA antibody]-[α(2,3) free PSA]-[DNA-labeled anti-PSA antibody]) is determined (measurement value 2).
The ratio of the measurement value 1 to the measurement value 2 (measurement value 1/measurement value 2) obtained is determined, and the obtained ratio is taken as the efficiency of capturing α(2,3) free PSA in this method.

## Surface plasmon resonance method

The surface plasmon resonance method is an intermolecular interaction analysis system that analyzes the interactions between biomolecules using the optical phenomenon of so-called surface plasmon resonance (SPR) which occurs in the case where surface plasmons are excited at the metal/liquid interface. The surface plasmon resonance method uses a surface plasmon resonance spectrometer to detect trace amounts of mass change occurring on the surface of the sensor chip due to binding and dissociation between two molecules as SPR signals. Since interactions between biomolecules are monitored in real time, kinetics information indicating that binding/dissociation between biomolecules is fast or slow is obtained.
As a representative analysis system of the surface plasmon resonance method, there is a Biacore™ method. The Biacore method is commonly simply referred to as Biacore. In addition, in the case of "Biacore", it sometimes refers to a Biacore device used for Biacore's analysis system.
The measurement by a surface plasmon resonance method may be carried out under optimum conditions for the measurement according to the attached instructions.
To carry out the determination method of the present invention by the surface plasmon resonance method, for example, the following methods can be mentioned.
That is, the anti-PSA antibody is immobilized on the surface of the sensor chip. In the case where detection light is projected from the backside of the sensor chip such that the light is totally reflected at the boundary surface between the gold thin film of the sensor chip and the glass, a portion having a reduced reflection intensity appears in part of the reflected light. The angle at which the dark portion of this light appears depends on the refractive index of the solvent on the surface of the sensor chip. Next, a biological sample is flowed from the flow channel of the surface plasmon resonance spectrometer to the surface of the sensor chip. In the case where the anti-PSA antibody immobilized on the

gold thin film surface binds to the PSA molecule contained in the biological sample flowing through the flow channel, the mass of the molecule immobilized on the gold thin film surface increases, and therefore the refractive index of the solvent changes. At this time, the position of the dark portion of the reflected light shifts according to the change of the mass. Conversely, in the case where molecules are dissociated, the position of the dark portion returns from the shifted position. The shift angle represents the mass change on the surface of the sensor chip. In the surface plasmon resonance method, the intermolecular bonding/dissociation is monitored from this angle change. On the basis of the obtained results, first, the amount of free PSA in a biological sample can be measured.

Next, the solution containing an affinity substance is flowed from the flow channel of the surface plasmon resonance spectrometer to the surface of the sensor chip. Similarly, in the case where the PSA of the "anti-PSA antibody PSA complex" formed on the surface of the gold thin film binds to the affinity substance flowing through the flow channel, the refractive index of the solvent changes in the same manner, so that the intermolecular bonding/dissociation is monitored in the same manner. On the basis of the obtained results, it is possible to measure the amount of α(2,3) free PSA in a biological sample.

The ratio of the amount of α(2,3) free PSA to the amount of free PSA obtained in a biological sample is determined.

As a method of measuring the ratio of α(2,3) free PSA to the amount of free PSA in a biological sample by the surface plasmon resonance method, for example, the following method can be mentioned.

That is, using one or more reference solutions whose ratio of the amount of α(2,3) free PSA to the amount of free PSA is known, prepared in advance using an α(2,3) free PSA standard, the measurement by the surface plasmon resonance method is carried out to obtain each sensorgram. Next, the measurement is similarly carried out using a test sample to obtain a sensorgram. By comparing the sensorgram obtained using the reference solution with the sensorgram obtained using the test sample, the ratio of α(2,3) free PSA to the amount of free PSA in the test sample is determined. As a reference solution to be used, one having a numerical value at which the ratio of the amount of α(2,3) free PSA to the amount of free PSA will serve as an index in the subsequent determination of Pca is used. For example, it is sufficient to use a reference solution in which the ratio is 25%, 45%, or 50%.

Examples of the method for preparing the reference solution are as described above.

As a method of determining the efficiency of capturing the amount of α(2,3) free PSA in the surface plasmon resonance method, for example, the following method can be mentioned, but the method is not limited thereto.

That is, the affinity substance is immobilized on the surface of the sensor chip. Next, a sample containing an α(2,3) free PSA standard of known concentration is flowed from the flow channel of the surface plasmon resonance spectrometer to the surface of the sensor chip. During the process, the measurement by the surface plasmon resonance method is carried out to obtain a sensorgram. The peak value of the sensorgram is determined (measurement value 1).

Separately, a sample containing the α(2,3) free PSA standard with the same concentration as that used to obtain the measurement value 1 is flowed from the flow channel of the surface plasmon resonance spectrometer to the surface of the sensor chip on which an anti-PSA antibody is immobilized. During the process, the measurement by the surface plasmon resonance method is carried out to obtain a sensorgram. The peak value of the sensorgram is determined (measurement value 2).

It should be noted that, in the case of determining the capture efficiency by this method, as for the anti-PSA antibody used to obtain the measurement value 2, it is necessary to select those having sufficient specificity and affinity with an antigen, in consideration of dissociation constants and the like.

The ratio of the measurement value 1 to the measurement value 2 obtained (measurement value 1/measurement value 2) is determined, and the obtained ratio is taken as the efficiency of capturing α(2,3) free PSA in this method.

Microarray method

A lectin microarray method developed by Glycomedicine Technology Research Center, National Institute of Advanced Industrial Science and Technology, or the like can also be used for the determination method of the present invention.

The lectin array (lectin microarray) is an array obtained by arranging and immobilizing lectins which are proteins having the property of binding with dozens of different glycans having different specificities in a spot shape on a slide glass. In addition, the evanescent field refers to a state where weak light exudes from the substrate (slide glass) interface. After allowing the fluorescently labeled glycoprotein to interact with the lectin microarray, excitation light is irradiated to the slide glass to generate an evanescent field. Since the excitation light reaches about 100 nm to 200 nm from the interface and the glycan bound to the lectin also exists between 100 nm and 200 nm from the interface, only the glycan bound to the lectin array can emit light. With this technique, the fluorescence of the lectin array can be detected without carrying out the washing operation. After allowing the glycoprotein without fluorescent labeling to interact with the lectin microarray, the antibody against the core protein of the glycoprotein may be reacted with the fluorescently labeled fluorescent antibody and then the fluorescence may be detected by the same method as described above.

The measurement with a lectin microarray may be carried out according to the protocol described in, for example, MICROARRAY METHODS AND PROTOCOLS (CRC Press), edited by Robert S. Matson, "Chapter 9: Lectin Microarrays", Masao Yamada, p.141, 2009.

In the case where, using the lectin microarray technique, the amount of α(2,3) free PSA according to the present invention is measured and the ratio of the amount of α(2,3) free PSA to the amount of free PSA is determined, for example, it can be done as follows.

A microarray in which a lectin which is the affinity substance according to the present invention and a plurality of lectins having an affinity for a glycan of PSA other than a glycan in which the terminal sialic acid residue of the glycan is α(2,3)-linked to the second galactose residue from the terminal of the glycan are immobilized is prepared. The desired microarray may be prepared in accordance with the method described in, for example, Kuno A. et al., Nat Methods. 2005 Nov, vol. 2, No. 11, pp. 851 to 856. Alternatively, a commercially available microarray (LecChip™, manufactured by GlycoTechnica Ltd.) or the like may be used.

biological sample if necessary pre-treated, and the fluorescently labeled anti-free PSA antibody are added dropwise into the microarray and allowed to react to form a [fluorescently labeled anti-free PSA antibody-α(2,3) free PSA-lectin] complex on the microarray. Next, the fluorescence derived from the fluorescently labeled anti-free PSA antibody is measured using an evanescent wave excitation fluorescence scanner. By carrying out the measurement in the same manner using an α(2,3) free PSA standard of known concentration and conversion into quantitative values on the basis of the obtained measurement value, the amount of α(2,3) free PSA in the sample is determined.

The ratio of amount of α(2,3) free PSA measured by the microarray method to the separately determined amount of free PSA is determined.

The method of measuring the amount of free PSA is as described in the section "(1) Method of measuring amount of free PSA".

As a method of determining the efficiency of capturing the amount of α(2,3) free PSA in the lectin microarray method, for example, the following method can be mentioned, but it is not limited thereto.

In a lectin microarray in which a lectin having an affinity for a glycan in which the terminal sialic acid residue of the glycan of PSA is α(2,3)-linked to the second galactose residue from the terminal of the glycan is immobilized, a sample containing an α(2,3) free PSA standard of known concentration is brought into contact with and reacted with the solid phase. On the other hand, the unreacted fraction (containing the washed fraction) is recovered, and the concentration of α(2,3) free PSA remaining in the fraction is measured by a known PSA measuring method.

The efficiency of capturing α(2,3) free PSA in this method can be determined (calculated) from the following equation.

[Concentration of α(2,3) free PSA standard used in measurement-Concentration of α(2,3) free PSA in unreacted fraction (containing washed fraction)]/Concentration of α(2,3) free PSA standard used in measurement

ELISA

As a method of determining the amount of α(2,3) free PSA and determining the ratio of the amount of α(2,3) free PSA to the amount of free PSA by ELISA, for example, the following [Method 1] and [Method 2] can be mentioned.

Method 1

An affinity substance is immobilized on a solid phase. A sample containing PSA is brought into contact with and reacted with the solid phase. After washing the solid phase, a labeled anti-PSA antibody in which an anti-PSA antibody (which may be an anti-free PSA antibody) is labeled with a detectable labeling substance is brought into contact with and reacted with the solid phase. The unreacted labeled anti-PSA antibody is removed by washing or the like, and then the amount of the labeling substance is measured by a measuring method corresponding to the labeling substance of the labeled anti-PSA antibody. On the basis of the obtained measurement results, the amount of α(2,3) free PSA is determined by conversion into quantitative values by a conventional method using the results obtained by carrying out the measurement using an α(2,3) free PSA standard of known concentration in advance. The amount of free PSA is determined by the method of measuring the amount of free PSA in a sample separately. The ratio of the amount of α(2,3) free PSA to the amount of free PSA obtained is determined.

In the method, as a method of determining the efficiency of capturing the amount of α(2,3) free PSA, for example, the following method can be mentioned, but it is not limited thereto.

On a solid phase on which an affinity substance is immobilized, a sample containing an α(2,3) free PSA standard

of known concentration is brought into contact with and reacted with the solid phase. On the other hand, the unreacted fraction (containing the washed fraction) is recovered, and the concentration of α(2,3) free PSA remaining in the fraction is measured by a known PSA measuring method.

The efficiency of capturing α(2,3) free PSA in this method can be determined (calculated) from the following equation.

$$[\text{Concentration of } \alpha(2,3) \text{ free PSA standard used in measurement-Concentration of } \alpha(2,3) \text{ free PSA in unreacted fraction (containing washed fraction)}]/\text{Concentration of } \alpha(2,3) \text{ free PSA standard used in measurement}$$

Method 2

Antibodies that specifically bind to free PSA are immobilized on a solid phase. A sample containing PSA is brought into contact with and reacted with the solid phase. After washing the solid phase, a labeled affinity substance in which an affinity substance is labeled with a detectable labeling substance is brought into contact with and reacted with the solid phase. The unreacted labeled affinity substance is removed by washing or the like, and then the labeling substance is measured by a method corresponding to the labeling substance of the labeled affinity substance. On the basis of the obtained measurement results, the amount of α(2,3) free PSA is determined by conversion into quantitative values by a conventional method using the results obtained by carrying out the measurement using an α(2,3) free PSA standard of known concentration in advance. The amount of free PSA is determined by the method of measuring the amount of free PSA in a sample separately. The ratio of the amount of α(2,3) free PSA to the amount of free PSA obtained is determined.

Method using mass spectrometer

The structure of the glycan of the component in a sample can be analyzed using a mass spectrometer. In the determination method of the present invention, the amount of free PSA, the amount of α(2,3) free PSA, and the amount of free PSA other than α(2,3) free PSA in the sample can be measured by analyzing the structure of the glycan of PSA in a sample using a mass spectrometer. Then, the ratio of the amount of α(2,3) free PSA to the amount of free PSA obtained is determined.

The amount of free PSA may be determined using a mass spectrometer. In addition, the sum of the amount of α(2,3) free PSA and the amount of free PSA other than α(2,3) free PSA obtained using a mass spectrometer may be taken as the amount of free PSA.

(4) Pca determination method

To carry out the Pca determination method of the present invention, first, the ratio of the amount of α(2,3) free PSA to the amount of free PSA obtained by the method is determined. In the case where the ratio is lower than 40%, it is determined that Pca is not developed (Pca-negative) or the probability of developing Pca is low.

In the case where the ratio is higher than 40%, preferably higher than 43.2%, it is determined that the subject is of Pca (Pca-positive) or has high probability of developing Pca.

In addition, in the case where the ratio is higher than 47%, preferably higher than 50%, it is determined that the subject is of Pca and has high malignancy of Pca.

Here, the phrase "high malignancy of Pca" corresponds to a case classified as so-called Gleason score of 4+3 or more.

Particularly, in the case where the determination method of the present invention is carried out on a patient who is difficult to distinguish whether or not Pca is developed from the serum PSA value, for example, a patient who has a total PSA value higher than zero to 50 ng/mL, in a conventional clinical examination, more detailed determination of Pca can be made. The "value higher than zero" indicates the total PSA value in the case where the total PSA is measured. Therefore, in the case where the total PSA value could not be measured, it does not correspond to the "value higher than zero".

In addition, by carrying out the determination method of the present invention on a patient having a total PSA value of 4 to 20 ng/mL, particularly a patient in a so-called gray zone having a total PSA value in the range of 4 to 10 ng/mL, it is possible to determine whether Pca is developed or the probability of developing Pca is high for a patient in the gray zone which could not be determined whether or not Pca is developed in the case of making a determination on the basis of the total PSA value which is the examination item of conventional clinical examination, which is therefore

particularly effective.

As still another determination method, the following method can be mentioned.

That is, using one or more reference solutions whose ratio of the amount of α(2,3) free PSA to the amount of free PSA is known, prepared in advance using an α(2,3) free PSA standard, the amount of α(2,3) free PSA is measured. At this time, a reference solution having a ratio of the amount of α(2,3) free PSA to the amount of free PSA, for example, 25%, 40%, or 50% is used as the reference solution. Subsequently, using a test sample, the amount of α(2,3) free PSA is measured in the same manner as described above. Then, the measurement results obtained using the reference solutions are compared with the measurement results obtained using the test sample.

In the case where the measurement results obtained using the test sample exhibit a lower value than the measurement results obtained using the reference solution of 25% and the measurement results obtained using the reference solution of 40%, it is determined to be Pca-negative.

In the case where the measurement results obtained using the test sample exhibit a higher value than the measurement results obtained using the reference solution of 40%, it is determined that Pca is developed or the probability of developing Pca is high.

In addition, in the case where the measurement results obtained using the test sample exhibit a higher value than the measurement results obtained using the reference solution of 40%, but exhibit a lower value than the measurement results obtained using the reference solution of 50%, it is determined that Pca is developed or the probability of developing Pca is high, but the malignancy of Pca is low. In the case where the measurement results obtained using the test sample exhibit a higher value than the measurement results obtained using the reference solution of 50%, it is determined that Pca is developed and the malignancy of Pca is high.

(5) Biological sample

Examples of the biological sample according to the present invention include blood, plasma, serum, semen, bladder wash, urine, tissue extract, prostate tissue section, prostate tissue biopsy sample, and those prepared therefrom. Among them, serum, plasma, or the like is preferable.

<Pca determination kit>

The Pca determination kit according to the present invention contains an affinity substance as a constituent feature.

Preferred aspects and specific examples of affinity substances and their constituent features are as described in the description of the prostate carcinoma determination method of the present invention. In addition, preferred aspects of reagent concentration and the like may also be appropriately selected from the concentration range commonly used in the art.

The kit may further contain an anti-PSA antibody according to the present invention as a constituent feature.

In addition, the reagents included in the kit may include reagents commonly used in the art, for example, buffering agents, reaction accelerators, saccharides, proteins, salts, stabilizers such as surfactants, and preservatives, which do not inhibit the stability of coexisting reagents or the like and which do not inhibit the reaction between α(2,3) free PSA and the affinity substance. In addition, the concentration thereof may be appropriately selected from the concentration range commonly used in the art.

In addition, instructions or the like for use in the Pca determination method of the present invention may be further included in the kit of the present invention. The "instructions" means an instruction manual, a package insert, a pamphlet (leaflet), or the like of the kit in which the characteristic, principle, operation procedure, determination procedure, and the like of the method are substantially described by sentences or charts.

Hereinafter, the present invention will be described in more detail with reference to Examples and the like, but the present invention is not limited in any way thereby.

EXAMPLES

Example 1: Separation/measurement of PSA and determination of ratio thereof

(1) Preparation of sample and test solution

1) Preparation of DNA-labeled anti-PSA antibody

According to the procedure shown in Fig. 2, a DNA-labeled PSA antibody Fab' fragment was prepared.

That is, first, a 250 bp DNA fragment introduced with an $NH_2$ group in the 5' terminal was purified by a conventional

method (purified terminally-aminated DNA), subsequently the $NH_2$ group introduced in this DNA fragment was reacted with a succinimide group of a sulfosuccinimidyl 4-(p-maleimidephenyl)butyrate (Sulfo-SMPB) linker (a linker having a succinimide group and a maleimide group, manufactured by Pierce Biotechnology, Inc.) by a conventional method, and then the unreacted linker was removed by subjecting the reaction solution to a gel filtration treatment, to obtain a linker-bound 250 bp DNA fragment. The obtained linker-bound 250 bp DNA fragment was reacted with an anti-PSA antibody PSA10 Fab' fragment prepared using an anti-human PSA mouse monoclonal antibody PSA10 (anti-PSA monoclonal antibody clone No. PSA 10, manufactured by Wako Pure Chemical Industries, Ltd. (own product)) according to a conventional method in advance. The obtained reaction product was purified using a DEAE column to prepare an anti-PSA antibody PSA10 Fab' fragment to which a 250 bp DNA fragment was bound (hereinafter, abbreviated as "DNA-labeled anti-PSA antibody").

It should be noted that the anti-human PSA mouse monoclonal antibody (Anti-PSA monoclonal antibody clone No. PSA10) used is an antibody having an affinity for human PSA and it can bind to both complexed PSA and free PSA. That is, the antibody binds to both α(2,3) free PSA and free PSA other than α(2,3) free PSA.

2) Preparation of fluorescently labeled anti-free PSA antibody

An anti-human PSA monoclonal antibody PSA12 which recognizes an epitope of PSA different from an anti-PSA monoclonal antibody PSA10 and specifically binds only to free PSA (Anti-PSA monoclonal antibody clone No. PSA12, manufactured by Wako Pure Chemical Industries, Ltd. (own product)) was treated by a conventional method to obtain an anti-PSA antibody PSA12 Fab' fragment. A fluorescent substance HiLyte 647 (manufactured by AnaSpec, Inc.) was introduced into an amino group of the resulting fragment by a conventional method, whereby a HiLyte 647-labeled anti-free PSA antibody PSA12 Fab' fragment (hereinafter, abbreviated as "fluorescently labeled anti-free PSA antibody") was obtained.

(2) Electrophoresis (microchip capillary electrophoresis)

Microchip capillary electrophoresis was carried out using a fully automated fluorescence immunoassay system μTAS Wako i30 (manufactured by Wako Pure Chemical Industries, Ltd.) according to the instructions of the apparatus by the following procedure.

1) Preparation of electrophoresis sample A

According to the method disclosed in 2. Materials and methods (2.7 Forced expression of FLAG-tag-fused S2,3PSA) of Non-Patent Literature 6, recombinant free PSA (hereinafter, abbreviated as "r free PSA") [containing recombinant α(2,3) free PSA (hereinafter, abbreviated as "r α(2,3) free PSA") and recombinant α(2,6) free PSA (hereinafter, abbreviated as "r α(2,6) free PSA")] was obtained. The concentration of PSA in the obtained r free PSA solution was measured, diluted with PBS(-) (manufactured by Wako Pure Chemical Industries, Ltd.) and adjusted to a 1 ng/mL PSA protein concentration to obtain a sample solution. To a 0.5 mL tube, 2 μL of the obtained sample solution, 1 μL of the 1 μM fluorescently labeled anti-free PSA antibody prepared in the section (1) 2), and 7 μL of electrophoresis buffer solution 1 [containing 5% (w/v) polyethylene glycol (PEG20000), 3% (w/v) glycerol, 150 mM NaCl, 0.01% BSA, 75 mM Tris-HCl, and 10 mM MES, pH 7.5] were added and mixed to prepare 10 μL of a reaction solution.

It should be noted that the final concentration of the fluorescently labeled anti-free PSA antibody in this reaction solution is 100 nM.

A reaction solution containing the [fluorescently labeled anti-free PSA antibody-r free PSA] complex obtained by the reaction mentioned above (that is, a solution containing the [fluorescently labeled anti-free PSA antibody-r α(2,3) free PSA] complex and the [fluorescently labeled anti-free PSA antibody-r α(2,6) free PSA] complex) (10 μL) was used as electrophoresis sample A.

2) Preparation of electrophoresis test solution

The following test solutions were prepared.

• Electrophoresis buffer solution 2 (containing MAA)

A 75 mM Tris-HCl buffer (pH 7.5) containing 4.5% (w/v) polyethylene glycol (PEG8000), 3% (w/v) glycerol, 10 mM NaCl, and 0.01% BSA was prepared. MAA (manufactured by VECTOR Co., Ltd.) was added thereto to a final concentration of 4 mg/mL and mixed to prepare electrophoresis buffer solution 2.

• Electrophoresis buffer solution 3

A buffer (pH not adjusted) containing 2% (w/v) polyethylene glycol (PEG20000), 3% (w/v) glycerol, 0.01% BSA, 125 mM HEPES, and 75 mM Tris-HCl was used as electrophoresis buffer solution 3.

• Electrophoresis buffer solution 4

A 75 mM Tris-HCl buffer (pH 7.5) containing 2% (w/v) polyethylene glycol (PEG20000), 3% (w/v) glycerol, and 0.01% BSA was used as electrophoresis buffer solution 4.

• DNA-labeled antibody solution (containing DNA-labeled anti-PSA antibody)

A buffer [containing 2% (w/v) polyethylene glycol (PEG20000), 0.5 mM EDTA(2Na), 3% (w/v) glycerol, 50 mM NaCl, 0.01% BSA, and 75 mM BisTris (pH 6.0)] containing 100 nM of the DNA-labeled anti-PSA antibody obtained in the section (1) 1) was prepared as a DNA-labeled antibody solution.

• Fluorescent solution

As a fluorescent solution, 50 mM BisTris (pH 6.0) containing 30 nM HiLyte 647 and 20% (w/v) glycerol was used. The fluorescent solution is used for adjustment such as position confirmation at the detection part of the measuring apparatus (μTAS Wako i30).

3) Electrophoresis procedure

i) Introduction of electrophoresis sample A and electrophoresis test solution

Into predetermined wells (SP wells) of μTAS Wako i30 dedicated microchip, 5.4 μL of the electrophoresis sample A prepared in the section (2) 1) was dispensed. Next, each of the test solutions prepared in the section (2) 2) was dispensed to each well of the microchip as described below.

- R2 well (R2(FLB) well, R2(LB) well): 10.0 μL of electrophoresis buffer solution 2,
- R3 well: 10.0 μL of electrophoresis buffer solution 3,
- R4 well: 5.4 μL of electrophoresis buffer solution 4,
- C1 well: 3.0 μL of DNA-labeled antibody solution,
- FD well: 7.0 μL of fluorescent solution.

A schematic diagram of the microchip used is shown in Fig. 3.
In Fig. 3, the Waste well is used as a waste reservoir (drain well) at the time of introducing the test solution of each well (R2, R3, R4, and C1) and the electrophoresis sample A into an analysis flow channel.
Subsequently, a pressure of -5 psi was applied between each of the four Waste wells (drain wells) for 30 seconds to introduce the electrophoresis sample A and each test solution into the analysis flow channel of the chip.

ii) ITP (reaction, concentration, separation) · detection

Fig. 4 schematically shows an in-chip flow channel of the microchip used.
In Fig. 4, W represents a Waste well. The R3 well side serves as a cathode and the R2(LB) well side serves as an anode. In addition, in Fig. 4, the placement portion of the electrophoresis sample A and the test solution in each well is indicated by coloring it into a dot portion and a white portion (a portion without a dot).
After introducing the electrophoresis sample A and each test solution into the analysis flow channel of the chip, PSA was separated and detected by the following method.
A voltage of 4000 V was applied between the R3 well and the R2(LB) well in Fig. 4, and the DNA-labeled anti-PSA antibody in the test solution was brought into contact with the [fluorescently labeled anti-free PSA antibody-r free PSA] complex in the electrophoresis sample A at 30°C to prepare a [fluorescently labeled anti-free PSA antibody-r free PSA-DNA-labeled anti-PSA antibody] complex, which was then concentrated by isotachophoresis (ITP). The migration direction of isotachophoresis is indicated by "ITP" and dotted line in Fig. 4.
The immune reaction time with each labeled antibody to capture free PSA was about 200 seconds.
Specifically, the complexes formed here are a [fluorescently labeled anti-free PSA antibody-r α(2,3) free PSA-DNA-labeled anti-PSA antibody] complex (Complex 1) and a [fluorescently labeled anti-free PSA antibody-r α(2,6) free

PSA-DNA-labeled anti-PSA antibody] complex (Complex 2).

After the complex was subjected to isotachophoresis to the R2(FLB) well and its passing through the R2(FLB) well was judged from a change in voltage, a cathode electrode was switched from R3 to R2(FLB). Then, the capillary gel electrophoresis (CE) was further carried out in the presence of MAA until a peak of the [fluorescently labeled anti-free PSA antibody-r free PSA-DNA-labeled anti-PSA antibody] complex was detected in the detection portion (a capillary portion 2 cm downstream from the channel crossing portion of R2(FLB) and R2(LB)). The position where CE was carried out and the electrophoresis direction of electrophoresis are indicated by "CE" and dotted line in Fig. 4.

It should be noted that the detection was carried out by measuring over time an intensity of fluorescence generated by 635 nm laser excitation in the capillary portion 2 cm downstream from the channel crossing portion of R2(FLB) and R2(LB), using a photodiode (manufactured by Fuji Film Co., Ltd.).

In addition, PSA was separated and detected in such a manner that the same method as described above was carried out using the same electrophoresis sample A and electrophoresis test solution and measuring apparatus as described above, except that electrophoresis buffer solution 2 not containing MAA was used.

(3) Results

The obtained electrophoretic profile (electropherogram) is shown in Fig. 5. In Fig. 5, the vertical axis represents the fluorescence intensity and the horizontal axis represents the mobility (sec).

In addition, the electrophoretic profile (electropherogram) obtained by carrying out the detection in the same manner using electrophoresis buffer solution 2 not containing MAA is shown in Fig. 5 together with a gray line (thin line of color). In addition, the peak appearing in this detection is shown as "Lectin(-)" in Fig. 5.

Since the [fluorescently labeled anti-free PSA antibody-r α(2,3) free PSA-DNA-labeled anti-PSA antibody] complex (Complex 1) which reacts with MAA takes longer time to migrate as compared with the [fluorescently labeled anti-free PSA antibody-r α(2,6) free PSA-DNA-labeled anti-PSA antibody] complex (Complex 2) which does not react with MAA, the appearance of the peak is delayed. That is, the peak of Complex 2 is the peak that appeared at the same position as the peak of "Lectin(-)", and the peak of Complex 1 is the peak that appeared immediately after the peak of Complex 2.

The peak area of the fraction of Complex 1 and the peak area of the fraction of Complex 2 obtained were determined with analysis software attached to the measuring apparatus.

Subsequently, using the peak area of the fraction of Complex 1 and the peak area of the fraction of Complex 2 obtained, the ratio (%) of the amount of α(2,3) free PSA to the amount of free PSA in a sample was calculated.

Specific calculation methods are as follows.

- ·Amount of free PSA (total amount of free PSA)=[peak area of fraction of Complex 1]+[peak area of fraction of Complex 2]

- ·Ratio (%) of amount of α(2,3) free PSA to amount of free PSA=[peak area of fraction of Complex 1]/[amount of free PSA]×100

As a result, the ratio of the amount of α(2,3) free PSA to the amount of free PSA was "49.9%".

On the basis of the results, it was found that Complex 1 (α(2,3) free PSA) and Complex 2 (free PSA other than α(2,3) free PSA; α(2,6) free PSA in this Example) can be separated and measured by capillary electrophoresis, and the ratio of the amount of α(2,3) free PSA to the amount of free PSA can be determined using the peak area obtained on the basis of the measurement.

Example 2: Confirmation of capture efficiency

(1) Preparation of electrophoresis sample A containing r α(2,3) free PSA or r α(2,6) free PSA

According to the method disclosed in 2. Materials and methods (2.7 Forced expression of FLAG-tag-fused S2,3PSA) of Non-Patent Literature 6, r free PSA (containing r α(2,3) free PSA and r α(2,6) free PSA) was obtained. From the obtained r free PSA, r α(2,3) free PSA and r α(2,6) free PSA were separated and purified. In the separation and purification method, first, r α(2,3) free PSA and r α(2,6) free PSA were separated by lectin column chromatography using an ACG lectin column (J-Oil Mills, Inc.) showing a high affinity for the sialyl α2,3-galactose structure. Then, gel

filtration was carried out to purify r α(2,3) free PSA and r α(2,6) free PSA, respectively.

Finally, 1.3 μg of r α(2,3) free PSA and 2.1 μg of r α(2,6) free PSA were obtained starting from r free PSA (29 μg).

Each of the obtained r α(2,3) free PSA and r α(2,6) free PSA was diluted with PBS(-) (manufactured by Wako Pure Chemical Industries, Ltd.) and adjusted to a 1 ng/mL PSA protein concentration to obtain a sample solution. To a 0.5 mL tube, 2 μL each of the obtained sample solution, 1 μL of the 1 μM fluorescently labeled anti-free PSA antibody prepared in the section (1) 2) of Example 1, and 7 μL of electrophoresis buffer solution 1 [containing 5% (w/v) polyethylene glycol (PEG20000), 3% (w/v) glycerol, 150 mM NaCl, 0.01% BSA, 75 mM Tris-HCl, and 10 mM MES, pH 7.5] were added and mixed to prepare 10 μL of each reaction solution.

It should be noted that the final concentration of the fluorescently labeled anti-free PSA antibody in the reaction solution is 100 nM.

The reaction solution (10 μL) containing the [fluorescently labeled anti-free PSA antibody-r α(2,3) free PSA] complex obtained by the reaction was taken as electrophoresis sample A containing r α(2,3) free PSA. In addition, the reaction solution (10 μL) containing the [fluorescently labeled anti-free PSA antibody-r α(2,6) free PSA] complex obtained by the reaction was taken as electrophoresis sample A containing r α(2,6) free PSA.

(2) Electrophoresis (microchip capillary electrophoresis)

The microchip capillary electrophoresis was carried out in the same manner as in Example 1, using the same electrophoresis test solution, measuring apparatus, and the like as those used in Example 1 and using the same reagents, measuring apparatus, and the like as in Example 1, except that the electrophoresis sample A containing r α(2,3) free PSA or the electrophoresis sample A containing r α(2,6) free PSA prepared in the section (1) was used.

(3) Results

The obtained electrophoretic profile (electropherogram) is shown in Figs. 6(1) and 6(2). In Figs. 6(1) and 6(2), the vertical axis represents the fluorescence intensity and the horizontal axis represents the mobility (sec).

The results obtained using the electrophoresis sample A containing r α(2,3) free PSA are shown in Fig. 6(1) together with a gray line (thin line of color).

In addition, an electrophoretic profile (electropherogram) obtained by carrying out the measurement in the same manner using electrophoresis sample A containing r α(2,3) free PSA and electrophoresis buffer solution 2 not containing MAA is shown in Fig. 6(1) together with a black line (dark line of color).

The results obtained using electrophoresis sample A containing r α(2,6) free PSA are shown in Fig. 6(2) together with a gray line (thin line of color).

In addition, an electrophoretic profile (electropherogram) obtained by carrying out the measurement in the same manner using electrophoresis sample A containing r α(2,6) free PSA and electrophoresis buffer solution 2 not containing MAA is shown in Fig. 6(2) together with a black line (dark line of color).

Since the interaction occurs between MAA and a glycan structure of α(2,3) free PSA with MAA affinity, that is, a glycan in which the terminal sialic acid residue of the glycan is α(2,3)-linked to the second galactose residue from the terminal of the glycan, the electrophoretic peak of α(2,3) free PSA obtained by the measurement using electrophoresis buffer solution 2 containing MAA should appear after the electrophoretic peak of α(2,3) free PSA obtained by the measurement using electrophoresis buffer solution 2 not containing MAA. As is clear from Fig. 6(1), it was confirmed that the electrophoretic peak of α(2,3) free PSA obtained by the measurement using electrophoresis buffer solution 2 containing MAA was delayed from the appearance position of the electrophoretic peak of α(2,6) free PSA obtained by the measurement using electrophoresis buffer solution 2 not containing MAA.

Next, on the basis of the results in Fig. 6(1), the area of the obtained peak was determined with analysis software attached to the apparatus. As a result, the peak area of α(2,3) free PSA obtained by the measurement using electrophoresis buffer solution 2 containing MAA was almost 100% of the peak area of α(2,3) free PSA obtained by the measurement using electrophoresis buffer solution 2 not containing MAA.

On the other hand, since α(2,6) free PSA does not have a glycan in which the terminal sialic acid residue of the glycan is α(2,3)-linked to the second galactose residue from the terminal of the glycan, no interaction with MAA occurs. Therefore, the electrophoretic peak of α(2,6) free PSA obtained by the measurement using electrophoresis buffer solution 2 containing MAA should appear at the same position as the electrophoretic peak of α(2,6) free PSA obtained by the measurement using electrophoresis buffer solution 2 not containing MAA. As is clear from Fig. 6(2), no change was observed in the electrophoretic peak position between the electrophoretic peak of α(2,6) free PSA obtained by the measurement using electrophoresis buffer solution 2 containing MAA and the electrophoretic peak of α(2,6) free PSA obtained by the measurement using electrophoresis buffer solution 2 not containing MAA.

Next, on the basis of the results in Fig. 6(2), the area of the obtained peak was determined with analysis software attached to the apparatus. As a result, the peak area of α(2,3) free PSA obtained by the measurement using electrophoresis buffer solution 2 containing MAA was almost 100% of the peak area of α(6,3) free PSA obtained by the measurement using electrophoresis buffer solution 2 not containing MAA.

From the results, it was confirmed in the present measurement system that the efficiency of capturing α(2,3) free PSA and α(6,3) free PSA by the interaction between "the MAA and the glycan in which the terminal sialic acid residue of the glycan is α(2,3)-linked to the second galactose residue from the terminal of the glycan" was almost 100%. In other words, it was demonstrated that the present measurement system is capable of measuring the amount of α(2,3) free PSA and the amount of α(6,3) free PSA without loss.

It should be noted that the present inventors have found that the method of measuring the amount of α(2,3) free PSA described in Patent Literature 1 has a low efficiency of capturing α(2,3) free PSA (less than 80%).

Example 3

(1) Preparation of electrophoresis sample A

The r α(2,3) free PSA and r α(2,6) free PSA prepared in Example 2 were each diluted with PBS(-) (manufactured by Wako Pure Chemical Industries, Ltd.) and adjusted to have a 1.5 ng/mL PSA protein concentration. Subsequently, the r α(2,3) free PSA solution was diluted with the resulting r α(2,6) free PSA solution to obtain a sample solution containing r α(2,3) free PSA in an amount of 10%, 20%, 30%, 40%, or 50%. The content rate of this r α(2,3) free PSA is defined as "theoretical value".

To a 0.5 mL tube, 2 μL of the obtained sample solution, 1 μL of the 1 μM fluorescently labeled anti-free PSA antibody prepared in the section (1) 2) of Example 1, and 7 μL of electrophoresis buffer solution 1 [containing 5% (w/v) polyethylene glycol (PEG20000), 3% (w/v) glycerol, 150 mM NaCl, 0.01% BSA, 75 mM Tris-HCl, and 10 mM MES, pH 7.5] were added and mixed to prepare 10 μL of a reaction solution.

The reaction solution containing the [fluorescently labeled anti-free PSA antibody-free PSA] complex obtained by the reaction (that is, a solution (10 μL) containing a [fluorescently labeled anti-free PSA antibody-r α(2,3) free PSA] complex and a [fluorescently labeled anti-free PSA antibody-r α(2,6) free PSA] complex) was used as electrophoresis sample A.

It should be noted that the final concentration of the fluorescently labeled anti-free PSA antibody in this reaction solution is 100 nM.

(2) Electrophoresis (microchip capillary electrophoresis)

The microchip capillary electrophoresis was carried out in the same manner as in Example 1, using the same electrophoresis test solution, measuring apparatus, and the like as those used in Example 1, except that the electrophoresis sample A prepared in the section (1) was used.

(3) Calculation of ratio

On the basis of the obtained electrophoretic profile (electropherogram), the peak area of Complex 1 ([fluorescently labeled anti-free PSA antibody-r α(2,3) free PSA] complex) and the peak area of Complex 2 ([fluorescently labeled anti-free PSA antibody-r α(2,6) free PSA] complex) were determined with analysis software attached to the apparatus. The obtained value may be referred to simply as "actual measurement value".

Then, on the basis of the peak area of the fraction of Complex 1 and the peak area of the fraction of Complex 2, the ratio (%) of the amount of α(2,3) free PSA to the amount of free PSA in the sample was determined by the same calculation method as in Example 1.

(4) Results

The results are shown in Figs. 7(1) and 7(2).

Fig. 7(1) shows the relationship between the ratio (%) of the amount of α(2,3) free PSA to the amount of free PSA calculated on the basis of the actual measurement value of each sample solution and the content (theoretical value) of α(2,3) free PSA of the sample solution used. In Fig. 7(1), ■ indicates the protein concentration of free PSA in each sample solution (fPSA). Protein concentrations in the respective samples used are all 1.5 ng/mL. ♦ indicates the ratio (%) of the amount of α(2,3) free PSA to the amount of free PSA in each sample solution calculated on the basis of the actual measurement value.

As shown in Fig. 7(1), the theoretical value (α(2,3) PSA ratio (%)) of the ratio of the amount of α(2,3) free PSA to the amount of free PSA in the used sample solution almost agreed with the ratio (α(2,3) PSA ratio (%)) of the amount of α(2,3) free PSA to the amount of free PSA calculated on the basis of the actual measurement value. For example,

the ratio (%) of the amount of α(2,3) free PSA to the amount of free PSA in the sample solution containing r α(2,3) free PSA in an amount of 10% (theoretical value) was 10% in terms of the theoretical value, whereas the calculated ratio on the basis of the actual measurement value was almost 10%.

Fig. 7(2) shows the relationship between the actual measurement value (■, peak area) of the peak area of the fraction of Complex 1 or the actual measurement value (♦, peak area) of the peak area of the fraction of Complex 2 obtained in the section (3) for each sample solution and the theoretical value of the sample solution.

The peak area of the fraction of Complex 1 reflects the concentration of α(2,3) free PSA, and the peak area of the fraction of Complex 2 reflects the concentration of α(2,6) free PSA.

For example, from Fig. 7(2), in the case where a sample with a theoretical value (α(2,3) PSA ratio) of 10% was used, the actual measurement value (■) of the peak area of the fraction of Complex 1 was about 2.7, and the actual measurement value (♦) of the peak area of the fraction of Complex 2 was about 24.2. From the foregoing, in the case of calculating on the basis of the actual measurement values,

$$\alpha(2,3)\text{ free PSA concentration/free PSA concentration}$$
$$= \text{peak area of fraction of Complex 1/(peak area of fraction of Complex 1+peak area of fraction of Complex 2)}$$
$$= 2.7/(2.7+24.2)\times 100=10.0\%$$

That is, the ratio of the amount of α(2,3) free PSA to the amount of free PSA calculated on the basis of the actual measurement values was almost the same as the theoretical value.

From the foregoing, it was confirmed that the ratio (%) of the amount of α(2,3) free PSA to the amount of free PSA obtained by the measuring method of this Example almost agreed with the actual ratio (%) of the amount of α(2,3) free PSA to the amount of free PSA.

Therefore, it was confirmed that an accurate "ratio (%) of the amount of α(2,3) free PSA to the amount of free PSA" can be obtained by carrying out the measuring method of this Example. In addition, it was confirmed that the measurement values of α(2,3) free PSA and α(2,6) free PSA show linearity. These results demonstrate that the efficiency of capturing α(2,3) free PSA in the present measurement system is almost 100%.

Example 4: Comparison of ratios obtained using samples derived from Pca patients and BPH patients

(1) Preparation of electrophoresis sample A

Sera collected from 22 patients with prostate carcinoma (Pca) who had a total PSA value of 10.0 ng/mL or less and 24 patients with non-cancerous benign prostatic hyperplasia (BPH) who had a total PSA value of 10.0 ng/mL or less were used as samples. Histopathological diagnosis of each patient was confirmed by prostate biopsy. The patient's background (age, PSA value (total PSA value), histopathological malignancy classification, and clinical stage) are shown in Table 1.

[Table 1]

| Items | Benign prostatic hyperplasia | | Prostate carcinoma | | p |
|---|---|---|---|---|---|
| Number of specimens (n) | 24 | | 22 | | |
| Age (median) | 53-82 | (67.0) | 51-79 | (68.5) | 0.7557 |
| PSA, ng/ml, (median) | 4.20-9.56 | (5.85) | 5.43-9.97 | (7.04) | 0.0289 |
| S2,3PSA, MFI (median) | 829-2030 | (1281) | 1132-2720 | (1872) | 0.0002 |
| %S2,3PSA (median) | 22.9-46.0 | (34.7) | 31.1-68.3 | (45.2) | <0.0001 |
| Total Gleason score at biopsy, n (%) | | | | | |
| 7 | | | 13 | | |
| 8 | | | 5 | (59.0) | |
| 9 | | | 4 | (22.7) | |
| Clinical stage, n, (%) | | | | (18.3) | |
| cT1c-cT2a | | | 18 | (81.6) | |
| cT2a | | | 3 | (13.6) | |

(continued)

| Items | Benign prostatic hyperplasia | Prostate carcinoma | | p |
|---|---|---|---|---|
| cT2b | | 1 | (4.8) | |

To a 0.5 mL tube, 2 μL of the sample, 1 μL of the 1 μM fluorescently labeled anti-free PSA antibody prepared in the section (1) 2) of Example 1, and 7 μL of electrophoresis buffer solution 1 [containing 5% (w/v) polyethylene glycol (PEG20000), 3% (w/v) glycerol, 150 mM NaCl, 0.01% BSA, 75 mM Tris-HCl (pH 7.5), and 10 mM MES] were added and mixed to prepare 10 μL of a reaction solution.
The reaction solution (10 μL) containing the [fluorescently labeled anti-free PSA antibody-free PSA] complex obtained by the reaction was used as electrophoresis sample A.

(2) Separation/measurement of PSA and determination of ratio thereof

The microchip capillary electrophoresis was carried out in the same manner as in Example 1, using the same electrophoresis test solution, measuring apparatus, and the like as those used in Example 1, except that the electrophoresis sample A prepared in the section (1) was used. Then, the ratio of the amount of α(2,3) free PSA to the amount of free PSA in each sample was determined in the same manner as in Example 1.
For the values obtained, a significant difference test (Mann-Whitney U-TEST) was carried out between Pca patients and BPH patients.

(3) Results

The obtained results are shown in Fig. 8.
As is clear from Fig. 8, the P value obtained by comparing the ratios (α(2,3) PSA ratio (%)) of the amount of α(2,3) free PSA to the amount of free PSA between Pca patients and BPH patients was "$P<0.0001$". Therefore, it was found that the ratio of the amount of α(2,3) free PSA to the amount of free PSA in the Pca patient-derived sample was significantly higher than the ratio of the amount of α(2,3) free PSA to the amount of free PSA in the BPH patient-derived sample.

Comparative Examples 1 and 2: Comparison with conventional determination method

(1) Sample

The same sample as used in Example 4 was used.

(2) Measurement of conventional determination items

The total PSA value in each sample was determined according to the protocol attached to the kit, using Lumipulse Presto PSA (Fujirebio, Inc.) which is an *in vitro* diagnostics (Comparative Example 1).
In addition, the free PSA value in each sample was determined using the Human Circulating Cancer BioMarker Panel 1 Select Kit (manufactured by LUMINEX Corporation), and according to the protocol attached to the kit.
On the basis of the obtained results, the ratio of the free PSA value to the total PSA value in each sample was determined (Comparative Example 2).
For each value obtained, a significant difference test (Mann-Whitney U-TEST) was carried out between Pca patients and BPH patients.

(3) Results

The results thus obtained, and the results obtained in Example 4 are also shown in the upper panel of Figs. 9(1) to 9(3).
The upper panel of Fig. 9(1) shows the results of comparing the ratios (α(2,3) PSA ratios) of the amount of α(2,3) free PSA to the amount of free PSA obtained in Example 4, between Pca patients and BPH patients (the same as in Fig. 2).
The upper panel in Fig. 9(2) shows the results of comparing the total PSA values (total PSA) between Pca patients and BPH patients (Comparative Example 1).
The upper panel of Fig. 9(3) shows the results of comparing the ratios (%fPSA) of the free PSA value to the

total PSA value between Pca patients and BPH patients (Comparative Example 2).

As is clear from Fig. 9(1) (upper panel), the P value obtained by comparing the ratios of the amount of $\alpha$(2,3) free PSA to the amount of free PSA between Pca patients and BPH patients was "<0.0001" (Fig. 9(1), upper panel). On the other hand, as a result of comparing the total PSA values between Pca patients and BPH patients, the P value was 0.0289 (Fig. 9(2), upper panel). In addition, as a result of comparing the ratios of the free PSA value to the total PSA value between Pca patients and BPH patients, the P value was 0.1458 (Fig. 9(3), upper panel).

From the foregoing, it was found that the method of determining Pca using the ratio of the amount of $\alpha$(2,3) free PSA to the amount of free PSA of the present invention is capable of achieving Pca determination with higher accuracy than a conventional method of determining Pca using the total PSA value or the ratio of the free PSA value to the total PSA value.

In addition, on the basis of these results, a Relative Operating Characteristic curve (ROC curve) analysis was further carried out. The results are also shown in the lower panel of Fig. 9.

As is clear from Fig. 9(1) (lower panel), as a result of determining Pca by the ratio of the amount of $\alpha$(2,3) free PSA to the amount of free PSA of the present invention, Area Under the Curve (AUC) was 0.8580 (Fig. 9(1), lower panel). On the other hand, as a result of determining Pca by a conventional total PSA value, AUC was 0.6875 (Fig. 9(2), lower panel). As a result of determining Pca by a conventional ratio of the free PSA value to the total PSA value, AUC was 0.6275 (Fig. 9(3), lower panel).

That is, it was found that the determination method of the present invention is superior in sensitivity and specificity of the measurement system as compared with a conventional determination method. In addition, it was found that since the results were obtained using a sample derived from a patient having a total PSA value of 10.0 ng/mL or less in a sample which is a gray zone for Pca determination, the determination method of the present invention is capable of making Pca determination for a patient having a total PSA value in the gray zone with higher accuracy than a conventional determination method.

Example 5: Test of cutoff value

(1) Preparation of electrophoresis sample A

Using serum samples derived from a total of 89 patients including serum samples used in Example 4 and serum samples derived from 43 patients (Pca-positive; 26 cases, and Pca-negative; 17 cases) showing a total PSA value of 10 to 50 ng/mL, electrophoresis sample A was prepared in the same manner as in Example 4(1).

(2) Separation/measurement of PSA and determination of ratio thereof

The microchip capillary electrophoresis was carried out in the same manner as in Example 1, using the same electrophoresis test solution, measuring apparatus, and the like as those used in Example 1, except that the electrophoresis sample A prepared in the section (1) was used. Then, the ratio of the amount of $\alpha$(2,3) free PSA to the amount of free PSA in each sample was determined in the same manner as in Example 1.

(3) Test of cutoff value

On the basis of the results obtained in the section (2), a Relative Operating Characteristic curve (ROC curve) analysis was carried out. The results are shown in Fig. 10.

Next, as usual, a straight line with an angle of 45 degrees in contact with the ROC curve was drawn, and the value of the intersection point with the straight line (indicated by the arrow in Fig. 10), that is, the value at which the "sensitivity-(1-specificity)" was the maximum was determined. As a result, the value (ratio of the amount of $\alpha$(2,3) free PSA to the amount of free PSA) was "43.1%".

Accordingly, in the verification of this Example, the cutoff value in the case of making Pca determination on the basis of the ratio of the amount of $\alpha$(2,3) free PSA to the amount of free PSA was "43.1%" (Fig. 10).

Example 6: Comparison with determination by known cutoff value

(1) Sample

WO2014/057983A (Patent Literature 3) discloses that the fluorescence intensity (MFI: Mean Fluorescence Intensity) in each sample was measured by the method described in Example 2 and the value was taken as the amount of $\alpha$(2,3) free PSA in each sample, and the cutoff value for Pca determination was defined as "fluorescence intensity (MFI)=1130". However, in the case where the present inventors measured the amount of $\alpha$(2,3) free PSA of the samples

(46 cases in total) used in Example 4 of the present specification, using the method described in Example 2 of Patent Literature 3, there was a case where it was actually negative (BPH) even in the case where the fluorescence intensity (MFI) was 1130 or more (false positive). That is, it was suggested that determination of Pca based only on the amount of α(2,3) free PSA may result in determination of false positives.

Therefore, using the sample of 5 specimens whose fluorescence intensity (MFI) was higher than the cutoff value "1130" defined in Patent Literature 3 despite being negative (BPH) as a result of the measurement by the present inventors, comparative study was made between the determination method of the present invention and the known determination method disclosed in Patent Literature 3.

(2) Determination of ratio of amount of α(2,3) free PSA to amount of free PSA

Electrophoresis samples A were prepared in the same manner as in Example 4(1) using the samples of 5 specimens selected in the section (1).

Next, the microchip capillary electrophoresis was carried out in the same manner as in Example 1, using the same electrophoresis test solution, measuring apparatus, and the like as those used in Example 1, except that the electrophoresis sample A prepared in above was used. Then, the ratio of the amount of α(2,3) free PSA to the amount of free PSA in each sample was determined in the same manner as in Example 1. The results are shown in Table 2.

(3) Measurement of α(2,3) free PSA

The amount of α(2,3) free PSA (fluorescence intensity (MFI)) was measured by carrying out the same method as described in Example 2 of Patent Literature 3, using the samples of 5 specimens of the section (1). The results are also shown in Table 2.

(4) Results

In Table 2, the "Ratio" represents the ratio of the amount of α(2,3) free PSA to the amount of free PSA in each sample.

In Table 2, the "MFI" represents the results of measuring the fluorescence intensity (MFI) in each sample by the method of the section (3).

[Table 2]

| Specimen No. | Ratio | MFI |
|---|---|---|
| 1 | 22.90% | 1166 |
| 2 | 29.90% | 1785 |
| 3 | 32.20% | 1235 |
| 4 | 30.90% | 1381 |
| 5 | 26.40% | 1190 |

The cutoff value in the case of determining Pca using the amount of α(2,3) free PSA determined by Patent Literature 3 as an index is 1130 in terms of fluorescence intensity (MFI). As is clear from the results in Table 2, the fluorescence intensity (MFI) was ≥1130 despite all the five specimens to be measured this time were negative (BPH). Therefore, as a result of determining Pca on the basis of the cutoff value described in Patent Literature 3, all 5 specimens were determined as Pca (determined as false positive).

On the other hand, as a result of determination using the cutoff value "43.1 %" of the ratio of the amount of α(2,3) free PSA to the amount of free PSA determined in Example 5, all of the ratios of the 5 specimens to be measured were below this cutoff value, and all were determined as negative.

From the foregoing, it was found that the determination method using the cutoff value on the basis of the ratio of the amount of α(2,3) free PSA to the amount of free PSA according to the present invention as an index is a method capable of carrying out determination of Pca with higher accuracy than a determination method using a cutoff value on the basis of the known α(2,3) free PSA amount as an index.

Example 7

(1) Sample

Sera collected from 28 patients with prostate carcinoma (Pca) who had a total PSA value of 20.0 ng/mL or less and 28 patients with non-cancerous benign prostatic hyperplasia (BPH) who had a total PSA value of 20.0 ng/mL or less were used as samples.

It should be noted that the samples used here were samples determined to be difficult to determine cancer, as a result of determination by using, as an index, the total PSA test and the ratio of the free PSA to the total PSA value which are conventional determination markers.

(2) Determination of ratio of amount of α(2,3) free PSA to amount of free PSA

Electrophoresis sample A was prepared in the same manner as in Example 4(1) using the sample of the section (1).

Next, the microchip capillary electrophoresis was carried out in the same manner as in Example 1, using the same electrophoresis test solution, measuring apparatus, and the like as those used in Example 1, except that the electrophoresis sample A prepared was used. Then, the ratio of the amount of α(2,3) free PSA to the amount of free PSA in each sample was determined in the same manner as in Example 1.

For the values obtained, a significant difference test (Mann-Whitney U-TEST) was carried out between Pca patients and BPH patients.

(3) Results

The results are shown in Figs. 11(1) and 11(2).

Fig. 11(1) shows the ratio (α(2,3) PSA ratio (%)) of the amount of α(2,3) free PSA to the amount of free PSA in each sample.

In addition, on the basis of the results in Fig. 11(1), a Relative Operating Characteristic curve (ROC curve) analysis was carried out. The results are shown in Fig. 11(2) ((a) of Fig. 11(2)).

Comparative Examples 3 and 4

(1) Measurement of total PSA value (Comparative Example 3)

Using the same sample as used in Example 7, the total PSA value in each sample was determined according to the protocol attached to the kit, using Lumipulse Presto PSA (Fujirebio, Inc.) which is an *in vitro* diagnostics. In addition, for the obtained values, a significant difference test (Mann-Whitney U-TEST) was carried out between Pca patients and BPH patients.

The results are shown in Fig. 12(1).

In addition, on the basis of the obtained results, a Relative Operating Characteristic curve (ROC curve) analysis was carried out. The results are also shown in Fig. 11(2) ((c) of Fig. 11(2)).

(2) Measurement of α(2,3) free PSA (Comparative Example 4)

Using the same sample as used in Example 7, the same method as described in Example 2 of Patent Literature 3 (WO2014/057983A) was carried out to measure the amount (Fluorescence intensity (MFI)) of α(2,3) free PSA. In addition, for the obtained value, a significant difference test (Mann-Whitney U-TEST) was carried out between Pca patients and BPH patients.

The results are also shown in Fig. 12(2).

In addition, on the basis of the obtained results, a Relative Operating Characteristic curve (ROC curve) analysis was carried out. The results are also shown in Fig. 11(2) ((b) of Fig. 11(2)).

(3) Results

1) Results of ROC curve analysis

The results of the ROC curve analysis obtained in Example 7 and Comparative Examples 3 and 4 are also shown in Fig. 11(2).

As a result of the analysis, AUC=0.7423 in the ratio of the amount of $\alpha$(2,3) free PSA to the amount of free PSA (Example 7, (a) of Fig. 11(2)). On the other hand, AUC=0.5344 in the amount of $\alpha$(2,3) free PSA (Comparative Example 4, (b) of Fig. 11(2)). In the amount of total PSA (Comparative Example 3, (c) of Fig. 11(2)), the value of AUC=0.5064.

In addition, in the case where the ratio of the amount of $\alpha$(2,3) free PSA to the amount of free PSA=40% was taken as the cutoff value in the determination of prostate carcinoma, the sensitivity was 85.7% and the specificity was 46.4%. On the other hand, in the case where the sensitivity of 85.7% was achieved in known Pca markers, amount of $\alpha$(2,3) free PSA (b) and amount of total PSA (c), the specificities were 17.9% and 14.3%, respectively.

From the foregoing, it was confirmed that the prostate carcinoma determination method of the present invention, in which the ratio of the amount of $\alpha$(2,3) free PSA to the amount of free PSA=40% is taken as the cutoff value in the determination of prostate carcinoma, is an excellent determination method with higher sensitivity and specificity for prostate carcinoma determination than the conventional determination method, even in cases where it is difficult to make a determination.

2) Results of significant difference test

As is clear from Fig. 11(1), the P value obtained by comparing the ratios ($\alpha$(2,3) PSA ratio (%)) of the amount of $\alpha$(2,3) free PSA to the amount of free PSA between Pca patients and BPH patients was "P=0.0019". Therefore, it was found that the ratio of the amount of $\alpha$(2,3) free PSA to the amount of free PSA in the sample derived from Pca patients was significantly higher than the ratio in the sample derived from BPH patients.

On the other hand, as is clear from Figs. 12(1) and 12(2), no significant difference was observed between Pca patients and BPH patients in the amount of total PSA (Fig. 12(1)) and the amount of $\alpha$(2,3) free PSA (Fig. 12(2)).

In addition, from the foregoing, it was found that the determination method of the present invention, in which the ratio of the amount of $\alpha$(2,3) free PSA to the amount of free PSA=40% is taken as a cutoff value and the determination of Pca is carried out on the basis of this value, is capable of making a determination of Pca with higher accuracy than a known method in which the determination of Pca is carried out on the basis of the total PSA value or the amount of $\alpha$(2,3) free PSA.

Example 8

(1) Preparation of electrophoresis sample A

Sera collected from 103 patients with prostate carcinoma (Pca) and 50 non-cancer subjects (those who are determined not to have prostate carcinoma, containing BPH patients) were used as samples. Histopathological diagnosis of each patient was confirmed by prostate biopsy. The patient's background (age and total PSA value) is shown in Table 3.

[Table 3]

| Cases | Demographic data of the patients | |
|---|---|---|
| non-Pca | N | 50 |
| Pca | Age [median] | 53-85 [70] |
| | tPSA ng/ml [median] | 1.9-20.4 [5.8] |
| | N | 103 |
| | Age [median] | 48-85 [70] |
| | tPSA ng/ml [median] | 1.4-21.7 [5.8] |

To a 0.5 mL tube, 2 $\mu$L of the sample, 1 $\mu$L of the 1 $\mu$M fluorescently labeled anti-free PSA antibody prepared in the section (1) 2) of Example 1, and 7 $\mu$L of electrophoresis buffer solution 1 [containing 5% (w/v) polyethylene glycol (PEG20000), 3% (w/v) glycerol, 150 mM NaCl, 0.01% BSA, 75 mM Tris-HCl (pH 7.5), and 10 mM MES] were added and mixed to prepare 10 $\mu$L of a reaction solution.

As the electrophoresis sample A, 10 $\mu$L of the obtained reaction solution was used.

(2) Separation/measurement of PSA and determination of ratio thereof

The microchip capillary electrophoresis was carried out in the same manner as in Example 1, using the same electrophoresis test solution, measuring apparatus, and the like as those used in Example 1, except that the electrophoresis sample A prepared in the section (1) was used. Then, the ratio of the amount of $\alpha$(2,3) free PSA to the amount

of free PSA in each sample was determined in the same manner as in Example 1.

(3) Results

For the values obtained, a significant difference test was carried out between Pca patients and non-cancer subjects (Mann-Whitney U-TEST) was carried out. The results obtained are shown in Fig. 13.

As is clear from Fig. 13, the P value obtained by comparing the ratios ($\alpha$(2,3) PSA ratios (%)) of the amount of $\alpha$(2,3) free PSA to the amount of free PSA between Pca patients and non-cancer subjects was "P<0.0001". Therefore, it was found that the ratio of the amount of $\alpha$(2,3) free PSA to the amount of free PSA in the sample derived from Pca patients was significantly higher than the ratio in the sample derived from non-cancer subjects.

In addition, on the basis of the obtained ratio, a Relative Operating Characteristic curve (ROC curve) analysis was further carried out. The results are also shown in Fig. 14 (Fig. 14(1)).

In addition, a Relative Operating Characteristic curve (ROC curve) analysis was carried out on the basis of the total PSA value already measured. The results are also shown in Fig. 14 (Fig. 14(2)).

As a result of the ROC curve analysis in Fig. 14, in the case of determining Pca by the ratio of the amount of $\alpha$(2,3) free PSA to the amount of free PSA of the present invention, AUC (Area Under the Curve)=0.851. On the other hand, in the case of determining Pca by the conventional total PSA value, AUC=0.658.

That is, it can be seen that the determination method of the present invention is superior to the conventional determination method in terms of the sensitivity and specificity of the measurement system, and the determination method of the present invention is a determination method with higher diagnostic accuracy than the determination method on the basis of the conventional amount of total PSA.

In addition, as a result of the ROC curve analysis in Fig. 14, in the case where the ratio of the amount of $\alpha$(2,3) free PSA to the amount of free PSA=40% was taken as the cutoff value in the determination of prostate carcinoma, the sensitivity was 81.6% and the specificity was 76.0%. On the other hand, in the case where the sensitivity of 81.6% was achieved in the determination method using the amount of total PSA, which is a known technique, the specificity was 46.0%. From the foregoing, it was confirmed that the prostate carcinoma determination method of the present invention, in which the ratio of the amount of $\alpha$(2,3) free PSA to the amount of free PSA=40% is taken as the cutoff value in the determination of prostate carcinoma, is an excellent determination method with higher sensitivity and specificity for prostate carcinoma determination than a conventional determination method.

(4) Test of cutoff value

On the basis of the result of the ratio of the amount of $\alpha$(2,3) free PSA to the amount of free PSA in the sample derived from Pca patients obtained in the section (3), a Relative Operating Characteristic curve analysis was carried out (ROC curve, Fig. 15). Next, as usual, a straight line with an angle of 45 degrees in contact with the ROC curve was drawn, and the value of the intersection point with the straight line (indicated by the arrow in Fig. 15), that is, the value at which the "sensitivity-(1-specificity)" was the maximum was determined. As a result, the value (ratio of the amount of $\alpha$(2,3) free PSA to the amount of free PSA) was "42.7%".

Therefore, in the verification of this Example, the cutoff value in the case of making Pca determination on the basis of the ratio of the amount of $\alpha$(2,3) free PSA to the amount of free PSA was "42.7%".

Example 9 and Comparative Example 5: Determination of malignancy of prostate carcinoma

(1) Sample

Sera collected from 36 prostate carcinoma (Pca) patients whose malignancy was determined by postoperative Gleason score (GS) determination and from 40 non-cancer subjects (those who were determined not to have prostate carcinoma, containing BPH patients) were used as samples. Histopathological diagnosis of each patient was confirmed by prostate biopsy. The patient's background (age, total PSA value, Gleason score, and the like) is shown in Table 4.

[Table 4]

| Pathology | Gleason score (after RP) | Patients (N) | Age median [median] | tPSA ng/ml [median] |
|---|---|---|---|---|
| non-Pca (N=40) | | 40 | 53-85 [70] | 4.2-20.4 [6.1] |
| Pca (N=36) | | | | |
| | Gleason 3+4 | 13 | 56-75 [68] | 4.5-11.9 [5.4] |
| | Gleason 4+3 | 9 | 51-74 [69] | 4.9-18.4 [8.6] |

(continued)

| Pathology | Gleason score (after RP) | Patients (N) | Age median [median] | tPSA ng/ml [median] |
|---|---|---|---|---|
| | Gleason 4+4 | 5 | 66-74 [72] | 5.5-10.0 [6.3] |
| | Gleason 4+5 | 7 | 60-73 [66] | 5.1-14.1 [7.2] |
| | Gleason 5+4, 5+5 | 2 | 57-71 [64] | 6.3-7.6 [7.0] |

(2) Determination of ratio of amount of α(2,3) free PSA to amount of free PSA (Example 9)

Electrophoresis sample A was prepared in the same manner as in the section (1) of Example 4 using the sample of the section (1).

Next, the microchip capillary electrophoresis was carried out in the same manner as in Example 1, using the same electrophoresis test solution, measuring apparatus, and the like as those used in Example 1, except that the electrophoresis sample A prepared was used. Then, the ratio of the amount of α(2,3) free PSA to the amount of free PSA in each sample was determined in the same manner as in Example 1.

The results are shown in Fig. 16(1).

In addition, on the basis of the ratio of the amount of α(2,3) free PSA to the amount of free PSA obtained using a serum sample derived from patients having a radical prostatectomy Gleason score of 4+3 (prostatectomy GS=4+3) or higher malignancy, a Relative Operating Characteristic curve (ROC curve) analysis was carried out. The results are shown in Fig. 17.

(2) Measurement of total PSA value (Comparative Example 5)

Using the same sample as used in Example 9, the total PSA value in each sample was determined according to the protocol attached to the kit, using Lumipulse Presto PSA (Fujirebio, Inc.) which is an *in vitro* diagnostics.

The results are shown in Fig. 16(2).

(3) Results

Fig. 16(1) is a diagram showing the ratio of the amount of α(2,3) free PSA to the amount of free PSA in samples derived from non-cancer subjects and Pca patients. For Pca patients, the ratio for each radical prostatectomy Gleason score (prostatectomy GS) was shown.

Fig. 16(2) is a diagram showing the amount of total PSA in samples derived from non-cancer subjects and Pca patients. For Pca patients, the ratio for each radical prostatectomy GS was shown.

As is clear from Fig. 16(1), in the case where a value at which the ratio of the amount of α(2,3) free PSA to the amount of free PSA is 40% was taken as the cutoff value, there is a strong tendency that the ratio in non-cancer subjects is lower than the cutoff value, and the ratio in Pca patients is higher than the cutoff value. From this, it was shown that satisfactory distinguishing between Pca patients and non-cancer cases is possible by using a cutoff value of 40%.

Next, as is clear from the analysis results by the ROC curve (Fig. 17), in the case where Pca was determined by the ratio of the amount of α(2,3) free PSA to the amount of free PSA of the present invention, AUC=0.921. In addition, the sensitivity was 91.3%, and the specificity was 90.6%.

In addition, as usual, a straight line with an angle of 45 degrees in contact with the ROC curve of Fig. 17 was drawn, and the value of the intersection point with the straight line (indicated by the arrow in Fig. 17), that is, the value at which the "sensitivity-(1-specificity)" was the maximum was determined. As a result, the value (ratio of the amount of α(2,3) free PSA to the amount of free PSA) was "47.2%". Accordingly, in the verification of this Example, the cutoff value in the case of determining the malignancy of Pca on the basis of the ratio of the amount of α(2,3) free PSA to the amount of free PSA was "47.2%" (Fig. 17).

In addition, in the case where a value at which the ratio of the amount of α(2,3) free PSA to the amount of free PSA is 47.2% was taken as the cutoff value, as is clear from the results in Fig. 16(1), there is a strong tendency that the ratio in patients having a Gleason score of 4+3 (GS=4+3) or more is higher than the cutoff value. That is, it showed good correlation with definite diagnosis by a radical prostatectomy Gleason score.

In addition, the ratio in the case of GS=3+4 tended to be higher than the cutoff value 40% but lower than 47.2%, so it could be identified as a case with Pca but low malignancy.

From the foregoing, it was found that, in the case where the "ratio of the amount of α(2,3) free PSA to the amount of free PSA"="47.2%" is taken as the cutoff value for malignancy determination, and the malignancy of Pca is determined using this cutoff value "47.2%, it is possible to select cases with low malignancy and in addition, there is a high possibility that excessive medical care could be avoided.

On the other hand, as is clear from Fig. 16(2), no relationship was found between total PSA value and Gleason score. From this, it can be seen that even in the case where the total PSA value is used as an index, neither Pca determination nor Pca malignancy determination can be carried out.

Example 10 and Comparative Example 6: On effects of glycan diversity in non-Japanese samples

A total of 19 types of glycans of PSA have been identified, and it has been clarified that the glycans of PSA are highly diverse (Non-Patent Literature 4). In addition, it has been reported that glycans of glycoproteins have diversity by race ("Difference of placental alkaline phosphatase molecule on the basis of genetic phenotype", Matsuo Sato, Journal of Tokyo Women's Medical University, 60(8), pp. 609 to 619, 1990).

Therefore, it was predicted that the glycans of PSA also have diversity depending on the country, region, and race behind the patient. There was concern that this glycan diversity would affect PSA measurement and Pca determination.

Therefore, using a sample other than Japanese sample, a known determination method (WO2014/057983: Patent Literature 3) using the amount of α(2,3) free PSA as an index and a determination method using the ratio of the amount of α(2,3) free PSA to the amount of free PSA of the present invention as an index were carried out and the results thereof were compared and examined.

(1) Sample

Sera collected from 29 Pca patients who were non-Japanese and had a total PSA value of 20.0 ng/mL or less and 10 non-cancer subjects (those who are determined not to have prostate carcinoma, containing BPH patients) who were non-Japanese, offered from the Dr. Pinthus Laboratory of St. Joseph's Healthcare Hamilton, Canada, were used as samples.

The PSA value (total PSA value) of the sample and the like are shown in Table 5 below.

[Table 5]

| | non-Pca | | Pca | |
|---|---|---|---|---|
| Patients, n | 10 | | 29 | |
| PSA ng/mL (median) | 1.9-6.5 | (4.3) | 1.4-18.0 | (5.6) |

(2) Separation/measurement of PSA and determination of ratio thereof (Example 10)

Electrophoresis sample A was prepared in the same manner as in the section (1) of Example 4 using the sample of the section (1).

Next, the microchip capillary electrophoresis was carried out in the same manner as in Example 1, using the same electrophoresis test solution, measuring apparatus, and the like as those used in Example 1, except that the electrophoresis sample A prepared was used. Then, the ratio of the amount of α(2,3) free PSA to the amount of free PSA in each sample was determined in the same manner as in Example 1.

(3) Measurement of amount of α(2,3) free PSA (Comparative Example 6)

The amount of α(2,3) free PSA (fluorescence intensity (MFI)) was measured by using the sample of the section (1) and carrying out the same method as described in Example 2 of Patent Literature 3.

(4) Results

The results are shown in Figs. 18(1) and 18(2). In Figs. 18(1) and 18(2), Fig. 18(1) shows the results (Example 10) of comparing the ratios (α(2,3) PSA ratios) of the amount of α(2,3) free PSA to the amount of free PSA between Pca patients (Pca) and non-cancer subjects (non-Pca). Fig. 18(2) shows the results (Comparative Example 6) of comparing the amounts (fluorescence intensity (MFI)) of α(2,3) free PSA between Pca patients (Pca) and non-cancer subjects (non-Pca).

In Figs. 18(1) and 18(2), the horizontal line in the box of the boxplot shows the median value of each result as usual.

In the case where a significant difference test (Mann-Whitney U-TEST) was carried out between Pca patients and non-cancer subjects, the P value obtained by comparing the ratios of the amount of α(2,3) free PSA to the amount

of free PSA between Pca patients and non-cancer subjects was "0.0062" (Example 10, Fig. 18(1)). On the other hand, the P value obtained by comparing the amounts of α(2,3) free PSA between Pca patients and non-cancer subjects was "0.0818" (Comparative Example 6, Fig. 18(2)).

As is clear from Fig. 18(1), the median value of the ratio of the amount of α(2,3) free PSA to the amount of free PSA in non-cancer subjects was less than 40% (around 38%). On the other hand, the median value of the ratio in Pca patients was much higher than 40% (around 47%).

From the foregoing, it was found that the determination method of the present invention, in which the ratio of the amount of α(2,3) free PSA to the amount of free PSA=40% is used as a cutoff value and the determination of Pca is carried out on the basis of this value, is capable of making a determination of Pca with higher accuracy than a known method in which the determination is carried out on the basis of the amount of α(2,3) free PSA. In addition, it was also found that the determination method of the present invention is capable of determining Pca with high accuracy even in the case of using samples obtained from Non-Japanese expected to cause concerns such as glycan diversity.

On the other hand, as is clear from Fig. 18(2), in the case where the determination was carried out using the cutoff value "1130" of the amount of α(2,3) free PSA, it can be seen that the amount of α(2,3) free PSA in non-cancer subjects and the amount of α(2,3) free PSA in Pca patients are also far higher than the cutoff value, so that accurate determination of Pca cannot be carried out.

Example 11: Determination by surface plasmon resonance method

Using a surface plasmon resonance (SPR) technique as a measurement principle different from microchip electrophoresis, the measurement of S2,3PSA content ratio using BIACORE™ (GE Bio, Inc.), which is a representative measurement apparatus, was carried out by the following method.

(1) Measuring instrument and the like

Measuring instrument: Biacore X (manufactured by GE Healthcare UK Ltd.)
Chip: Sensor Chip CM5 (manufactured by GE Healthcare UK Ltd.)
Running buffer: HBS-EP buffer (10 mM HEPES, 0.15 M NaCl, 3 mM EDTA, 0.005% Surfactant P 20, pH 7.4, manufactured by GE Healthcare UK Ltd.)

(2) Sample solution

The r α(2,3) free PSA and r α(2,6) free PSA purified and prepared in Example 2 were each adjusted so as to have a 1000 ng/mL PSA protein concentration. Subsequently, r α(2,3) free PSA solution was diluted with the resulting r α(2,6) free PSA solution to obtain a sample solution containing r α(2,3) free PSA in an amount of 25%, 45%, or 55%. This content rate of r α(2,3) free PSA is defined as "theoretical value".

It should be noted that r α(2,3) free PSA and r α(2,6) free PSA have a peptide sequence of FLAG tag.

(3) Immobilization of LCA on sensor chip

An anti-FLAG tag antibody (ANTI-FLAG M2 Monoclonal Antibody, manufactured by Sigma-Aldrich Corporation) was immobilized on a sensor chip of Sensor Chip CM5 (CM sensor chip, manufactured by GE Healthcare UK Ltd.) using an amine coupling kit (manufactured by GE Healthcare UK Ltd.).

(4) Implementation of surface plasmon resonance method

The following measurement was carried out using Biacore X (manufactured by GE Healthcare UK Ltd.).

Under conditions of a temperature of 20°C, a flow rate of 10 μL/min, and a binding time of 10 minutes, 100 μL of the sample solution prepared in the section (2) was slowly delivered and flowed to the sensor chip on which an anti-tag (FLAG) antibody was immobilized, so that α(2,3) free PSA and α(2,6) free PSA contained in the sample at certain ratios, respectively were reacted with the anti-FLAG tag antibody. The signal (shift of resonance angle) was measured over time immediately after the solution was delivered.

Subsequently, HBS-EP buffer containing 15 mg/mL of MAA was slowly delivered under conditions of a temperature of 20°C, a flow rate of 30 μL/min, and a binding time of 2 minutes and flowed to the sensor chip on which α(2,3) free PSA and α(2,6) free PSA were immobilized at certain ratios through an anti-FLAG tag FLAG antibody, and the interaction between the target glycan of α(2,3) free PSA on the sensor chip and the MAA was assayed. The signal (shift of resonance angle) was measured over time immediately after the solution was delivered.

Next, only the HBS-EP buffer was delivered for 180 seconds (dissociation time).

The obtained measurement results were analyzed using BIAevaluation (Version4.1) which is dedicated analysis software for Biacore to obtain a sensorgram.

(5) Results

The obtained sensorgram is shown in Fig. 19.
In Fig. 19, the horizontal axis represents time (s (sec)) and the vertical axis represents signal intensity (RU, Resonance Unit).
In addition, in Fig. 19, (1) shows the results obtained using a sample containing α(2,3) free PSA at 55%, (2) shows the results obtained using a sample containing α(2,3) free PSA at 45%, and (3) shows the results obtained using a sample containing α(2,3) free PSA at 25%, respectively.
As can be seen from Fig. 19, the response was significantly higher in the sensorgrams obtained using samples containing α(2,3) free PSA of "45%" and "55%" exceeding the cutoff value (40%) according to the determination method of the present invention, relative to the sensorgram obtained using the sample containing α(2,3) free PSA in the normal range (25%) corresponding to non-cancer in the present invention. From the foregoing, it was found that the determination method using the cutoff value of the present invention (cutoff value of Pca determination: 40%, cutoff value of Pca malignancy determination: 47%) can also be carried out by the surface plasmon resonance method.

Industrial Applicability

The Pca determination method of the present invention enables non-invasive and convenient determination (diagnosis or inspection) of Pca and malignancy thereof with high accuracy. In particular, it is possible to determine whether or not Pca is developed or the probability of developing Pca is high with high diagnostic accuracy in a patient whose total PSA value is in a gray zone, who has been conventionally difficult to be determined.
In addition, since the Pca determination method of the present invention can determine the malignancy of Pca, the determination result obtained by the determination method of the present invention is an important guideline for setting a therapeutic strategy of Pca thereafter.

## Claims

1. An *in vitro* prostate carcinoma determination method, comprising:

    determining a ratio of an amount of free prostate specific antigen (hereinafter, the prostate specific antigen is referred to as "PSA") having a glycan of which a terminal sialic acid residue is α(2,3)-linked to a second galactose residue from a terminal of the glycan (hereinafter, referred to as "α(2,3) free PSA") to an amount of free PSA in a biological sample; and
    determining that prostate carcinoma is developed or the probability of developing prostate carcinoma is high in the case where the ratio is 40% or higher.

2. The determination method according to claim 1, wherein the method comprises,
measuring the amount of free PSA and the amount of α(2,3) free PSA in the biological sample,
determining the ratio of the amount of α(2,3) free PSA to the amount of free PSA obtained, and
determining that prostate carcinoma is developed or the probability of developing prostate carcinoma is high in the case where the ratio is 40% or higher.

3. The method according to claim 1 or 2, wherein a total PSA value in the biological sample is a value of higher than 0 to 50 ng/mL.

4. The method according to claim 1 or 2, wherein the total PSA value in the biological sample is 4 to 10 ng/mL.

5. The method according to claim 2, wherein the method of measuring the amount of α(2,3) free PSA is a method of measuring the amount of α(2,3) free PSA, utilizing an interaction between a glycan of α(2,3) free PSA in which the terminal sialic acid residue of the glycan is α(2,3)-linked to the second galactose residue from the terminal of the glycan and a substance (hereinafter, referred to as "affinity substance") having an affinity for the glycan in which the terminal sialic acid residue is α(2,3)-linked to the second galactose residue from the terminal of the glycan.

6. The method according to claim 5, wherein the method of measuring the amount of α(2,3) free PSA utilizing the

interaction between the glycan of α(2,3) free PSA in which the terminal sialic acid residue of the glycan is α(2,3)-linked to the second galactose residue from the terminal of the glycan and the affinity substance is a method of forming a complex of α(2,3) free PSA with the affinity substance by the interaction between the glycan of α(2,3) free PSA in which the terminal sialic acid residue of the glycan is α(2,3)-linked to the second galactose residue from the terminal of the glycan and the affinity substance; measuring the amount of the complex; and on the basis of the results, determining the amount of α(2,3) free PSA.

**7.** The method according to claim 5, wherein the method of measuring the amount of α(2,3) free PSA utilizing the interaction between the glycan of α(2,3) free PSA in which the terminal sialic acid residue of the glycan is α(2,3)-linked to the second galactose residue from the terminal of the glycan and the affinity substance is a method of forming a complex of α(2,3) free PSA with the affinity substance by the interaction between the glycan of α(2,3) free PSA in which the terminal sialic acid residue of the glycan is α(2,3)-linked to the second galactose residue from the terminal of the glycan and the affinity substance; measuring the amount of the complex without removing components other than the complex from the system; and on the basis of the results, determining the amount of α(2,3) free PSA.

**8.** The method according to claim 1, 2, or 5, wherein the method of measuring the amount of α(2,3) free PSA is a measuring method in which an efficiency of capturing α(2,3) free PSA is 80% or more.

**9.** The method according to claim 5, wherein the affinity substance is a lectin.

**10.** The method according to claim 9, wherein the lectin is a lectin having an affinity for the glycan of which a terminal sialic acid residue is α(2,3)-linked to a second galactose residue from a terminal of the glycan.

**11.** The method according to claim 9, wherein the lectin is a *Maackia amurensis* lectin (MAA).

**12.** The method according to claim 1 or 2, further comprising:

determining that prostate carcinoma is developed or the probability of developing prostate carcinoma is high in the case where the ratio of the amount of α(2,3) free PSA to the amount of free PSA is 40% or higher; and
determining that the probability of high malignancy of prostate carcinoma is high in the case where the ratio is 47% or higher.

**13.** The method according to claim 2, wherein the method of measuring the amount of α(2,3) free PSA is a capillary electrophoresis method, a biacore method, a mass spectrometry method, or a lectin microarray method.

## Patentansprüche

**1.** *In-vitro*-Prostatakarzinom-Bestimmungsverfahren, umfassend:

Bestimmen eines Verhältnisses von einer Menge an freiem Prostataspezifischem Antigen (nachfolgend wird das Prostata-spezifische Antigen als "PSA" bezeichnet) mit einem Glykan, von welchem ein terminaler Sialinsäurerest $\alpha$(2,3)-verknüpft ist mit einem zweiten Galaktoserest von einem Ende des Glykans (nachfolgend als "$\alpha$(2,3)-freies PSA" bezeichnet), zu einer Menge an freiem PSA in einer biologischen Probe; und
Bestimmen, dass Prostatakarzinom entwickelt ist oder die Wahrscheinlichkeit der Entwicklung eines Prostatakarzinoms hoch ist in dem Fall, wo das Verhältnis 40 % oder höher ist.

**2.** Bestimmungsverfahren nach Anspruch 1, wobei das Verfahren umfasst:

Messen der Menge an freiem PSA und der Menge an $\alpha$(2,3)-freiem PSA in der biologischen Probe,
Bestimmen des Verhältnisses von der Menge an $\alpha$(2,3)-freiem PSA zur Menge des erhaltenen freien PSA, und
Bestimmen, dass Prostatakarzinom entwickelt ist oder die Wahrscheinlichkeit der Entwicklung eines Prostatakarzinoms hoch ist in dem Fall, wo das Verhältnis 40 % oder höher ist.

**3.** Verfahren nach Anspruch 1 oder 2, wobei ein Gesamt-PSA-Wert in der biologischen Probe ein Wert von höher als 0 bis 50 ng/mL ist.

**4.** Verfahren nach Anspruch 1 oder 2, wobei der Gesamt-PSA-Wert in der biologischen Probe 4 bis 10 ng/mL beträgt.

**5.** Verfahren nach Anspruch 2, wobei das Verfahren zur Messung der Menge an $\alpha$(2,3)-freiem PSA ein Verfahren zur Messung der Menge an $\alpha$(2,3)-freiem PSA ist, welches eine Wechselwirkung verwendet zwischen einem Glykan von $\alpha$(2,3)-freiem PSA, in welchem der terminale Sialinsäurerest des Glykans $\alpha$(2,3)-verknüpft ist mit dem zweiten Galaktoserest von dem Ende des Glykans, und einer Substanz (nachfolgend als "Affinitätssubstanz" bezeichnet) mit einer Affinität für das Glykan, in welchem der terminale Sialinsäurerest $\alpha$(2,3)-verknüpft ist mit dem zweiten Galaktoserest von dem Ende des Glykans.

**6.** Verfahren nach Anspruch 5, wobei das Verfahren zur Messung der Menge an $\alpha$(2,3)-freiem PSA, welches die Wechselwirkung verwendet zwischen dem Glykan von $\alpha$(2,3)-freiem PSA, in welchem der terminale Sialinsäurerest des Glykans $\alpha$(2,3)-verknüpft ist mit dem zweiten Galaktoserest von dem Ende des Glykans, und der Affinitätssubstanz ein Verfahren ist zur Bildung eines Komplexes von $\alpha$(2,3)-freiem PSA mit der Affinitätssubstanz durch die Wechselwirkung zwischen dem Glykan von $\alpha$(2,3)-freiem PSA, in welchem der terminale Sialinsäurerest des Glykans $\alpha$(2,3)-verknüpft ist mit dem zweiten Galaktoserest von dem Ende des Glykans, und der Affinitätssubstanz; Messen der Menge des Komplexes; und, auf der Grundlage der Ergebnisse, Bestimmen der Menge an $\alpha$(2,3)-freiem PSA.

**7.** Verfahren nach Anspruch 5, wobei das Verfahren zur Messung der Menge an $\alpha$(2,3)-freiem PSA, welches die Wechselwirkung verwendet zwischen dem Glykan von $\alpha$(2,3)-freiem PSA, in welchem der terminale Sialinsäurerest des Glykans $\alpha$(2,3)-verknüpft ist mit dem zweiten Galaktoserest von dem Ende des Glykans, und der Affinitätssubstanz ein Verfahren ist zur Bildung eines Komplexes von $\alpha$(2,3)-freiem PSA mit der Affinitätssubstanz durch die Wechselwirkung zwischen dem Glykan von $\alpha$(2,3)-freiem PSA, in welchem der terminale Sialinsäurerest des Glykans $\alpha$(2,3)-verknüpft ist mit dem zweiten Galaktoserest von dem Ende des Glykans, und der Affinitätssubstanz; Messen der Menge des Komplexes ohne andere Komponenten als den Komplex aus dem System zu entfernen; und, auf der Grundlage der Ergebnisse, Bestimmen der Menge an $\alpha$(2,3)-freiem PSA.

**8.** Verfahren nach Anspruch 1, 2 oder 5, wobei das Verfahren zur Messung der Menge an $\alpha$(2,3)-freiem PSA ein Messverfahren ist, in welchem eine Effizienz der Erfassung von $\alpha$(2,3)-freiem PSA 80% oder mehr beträgt.

**9.** Verfahren nach Anspruch 5, wobei die Affinitätssubstanz ein Lektin ist.

**10.** Verfahren nach Anspruch 9, wobei das Lektin ein Lektin ist mit einer Affinität für das Glykan, von welchem ein terminaler Sialinsäurerest $\alpha$(2,3)-verknüpft ist mit einem zweiten Galaktoserest von einem Ende des Glykans.

**11.** Verfahren nach Anspruch 9, wobei das Lektin ein *Maackia amurensis*-Lektin (MAA) ist.

**12.** Verfahren nach Anspruch 1 oder 2, ferner umfassend:

Bestimmen, dass Prostatakarzinom entwickelt ist oder die Wahrscheinlichkeit der Entwicklung eines Prostatakarzinoms hoch ist in dem Fall, wo das Verhältnis der Menge an $\alpha$(2,3)-freiem PSA zu der Menge an freiem PSA 40 % oder höher ist; und
Bestimmen, dass die Wahrscheinlichkeit einer hohen Malignität eines Prostatakarzinoms hoch ist in dem Fall, wo das Verhältnis 47 % oder höher ist.

**13.** Verfahren nach Anspruch 2, wobei das Verfahren zur Messung der Menge an $\alpha$(2,3)-freiem PSA ein Kapillarelektrophorese-Verfahren, ein Biacore-Verfahren, ein Massenspektrometrie-Verfahren oder ein Lektin-Mikroarray-Verfahren ist.

**Revendications**

**1.** Procédé de détermination d'un carcinome de la prostate *in vitro,* comprenant :

la détermination d'un rapport entre une quantité d'antigène spécifique de la prostate libre (ci-après, l'antigène spécifique de la prostate est désigné « PSA ») comportant un glycane dont un résidu d'acide sialique terminal est lié en $\alpha$(2,3) à un second résidu galactose d'une terminaison du glycane (ci-après, désigné « PSA libre $\alpha$(2,3) ») et une quantité de PSA libre dans un échantillon biologique ; et
la détermination du fait qu'un carcinome de la prostate s'est développé ou que la probabilité de développement d'un carcinome de la prostate est élevée dans le cas où le rapport est de 40 % ou plus.

2. Procédé de détermination selon la revendication 1, où le procédé comprend,
la mesure de la quantité de PSA libre et de la quantité de PSA libre α(2,3) dans l'échantillon biologique,
la détermination du rapport entre la quantité de PSA libre α(2,3) et la quantité de PSA libre obtenue, et
la détermination du fait qu'un carcinome de la prostate s'est développé ou que la probabilité de développement d'un carcinome de la prostate est élevée dans le cas où le rapport est de 40 % ou plus.

3. Procédé selon la revendication 1 ou 2, dans lequel une valeur du PSA total dans l'échantillon biologique est une valeur supérieure à 0 à 50 ng/mL.

4. Procédé selon la revendication 1 ou 2, dans lequel la valeur du PSA total dans l'échantillon biologique est de 4 à 10 ng/mL.

5. Procédé selon la revendication 2, où le procédé de mesure de la quantité de PSA libre α(2,3) est un procédé de mesure de la quantité de PSA libre α(2,3), utilisant une interaction entre un glycane du PSA libre α(2,3) dans lequel le résidu d'acide sialique terminal du glycane est lié en α(2,3) au second résidu galactose de la terminaison du glycane et une substance (ci-après, désignée « substance d'affinité ») possédant une affinité pour le glycane dans lequel le résidu d'acide sialique terminal est lié en α(2,3) au second résidu galactose de la terminaison du glycane.

6. Procédé selon la revendication 5, où le procédé de mesure de la quantité de PSA libre α(2,3) utilisant l'interaction entre le glycane du PSA libre α(2,3) dans lequel le résidu d'acide sialique terminal du glycane est lié en α(2,3) au second résidu galactose de la terminaison du glycane et la substance d'affinité est un procédé de formation d'un complexe entre le PSA libre α(2,3) et la substance d'affinité par l'interaction entre le glycane du PSA libre α(2,3) dans lequel le résidu d'acide sialique terminal du glycane est lié en α(2,3) au second résidu galactose de la terminaison du glycane et la substance d'affinité ; de mesure de la quantité du complexe ; et, en se basant sur les résultats, de détermination de la quantité de PSA libre α(2,3).

7. Procédé selon la revendication 5, où le procédé de mesure de la quantité de PSA libre α(2,3) utilisant l'interaction entre le glycane du PSA libre α(2,3) dans lequel le résidu d'acide sialique terminal du glycane est lié en α(2,3) au second résidu galactose de la terminaison du glycane et la substance d'affinité est un procédé de formation d'un complexe entre le PSA libre α(2,3) et la substance d'affinité par l'interaction entre le glycane du PSA libre α(2,3) dans lequel le résidu d'acide sialique terminal du glycane est lié en α(2,3) au second résidu galactose de la terminaison du glycane et la substance d'affinité ; de mesure de la quantité du complexe sans retirer de composants autres que le complexe du système ; et, en se basant sur les résultats, de détermination de la quantité de PSA libre α(2,3).

8. Procédé selon la revendication 1, 2 ou 5, où le procédé de mesure de la quantité de PSA libre α(2,3) est un procédé de mesure dans lequel une efficacité de capture du PSA libre α(2,3) est de 80 % ou plus.

9. Procédé selon la revendication 5, dans lequel la substance d'affinité est une lectine.

10. Procédé selon la revendication 9, dans lequel la lectine est une lectine possédant une affinité pour le glycane dont un résidu d'acide sialique terminal est lié en α(2,3) à un second résidu galactose d'une terminaison du glycane.

11. Procédé selon la revendication 9, dans lequel la lectine est une lectine de *Maackia amurensis* (MAA).

12. Procédé selon la revendication 1 ou 2, comprenant en outre :

    la détermination du fait qu'un carcinome de la prostate s'est développé ou que la probabilité de développement d'un carcinome de la prostate est élevée dans le cas où le rapport entre la quantité de PSA libre α(2,3) et la quantité de PSA libre est de 40 % ou plus ; et
    la détermination du fait que la probabilité de forte malignité du carcinome de la prostate est élevée dans le cas où le rapport est de 47 % ou plus.

13. Procédé selon la revendication 2, où le procédé de mesure de la quantité de PSA libre α(2,3) est un procédé d'électrophorèse capillaire, un procédé biacore, un procédé de spectrométrie de masse, ou un procédé utilisant un microréseau de lectines.

FIG. 1

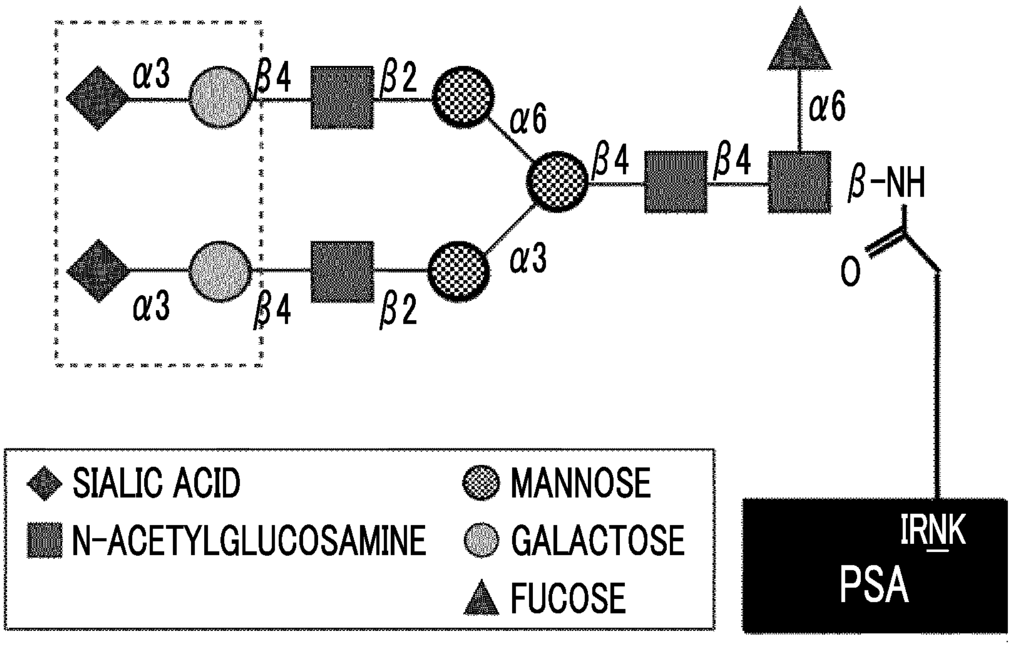


FIG. 2

PURIFIED TERMINALLY-AMINATED DNA

← Sulfo-SMPB LINKER REACTION

← REMOVAL OF UNREACTED LINKER BY GEL FILTRATION

← REACTION WITH ANTI-PSA ANTIBODY PSA10 Fab' FRAGMENT

← PURIFICATION OF DNA-LABELED ANTI-PSA ANTIBODY BY DEAE COLUMN

DNA-LABELED ANTI-PSA ANTIBODY

FIG. 3

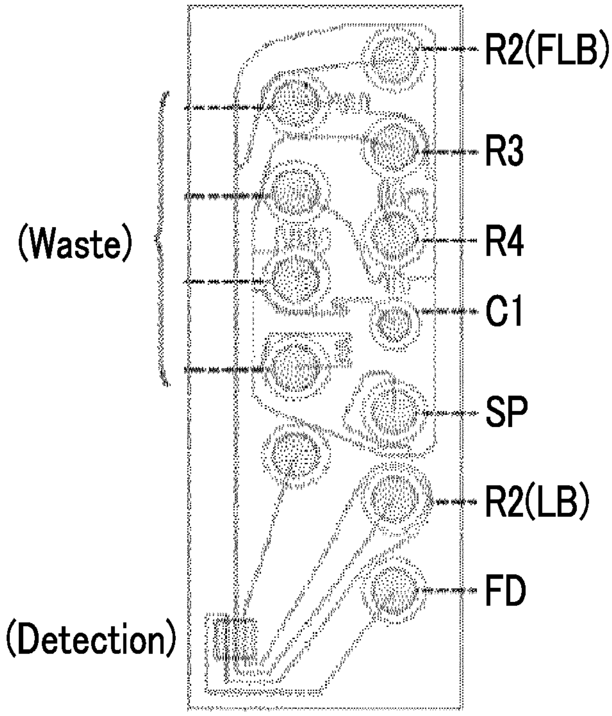
R2(FLB)
R3
R4
C1
SP
R2(LB)
FD
(Waste)
(Detection)

FIG. 4

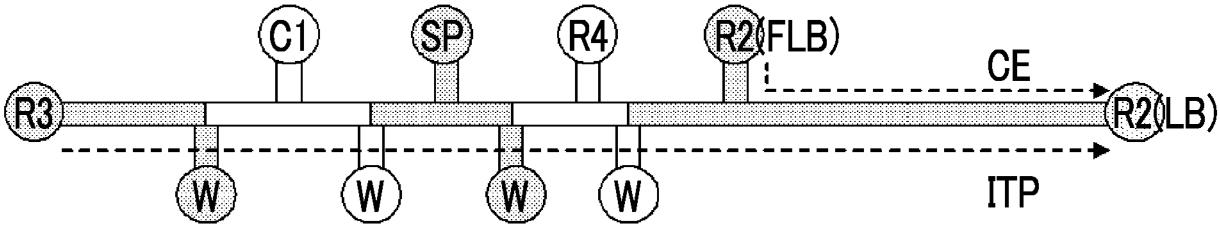
C1
SP
R4
R2(FLB)
CE
R3
R2(LB)
W
W
W
W
ITP

FIG. 5

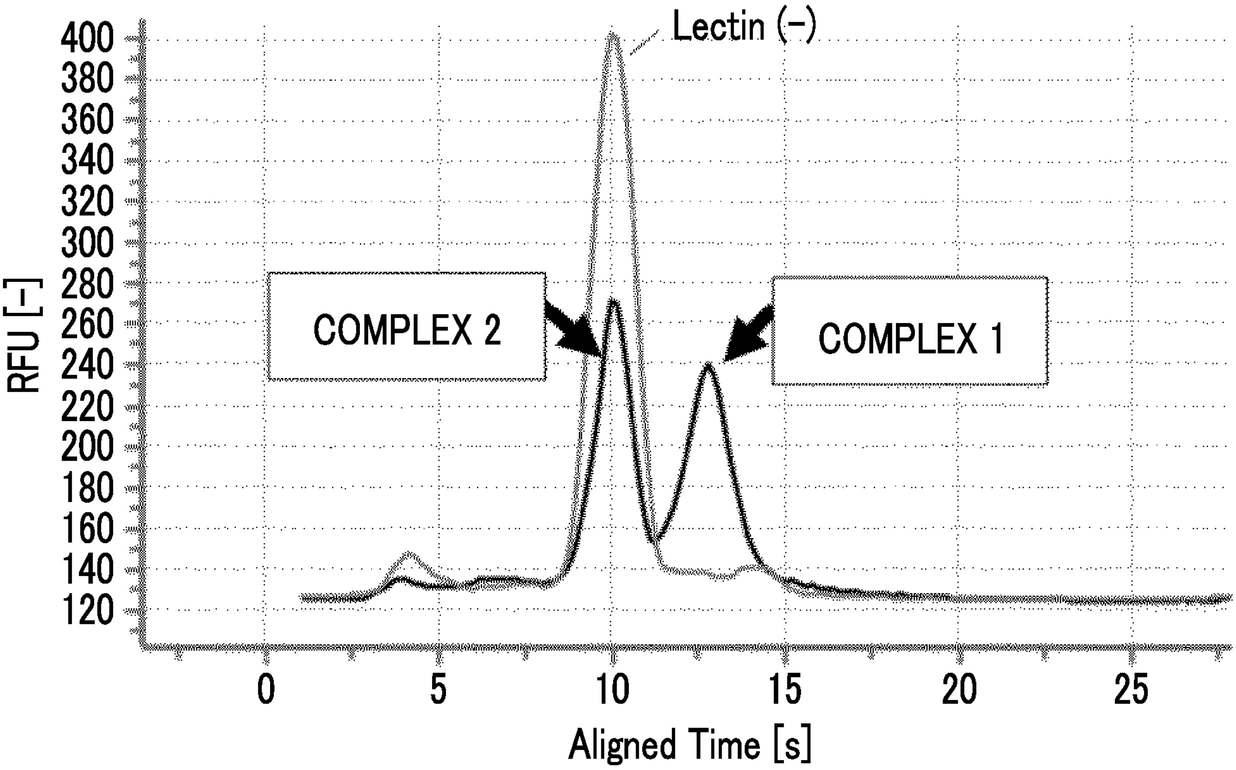
Lectin (-)
COMPLEX 2
COMPLEX 1
RFU [-]
400
380
360
340
320
300
280
260
240
220
200
180
160
140
120
0
5
10
15
20
25
Aligned Time [s]

FIG. 6(1)

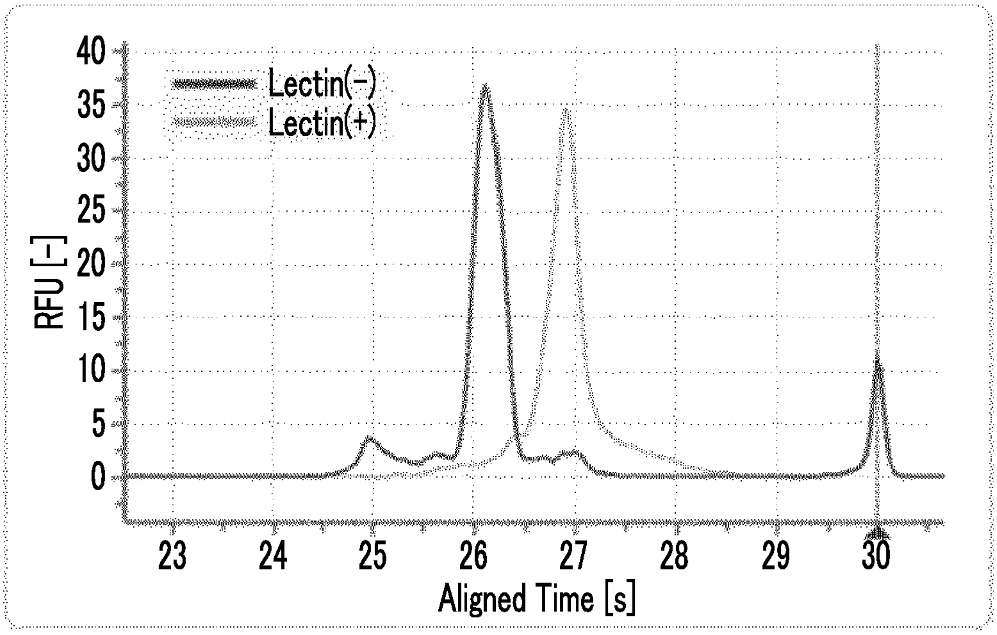
Lectin(-)
Lectin(+)
RFU [-]
40
35
30
25
20
15
10
5
0
23
24
25
26
27
28
29
30
Aligned Time [s]

FIG. 6(2)

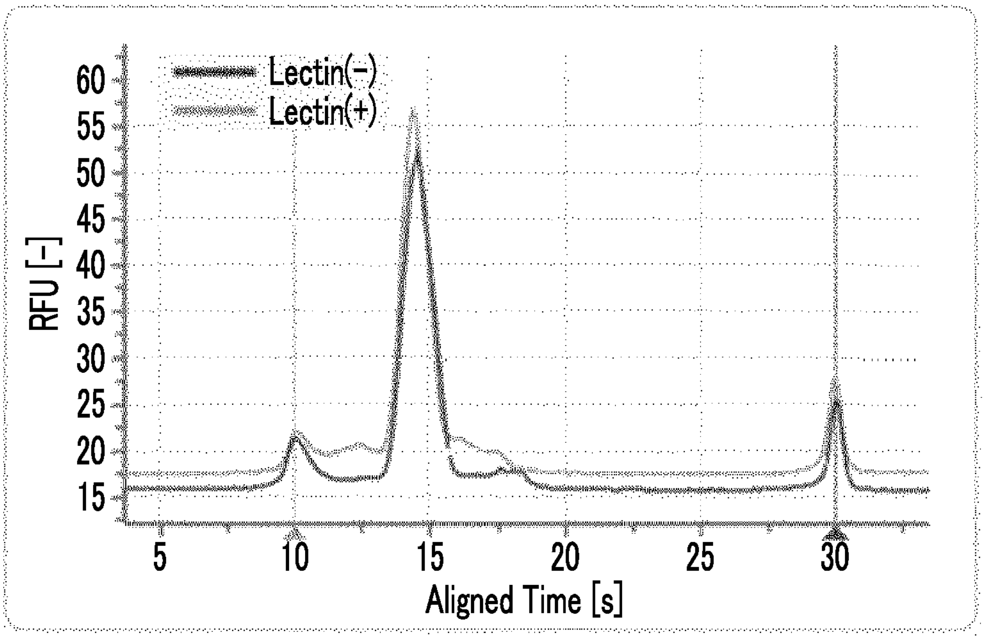
Lectin(-)
Lectin(+)
RFU [-]
60
55
50
45
40
35
30
25
20
15
5
10
15
20
25
30
Aligned Time [s]

FIG. 7(1)

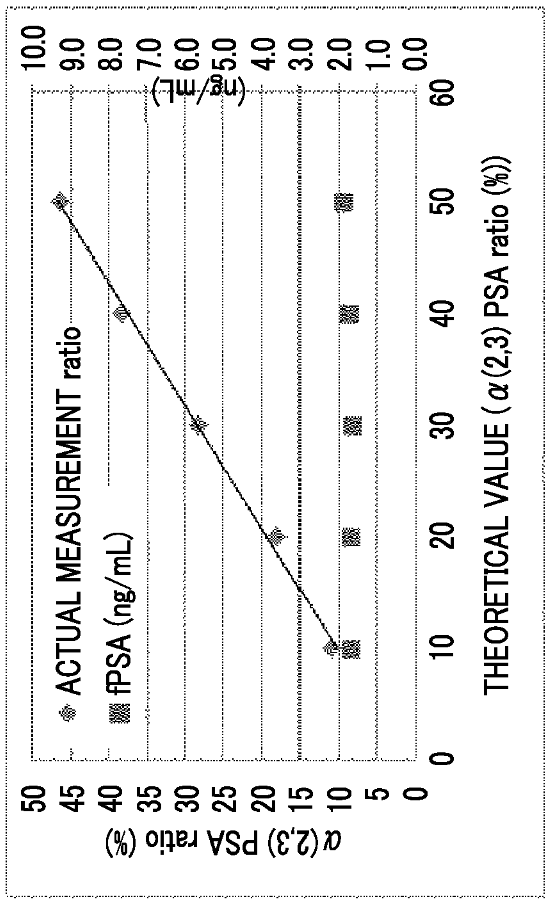
ACTUAL MEASUREMENT ratio
fPSA (ng/mL)
α(2,3) PSA ratio (%)
(ng/mL)
THEORETICAL VALUE (α(2,3) PSA ratio (%))
0
5
10
15
20
25
30
35
40
45
50
0.0
1.0
2.0
3.0
4.0
5.0
6.0
7.0
8.0
9.0
10.0
60

FIG. 7(2)

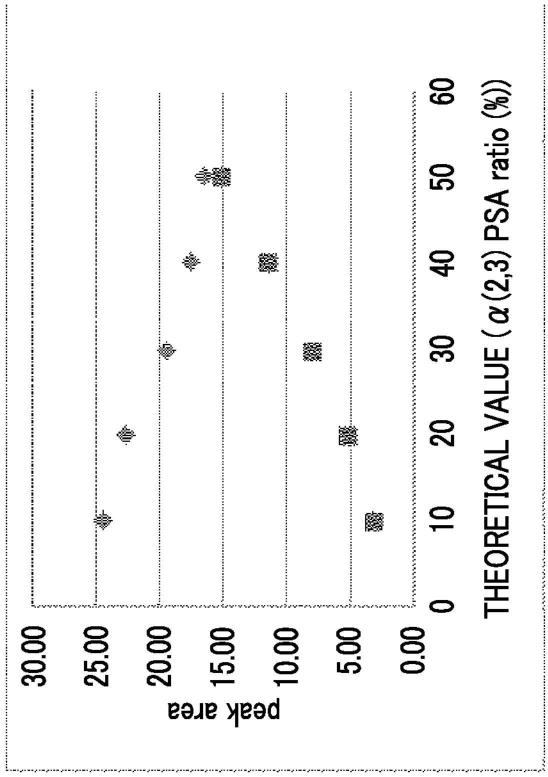
peak area
0.00
5.00
10.00
15.00
20.00
25.00
30.00
0
10
20
30
40
50
60
THEORETICAL VALUE (α(2,3) PSA ratio (%))

FIG. 8

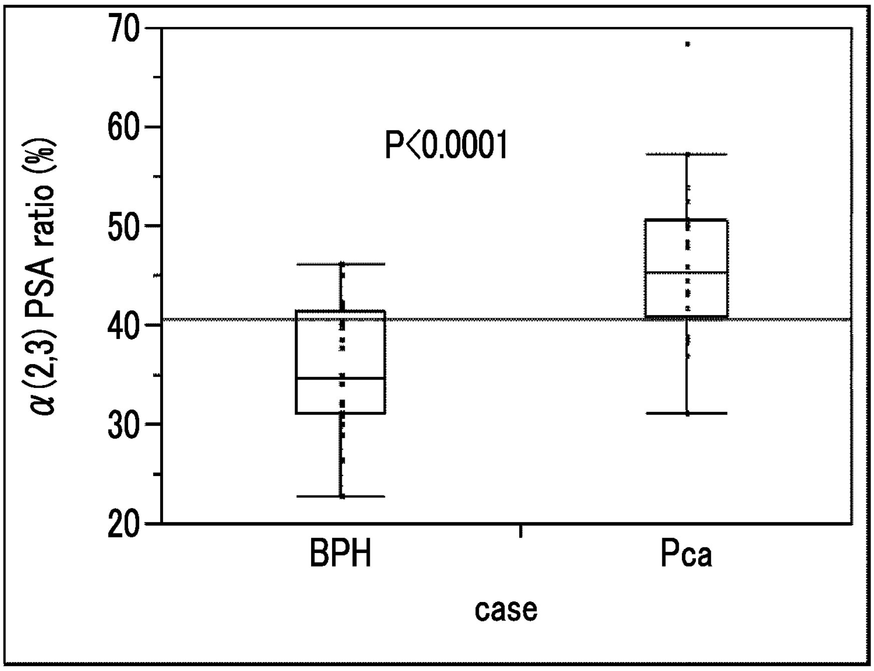


FIG. 9(1)

FIG. 9(2)

FIG. 9(3)

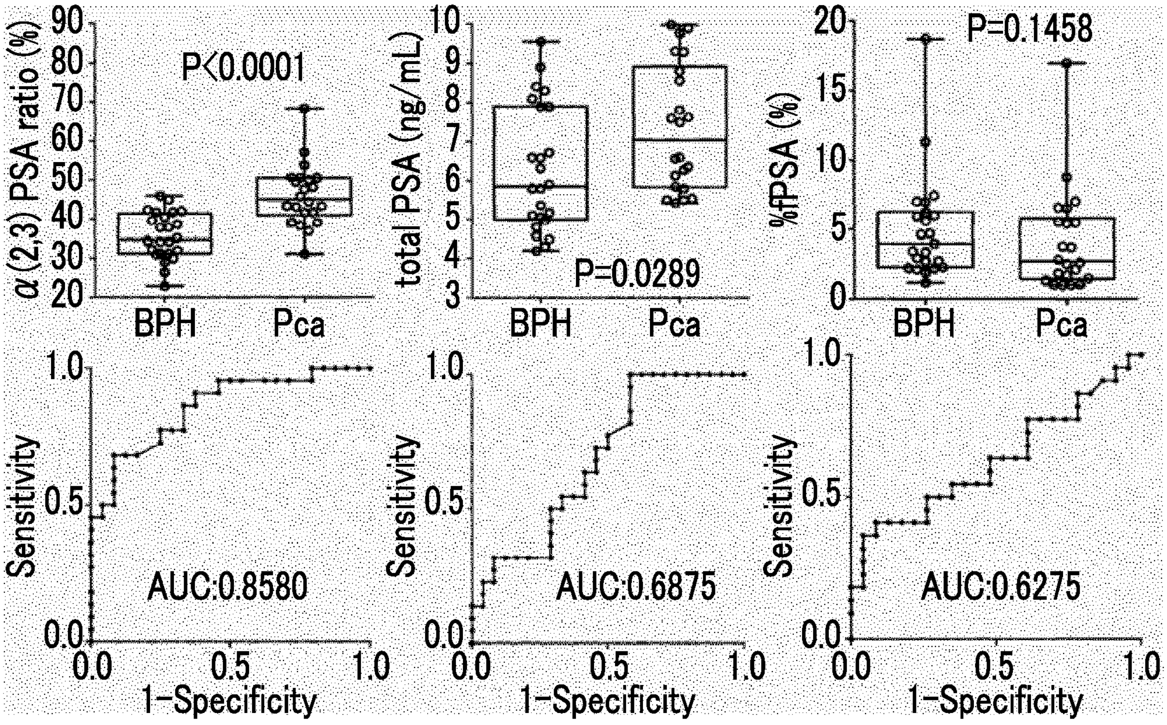

FIG. 10

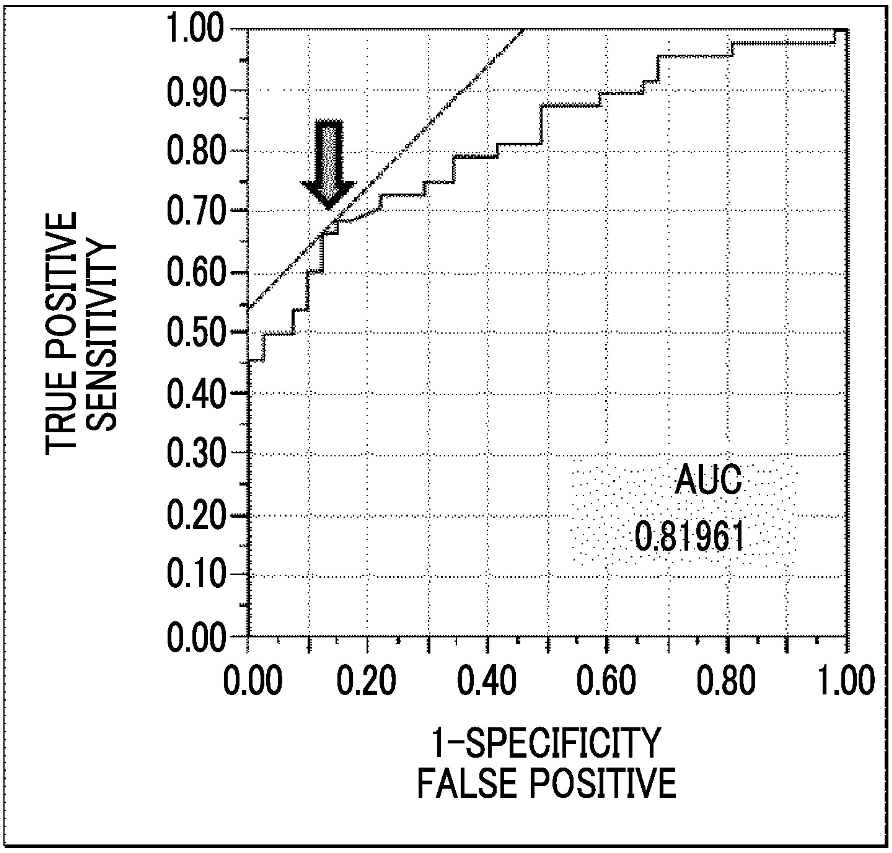


FIG. 11(1)

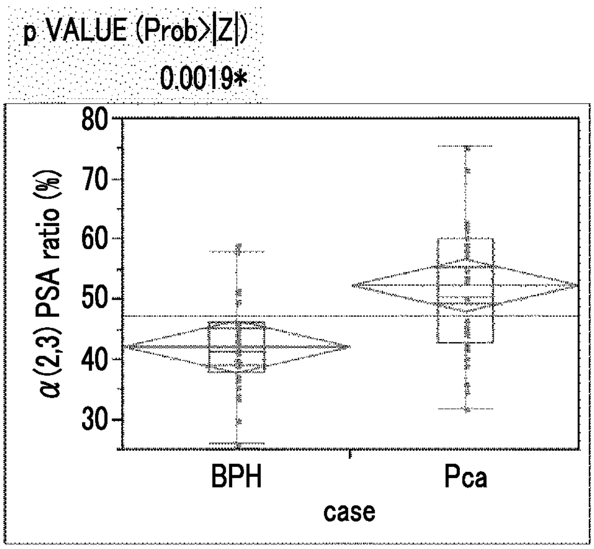


FIG. 11(2)

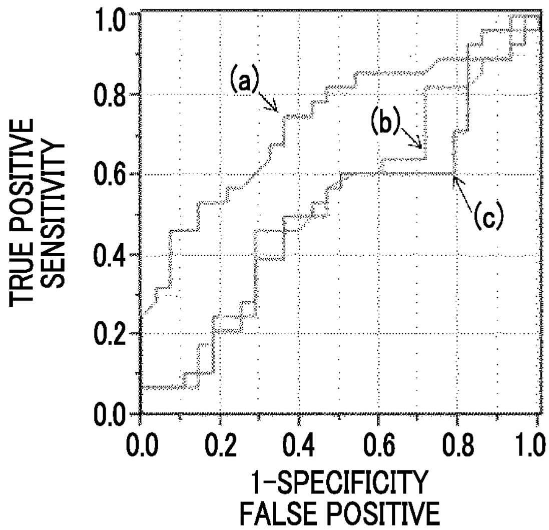

FIG. 12(1)

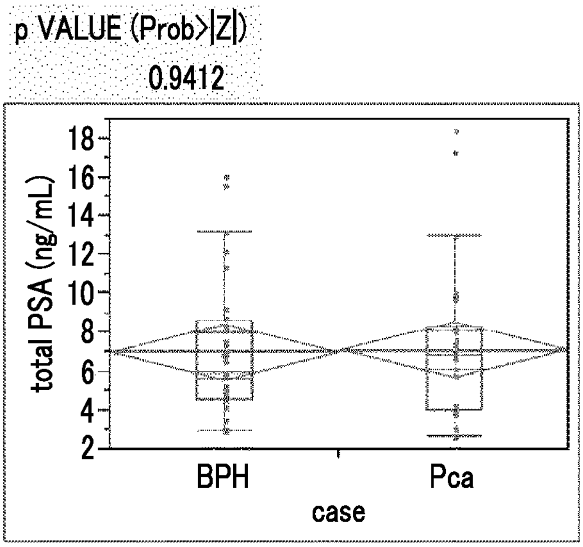


FIG. 12(2)

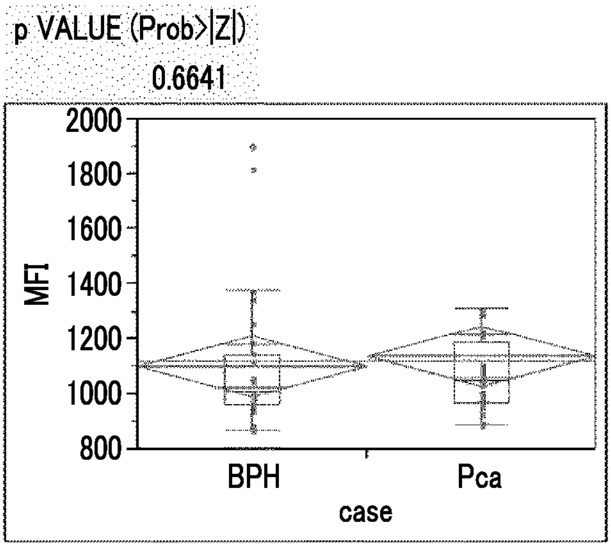


FIG. 13

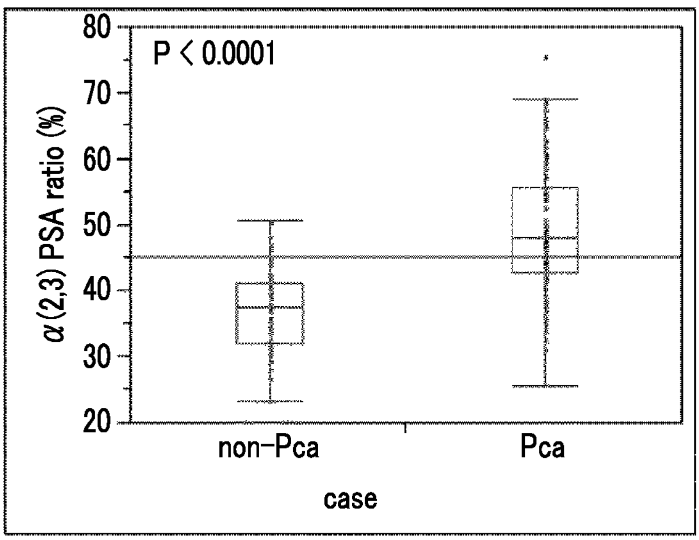

FIG. 14

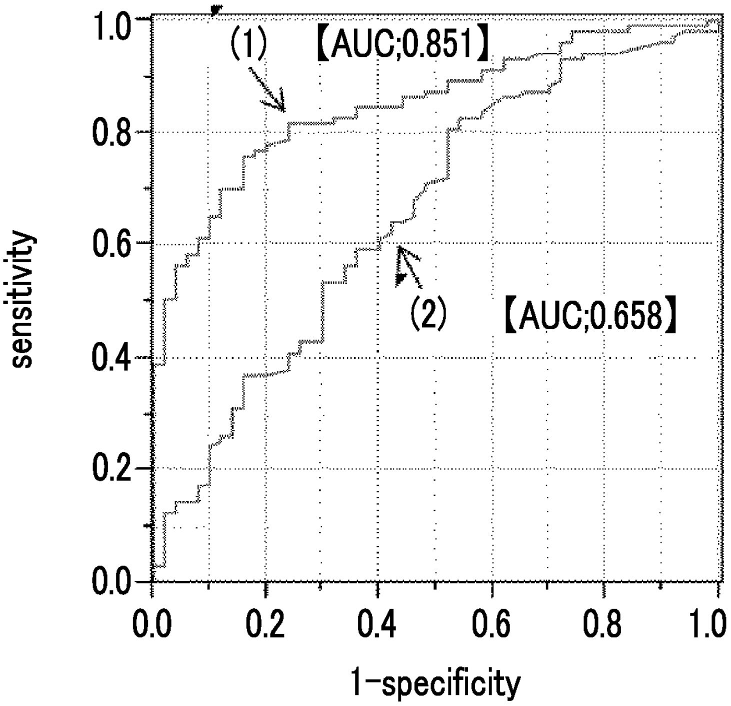
(1) 【AUC;0.851】
(2) 【AUC;0.658】
sensitivity
1-specificity
0.0
0.2
0.4
0.6
0.8
1.0

FIG. 15

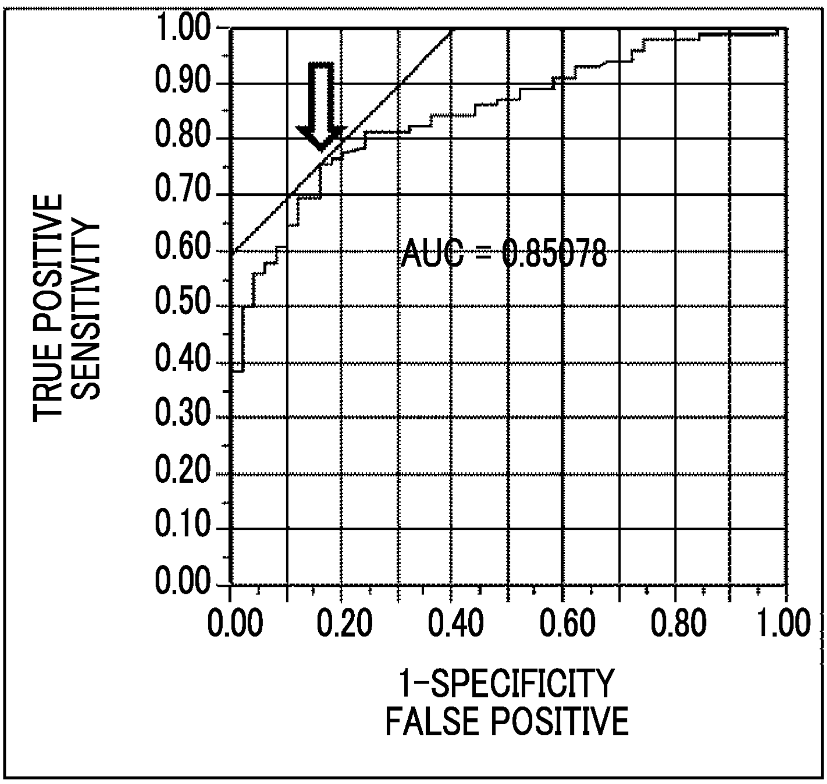
TRUE POSITIVE
SENSITIVITY
AUC = 0.85078
1-SPECIFICITY
FALSE POSITIVE
0.00
0.10
0.20
0.30
0.40
0.50
0.60
0.70
0.80
0.90
1.00

FIG. 16(1)

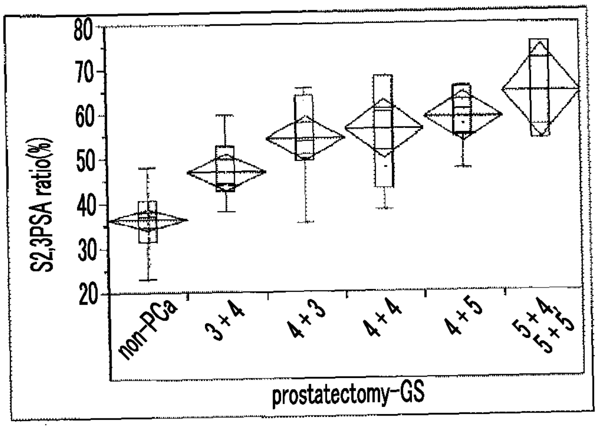


FIG. 16(2)

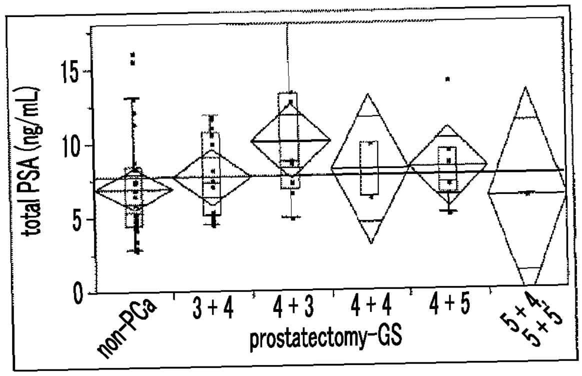


FIG. 17

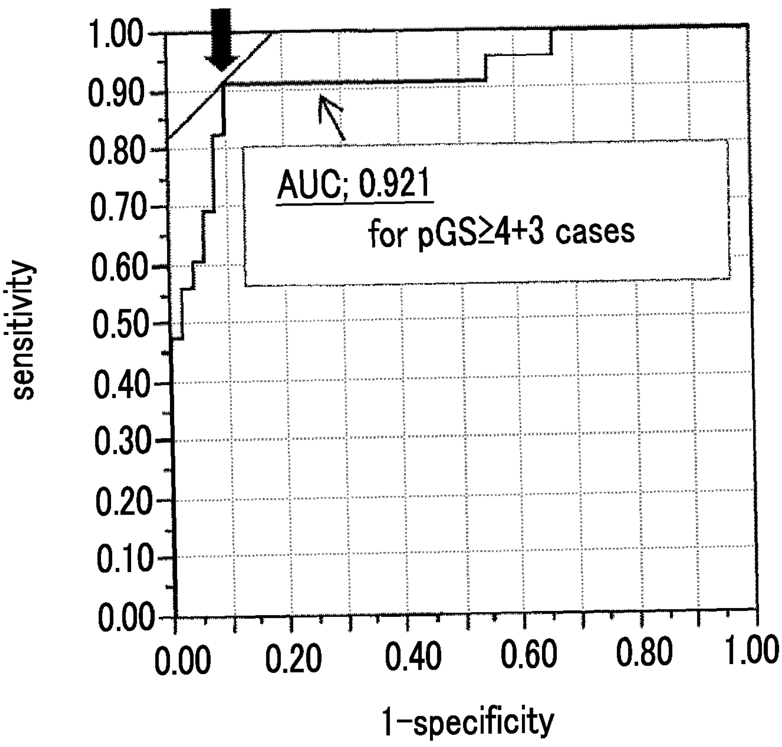

FIG. 18(1)

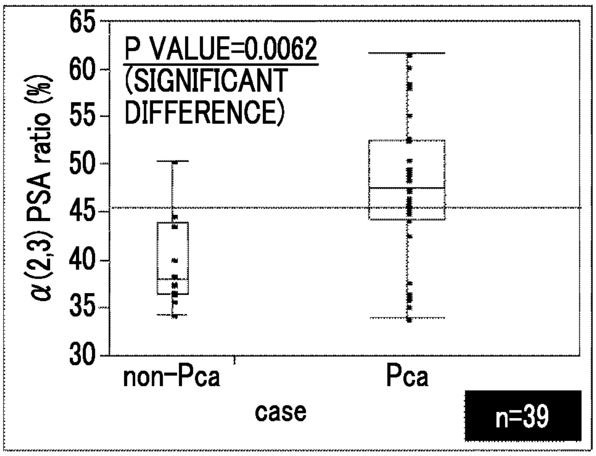


FIG. 18(2)

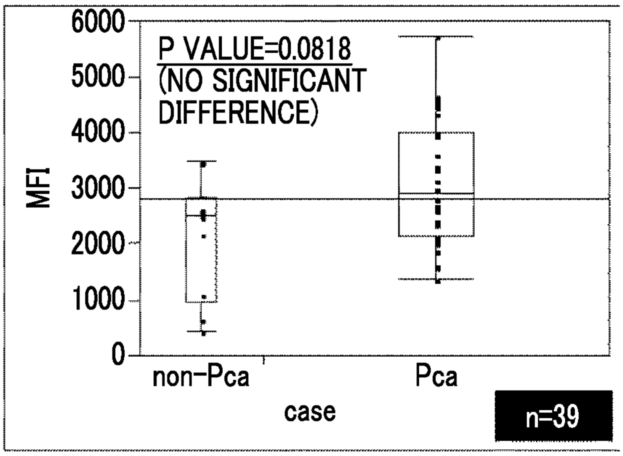

FIG. 19

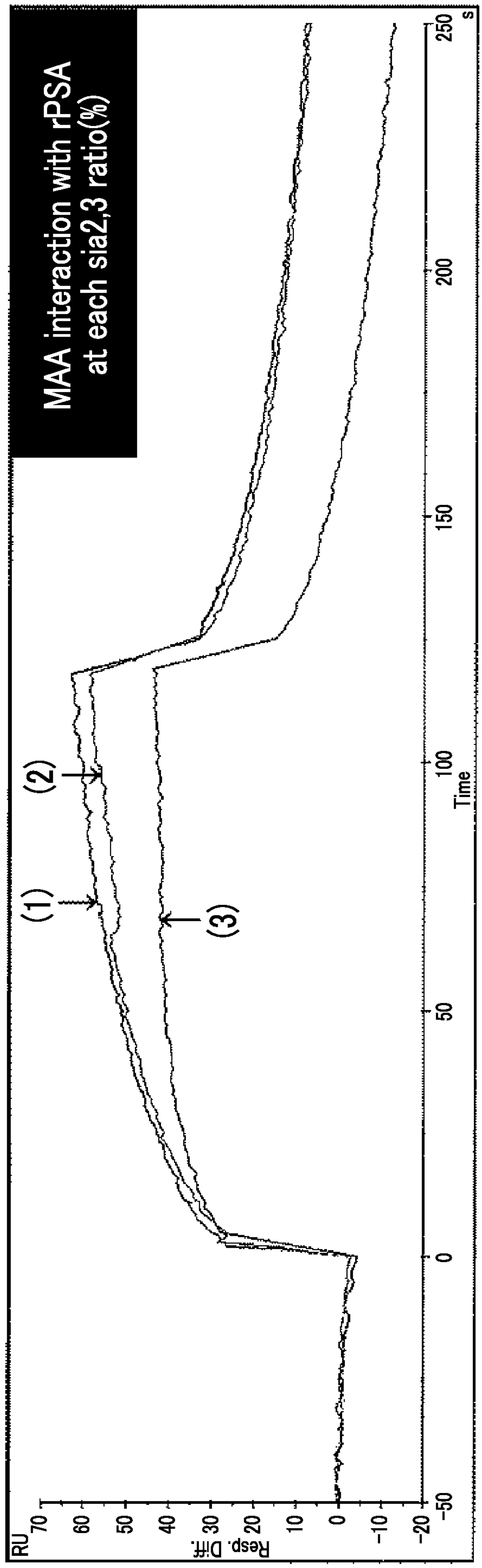
MAA interaction with rPSA
at each sia2,3 ratio(%)
(1)
(2)
(3)
RU
Resp. Diff.
70
60
50
40
30
20
10
0
−10
−20
−50
0
50
100
150
200
250
s
Time

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4514919 B
- JP 2011529184 A
- WO 2014057983 A
- JP 4214779 B
- JP 4862093 B
- WO 2007027495 A

### Non-patent literature cited in the description


- **STAMEY T.A. et al.** *N. Engl. J. Med.,* 1987, vol. 317, 909-916
- **CATALONA W.J. et al.** *JAMA,* 1998, vol. 279, 1542-1547
- **BELANGER A ; VAN HALBEEK H ; GRAVUXES HC et al.** *Prostate,* 1995, vol. 27, 187-197
- **OHYAMA C. et al.** *Glycobiology,* 2004, vol. 14, 671-679
- **TAJIRI M. ; OHYAMA C. ; WADA Y.** *Glycobiolgy,* 2008, vol. 18, 2-8
- **YONEYAMA T. et al.** *Biochem Biophys Res Commun.,* 2014, vol. 448 (4), 390-396
- **YUICHI YAMAMURA.** Course on Experimental Medical Chemistry. Nakayama Shoten Co., Ltd, 1971, vol. 8
- **AKIRA KAWAOI.** Illustrative Description of Fluorescent Antibody. Soft Science Inc, 1983
- Enzyme Immunoassay. Igaku-Shoin Ltd, 1982
- **J. SAMBROOK ; E. F. FRITSCH ; T. MANIATIS.** Molecular Cloning: A Laboratory Manual. Cold Spring Harbor Laboratory Press
- *J. Chromatogr.,* 1992, vol. 593, 253-258
- *Anal. Chem.,* 1992, vol. 64, 1926-1932
- Lectin Microarrays. **MASAO YAMADA.** MICROARRAY METHODS AND PROTOCOLS. CRC Press, 2009, 141
- **KUNO A. et al.** *Nat Methods.,* November 2005, vol. 2 (11), 851-856
- **MATSUO SATO.** Difference of placental alkaline phosphatase molecule on the basis of genetic phenotype. *Journal of Tokyo Women's Medical University,* 1990, vol. 60 (8), 609-619